# EUROPÄISCHE PATENTSCHRIFT

(11) **EP 3 335 254 B1**
(45) Veröffentlichungstag und Bekanntmachung des Hinweises auf die Patenterteilung: **08.04.2020**
(21) Anmeldenummer: 16744660.8
(22) Anmeldetag: 13.07.2016
(51) Int. Cl.: C07D 403/10, H05B 33/14, H01L 51/50, C09K 11/06

(54) **MATERIALIEN FÜR ORGANISCHE ELEKTROLUMINESZENZVORRICHTUNGEN**
MATERIALS FOR ORGANIC ELECTROLUMINESCENT DEVICES
MATÉRIAUX POUR DISPOSITIFS ÉLECTROLUMINESCENTS ORGANIQUES

(30) Priorität: 11.08.2015 EP 15180585
(43) Veröffentlichungstag der Anmeldung: 20.06.2018
(73) Patentinhaber: Merck Patent GmbH, 64293 Darmstadt (DE)
(72) Erfinder: ANÉMIAN, Rémi Manouk, Seoul 657-169 (KR); PARHAM, Amir Hossain, 60486 Frankfurt am Main (DE); LEE, Dong-Hee, Gunpo-shi Gyeonggi-do 435-019 (KR); GROSSMANN, Tobias, 64297 Darmstadt (DE); EBERLE, Thomas, 76829 Landau (DE); JATSCH, Anja, 60489 Frankfurt am Main (DE)
(86) Internationale Anmeldenummer: PCT/EP2016/001204
(87) Internationale Veröffentlichungsnummer: WO 2017/025164

(56) Entgegenhaltungen:
- EP-A1- 2 581 365
- WO-A1-2012/069121
- WO-A1-2015/046916
- KR-A- 20150 111 271

## Beschreibung

Die vorliegende Erfindung betrifft Materialien für die Verwendung in elektronischen Vorrichtungen, insbesondere in organischen Elektrolumineszenzvorrichtungen, sowie elektronische Vorrichtungen, insbesondere organische Elektrolumineszenzvorrichtungen, enthaltend diese Materialien.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden hierbei zunehmend metallorganische Komplexe eingesetzt, die Phosphoreszenz statt Fluoreszenz zeigen. Generell gibt es bei OLEDs, insbesondere auch bei OLEDs, die Triplettemission (Phosphoreszenz) zeigen, jedoch immer noch Verbesserungsbedarf, beispielsweise im Hinblick auf Betriebsspannung und Lebensdauer.

Die Eigenschaften phosphoreszierender OLEDs werden nicht nur von den eingesetzten Triplettemittern bestimmt. Hier sind insbesondere auch die anderen verwendeten Materialien, wie Matrixmaterialien und Ladungstransportmaterialien, von besonderer Bedeutung. Verbesserungen dieser Materialien und deren Ladungstransporteigenschaften können somit auch zu deutlichen Verbesserungen der OLED-Eigenschaften führen.

Gemäß dem Stand der Technik werden unter anderem Indenocarbazolderivate, z. B. gemäß WO 2010/136109, WO 2012/069121, WO 2014/146752, WO 2013/041176, WO 2014/196805 oder KR 2010-028471, als Matrixmaterialien für phosphoreszierende Emitter oder auch als Lochblockier- bzw. Elektronentransportmaterialien in organischen Elektrolumineszenzvorrichtungen eingesetzt. Hier sind weitere Verbesserungen wünschenswert, insbesondere in Bezug auf die Lebensdauer, die Spannung und die Leistungseffizienz. Aus WO 2015/046919 sind verschiedene Indeno- und Indolocarbazolderivate bekannt. Aus EP 2581365 sind erweiterte Indenocarbazolderivate bekannt.

Aufgabe der vorliegenden Erfindung ist die Bereitstellung von verbesserten Materialien, welche sich insbesondere für den Einsatz als Matrixmaterial für phosphoreszierende Emitter und/oder als Lochblockier- bzw. Elektronentransportmaterial eignen.

Überraschend wurde gefunden, dass bestimmte, unten näher beschriebene Verbindungen diese Aufgabe lösen, sich gut für die Verwendung in OLEDs eignen und zu Verbesserungen der organischen Elektrolumineszenzvorrichtung führen, wobei die Verbesserungen insbesondere die Lebensdauer, die Spannung und die Leistungseffizienz betreffen. Es handelt sich bei diesen Materialien um N-Phenyl-Indenocarbazolverbindungen, welche an der Phenylgruppe sowohl einen bestimmten elektronen- oder lochleitenden heteroaromatischen Substituenten wie auch auch elektronisch neutralen bzw. elektronenreichen aromatischen oder heteroaromatischen Substituenten aufweisen. Diese Verbindungen sowie elektronische Vorrichtungen, insbesondere organische Elektrolumineszenzvorrichtungen, welche derartige Verbindungen enthalten, sind daher der Gegenstand der vorliegenden Erfindung.

Gegenstand der vorliegenden Erfindung ist eine Verbindung gemäß der folgenden Formel (1), 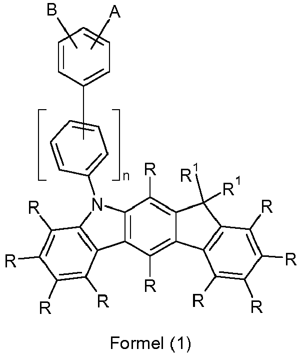 wobei für die verwendeten Symbole und Indizes gilt:
- A: ist ausgewählt aus den Strukturen der folgenden Formeln (A-1) bis (A-6), 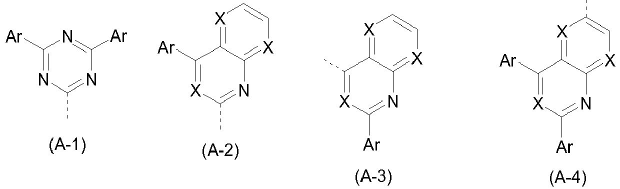 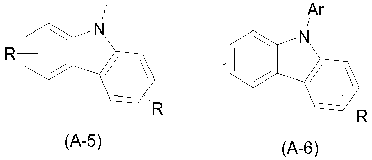 wobei die gestrichelte Bindung die Verknüpfung dieser Gruppen darstellt und wobei an die Gruppen (A-5) und (A-6) optional eine Benzogruppe ankondensiert sein kann, die durch einen oder mehrere Reste R substituiert sein kann;
- B: ist ein aromatisches Ringsystem mit 6 bis 30 aromatischen Ring-atomen oder ein elektronenreiches heteroaromatisches Ring-system mit 13 bis 30 aromatischen Ringatomen, welches jeweils mit einem oder mehreren nicht-aromatischen Resten R substituiert sein kann;
- X: ist bei jedem Auftreten gleich oder verschieden CH oder N, wobei maximal ein X für N steht;
- Ar: ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R substituiert sein kann;
- R, R¹: ist bei jedem Auftreten gleich oder verschieden ausgewählt aus der Gruppe bestehend aus H, D, F, Cl, Br, I, Si(R²)₃, einer geradkettigen Alkyl-, Alkoxy- oder Thioalkylgruppe mit 1 bis 20 C-Atomen oder einer verzweigten oder cyclischen Alkyl-, Alkoxy-oder Thioalkylgruppe mit 3 bis 20 C-Atomen oder einer Alkenyl-oder Alkinylgruppe mit 2 bis 20 C-Atomen, die jeweils mit einem oder mehreren Resten R² substituiert sein kann, wobei eine oder mehrere nicht-benachbarte CH₂-Gruppen durch R²C=CR², C=C, Si(R²)₂, C=O, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R² substituiert sein kann, oder einer Aryloxy- oder Heteroaryloxy-gruppe mit 5 bis 40 aromatischen Ringatomen, die mit einem oder mehreren Resten R² substituiert sein kann; dabei können optional zwei benachbarte Substituenten R¹ ein monocyclisches oder polycyclisches, aliphatisches, aromatisches oder heteroaromatisches Ringsystem bilden, das mit einem oder mehreren Resten R² substituiert sein kann;
- R²: ist bei jedem Auftreten gleich oder verschieden ausgewählt aus der Gruppe bestehend aus H, D, F, CN, einem aliphatischen Kohlenwasserstoffrest mit 1 bis 20 C-Atomen, oder einem aromatischen oder heteroaromatischen Ringsystem mit 5 bis 30 aromatischen Ringatomen, in dem ein oder mehrere H-Atome durch D, F, CN oder eine Alkylgruppe mit 1 bis 10 C-Atomen ersetzt sein können, wobei zwei oder mehr benachbarte Substituenten R² miteinander ein mono- oder polycyclisches, aliphatisches Ring-system bilden können;
- n: ist 0 oder 1.

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei enthält die Heteroarylgruppe bevorzugt maximal drei Heteroatome, von denen maximal eines ausgewählt ist aus O oder S und die weiteren Heteroatome N sind. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl oder Bipyridin, werden dagegen nicht als Aryl- oder Heteroarylgruppe, sondern als aromatisches bzw. heteroaromatisches Ringsystem bezeichnet.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Dabei enthält das heteroaromatische Ringsystem bevorzugt pro Heteroarylgruppe, die in dem Ringsystem enthalten ist, maximal drei Heteroatome, von denen maximal eines ausgewählt ist aus O oder S und die weiteren Heteroatome N sind. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden.

Ein elektronenreiches heteroaromatisches Ringsystem im Sinne der vorliegenden Erfindung ist ein heteroaromatisches Ringsystem, welches keine elektronenarmen Heteroarylringe enthält. Elektronenarme Heteroarylringe im Sinne der vorliegenden Erfindung sind 5-Ring-Heteroarylringe mit zwei oder mehr Heteroatomen und 6-Ring-Heteroarylringe. Ein elektronenreiches heteroaromatisches Ringsystem enthält somit nur Arylringe sowie 5-Ring-Heteroarylringe mit genau einem Heteroatom, insbesondere Pyrrol, Furan und/oder Thiophen, wobei die Arylringe und die 5-Ring-Heteroarylringe auch aneinander ankondensiert sein können. Beispiele für geeignete elektronenreiche heteroaromatische Ringsysteme sind Carbazol, Dibenzofuran und Dibenzothiophen.

Bei den Gruppen der Formeln (A-1) bis (A-4) handelt es sich um elektronenarme Substituenten. Bei den Gruppen der Formeln (A-5) und (A-6) handelt es sich um elektronenreiche Substituenten.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH2-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 aromatischen Ringatomen, welches noch jeweils mit den oben genannten Resten R² oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.

Unter benachbarten Resten bzw. benachbarten Substituenten im Sinne der vorliegenden Anmeldung werden Substituenten verstanden, die an C-Atome gebunden sind, welche wiederum direkt aneinander gebunden sind, oder Substituenten, die an dasselbe C-Atom gebunden sind.

In einer bevorzugten Ausführungsform der Erfindung weist das Indenocarbazolgrundgerüst insgesamt maximal drei Substituenten R auf, die ungleich H sind, besonders bevorzugt maximal zwei Substituenten R, die ungleich H sind, und ganz besonders bevorzugt maximal einen Substituenten R, der ungleich H ist. Ganz besonders bevorzugt handelt es sich um eine Verbindung der folgenden Formel (2), 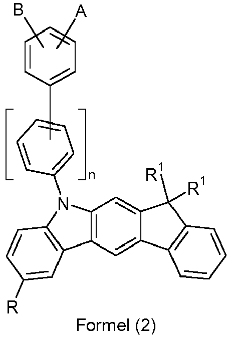 wobei die verwendeten Symbole und Indizes die oben genannten Bedeutungen aufweisen.

In einer bevorzugten Ausführungsform der Formel (2) ist der Rest R am Indenocarbazolgrundgerüst Wasserstoff, so dass es sich um eine Struktur der folgenden Formel (2a) handelt, 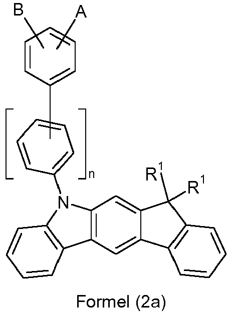 wobei die verwendeten Symbole und Indizes die oben genannten Bedeutungen aufweisen.

Wenn der Index n = 1 ist, kann die Phenylengruppe über die ortho-, meta- oder para-Positionen verknüpft sein. In einer bevorzugten Ausführungsform ist n = 0, also die entsprechende Phenylengruppe nicht vorhanden, oder n = 1 und die Phenylengruppe ist über die para-Positionen verknüpft. Bevorzugt sind die Strukturen der folgenden Formeln (3) und (4), insbesondere die Verbindung der Formel (3), 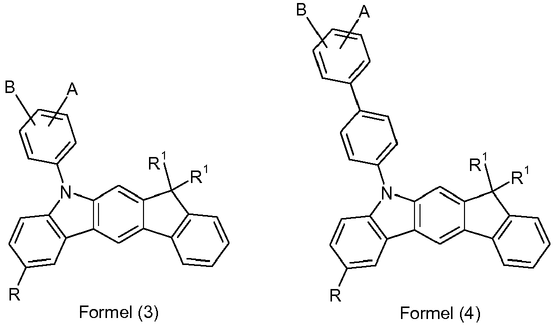 wobei die verwendeten Symbole die oben genannten Bedeutungen aufweisen. Besonders bevorzugt ist die Verbindung der Formel (3).

Besonders bevorzugt sind die Verbindungen der folgenden Formeln (3a) und (4a), insbesondere die Verbindung der Formel (3a), 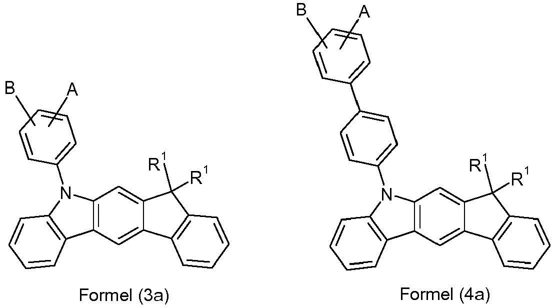 wobei die verwendeten Symbole die oben genannten Bedeutungen aufweisen.

Im Folgenden wird die Phenylgruppe, an die die Gruppen A und B gebunden sind, genauer beschrieben. Dabei können die Gruppen A und B in beliebigen Positionen an der Phenylgruppe gebunden sein. Dadurch ergeben sich die Strukturen der folgenden Formeln (P-1) bis (P-10), 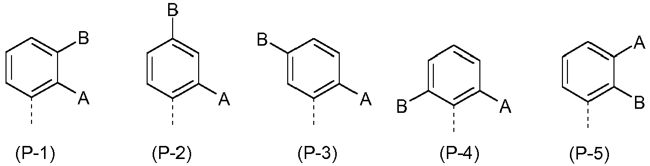 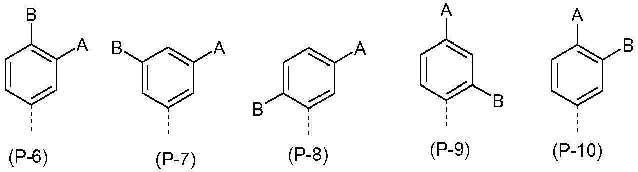 wobei die Gruppen A und B jeweils mit eingezeichnet sind, die gestrichelte Bindung für n = 0 die Bindung an das Stickstoffatom des Indenocarbazols und für n = 1 die Bindung an die Phenylengruppe darstellt und A und B die oben genannten und im Folgenden genauer ausgeführten Bedeutungen aufweisen.

Dabei sind die Strukturen bevorzugt, in denen die Gruppe A in meta-Position zur Verknüpfung der Phenylgruppe gebunden ist, also die Strukturen (P-5) bis (P-8). Besonders bevorzugt ist die Struktur (P-7).

Im Folgenden wird die Gruppe A genauer beschrieben. Bevorzugte Ausführungsformen der Gruppen (A-2) bis (A-4) sind die Gruppen der folgenden Formeln (A-2a) bis (A-2d), (A-3a) und (A-4a), 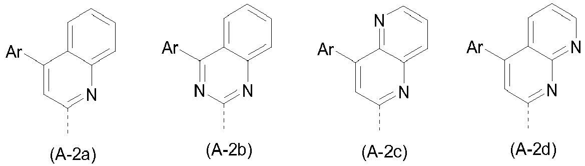 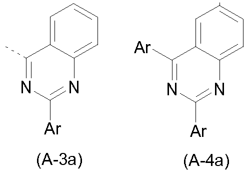 wobei die verwendeten Symbole die oben genannten Bedeutungen aufweisen.

Dabei ist es bevorzugt, wenn Ar für eine Phenylgruppe steht, die mit einem oder mehreren Resten R substiutiert sein kann, bevorzugt aber unsubstituiert ist.

Unter den oben genannten Gruppen sind die Gruppen (A-2a) bis (A-2d) und (A-3a) besonders bevorzugt. Ganz besonders bevorzugt sind die Gruppen (A-2a) und (A-3a).

Bevorzugte Ausführungsformen der Gruppe (A-5) sind die Gruppen der folgenden Formeln (A-5a) bis (A-5d), 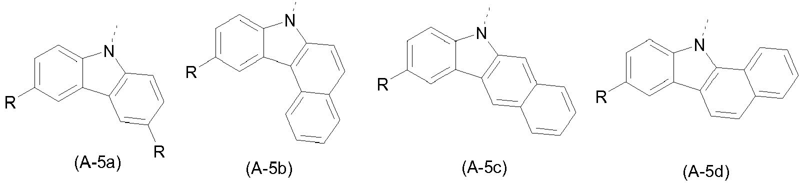 wobei die verwendeten Symbole die oben genannten Bedeutungen haben und R bevorzugt für H steht.

Bevorzugte Ausführungsformen der Gruppe (A-6) sind die Gruppen der folgenden Formeln (A-6a) bis (A-6f), 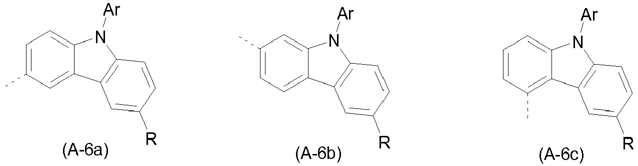 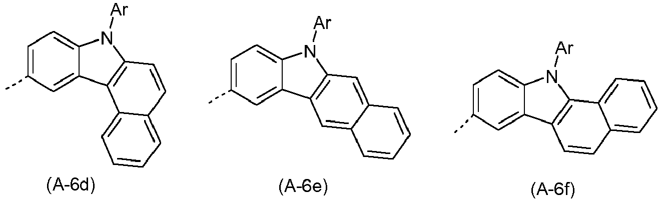 wobei die verwendeten Symbole die oben genannten Bedeutungen haben und R bevorzugt für H steht.

Besonders bevorzugt ist die Gruppe A gewählt aus den Gruppen (A-1), (A-5) und (A-6), und ganz besonders bevorzugt ist die Gruppe A eine Gruppe (A-1), also ein substituiertes Triazin.

Ganz besonders bevorzugt sind daher die Verbindungen der folgenden Formeln (3b) und (4b), insbesondere Verbindungen der Formel (3b), 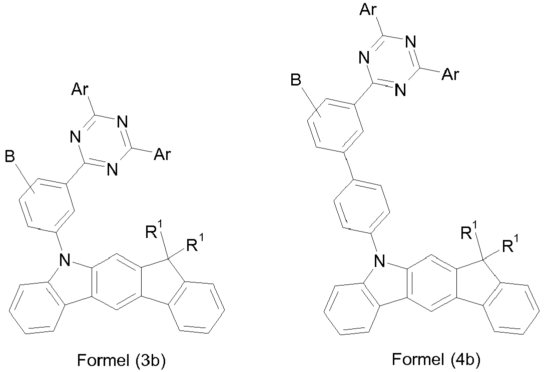 wobei die verwendeten Symbole die oben genannten Bedeutungen aufweisen.

Insbesondere bevorzugt sind die Verbindungen der folgenden Formeln (3c) und (4c), insbesondere der Formel (3c) 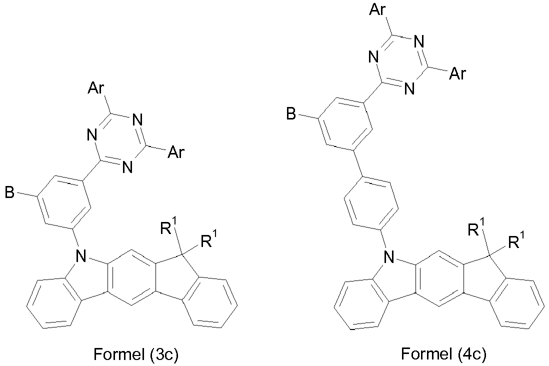 wobei die verwendeten Symbole die oben genannten Bedeutungen aufweisen.

In einer bevorzugten Ausführungsform der Gruppen (A-1) bis (A-4) und (A-6) und den oben genannten bevorzugten Ausführungsformen ist die Gruppe Ar gewählt aus einem aromatischen oder heteroaromatischen Ringsystem mit 6 bis 24 aromatischen Ringatomen, bevorzugt mit 6 bis 12 aromatischen Ringatomen, welches durch einen oder mehrere nicht-aromatische Reste R substituiert sein kann, bevorzugt Phenyl, Biphenyl, insbesondere ortho-, meta- oder para-Biphenyl, Terphenyl, insbesondere ortho-, meta-, para- oder verzweigtes Terphenyl, Quaterphenyl, insbesondere ortho-, meta-, para- oder verzweigtes Quaterphenyl, Fluorenyl, insbesondere 1-, 2-, 3- oder 4-Fluorenyl, Spirobifluorenyl, insbesondere 1-, 2-, 3- oder 4-Spirobifluorenyl, Dibenzofuranyl, insbesondere 1-, 2-, 3- oder 4-Dibenzofuranyl, Dibenzothienyl, insbesondere 1-, 2-, 3- oder 4-Dibenzothienyl, oder N-Phenyl-Carbazolyl, insbesondere N-Phenyl-1-, 2-, 3- oder 4-Carbazolyl, wobei diese Gruppen jeweils durch einen oder mehrere nicht-aromatische Reste R substituiert sein können, bevorzugt aber unsubstituiert sind. Ganz besonders bevorzugt sind Phenyl, Biphenyl, Terphenyl, Quaterphenyl, Fluorenyl, Dibenzofuranyl und Dibenzothienyl, insbesondere Phenyl.

Im Folgenden wird die Gruppe B genauer beschrieben. In einer bevorzugten Ausführungsform der Erfindung ist die Gruppe B gewählt aus einem aromatischen Ringsystem mit 6 bis 24 aromatischen Ringatomen, bevorzugt mit 6 bis 12 aromatischen Ringatomen oder einem elektronenreichen heteroaromatischen Ringsystem mit 13 bis 24 aromatischen Ringatomen, bevorzugt mit 13 bis 20 aromatischen Ringatomen, besonders bevorzugt mit 13 aromatischen Ringatomen, welches jeweils durch einen oder mehrere nicht-aromatische Reste R substituiert sein kann. Bevorzugte Gruppen B sind gewählt aus Phenyl, Biphenyl, insbesondere ortho-, meta- oder para-Biphenyl, Terphenyl, insbesondere ortho-, meta-, para- oder verzweigtes Terphenyl, Quaterphenyl, insbesondere ortho-, meta-, para- oder verzweigtes Quaterphenyl, Fluorenyl, insbesondere 1-, 2-, 3- oder 4-Fluorenyl, Spirobifluorenyl, insbesondere 1-, 2-, 3- oder 4-Spirobifluorenyl, Naphthyl, Phenanthrenyl, Triphenylenyl, Dibenzofuranyl, insbesondere 1-, 2-, 3- oder 4-Dibenzofuranyl, Dibenzothienyl, insbesondere 1-, 2-, 3- oder 4-Dibenzothienyl, oder N-Phenyl-Carbazolyl, insbesondere N-Phenyl-1-, 2-, 3- oder 4-Carbazolyl, wobei diese Gruppen jeweils durch einen oder mehrere nicht-aromatische Reste R substituiert sein können, bevorzugt aber unsubstituiert sind.

Besonders bevorzugt ist die Gruppe B ausgewählt aus den Strukturen der folgenden Formeln (B-1) bis (B-10), 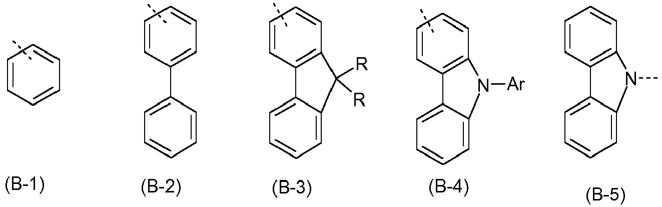 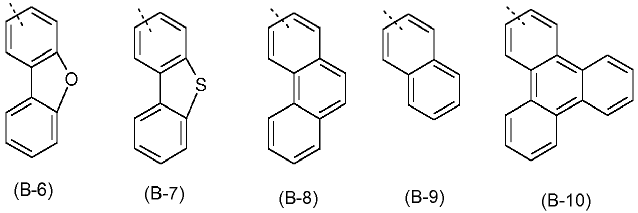 wobei die gestrichelte Bindung die Verknüpfung dieser Gruppe darstellt und die weiteren verwendeten Symbole die oben genannten Bedeutungen aufweisen.

Bevorzugte Ausführungsformen der Gruppe (B-2), (B-3), (B-4), (B-6), (B-7), (B-8), (B-9) und (B-10) sind die Gruppen der folgenden Formeln (B-2a) bis (B-2c), (B-3a) bis (B-3d), (B-4a) bis (B-4d), (B-6a) bis (B-6d), (B-7a) bis (B-7d), (B-8a) bis (B-8e), (B-9a), (B-9b), (B-10a) und (B-10b), 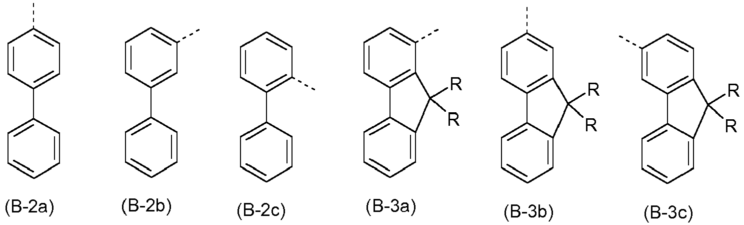 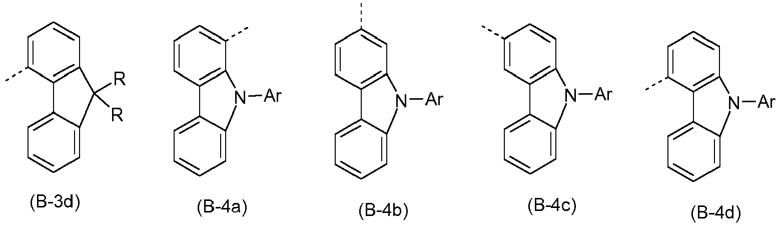 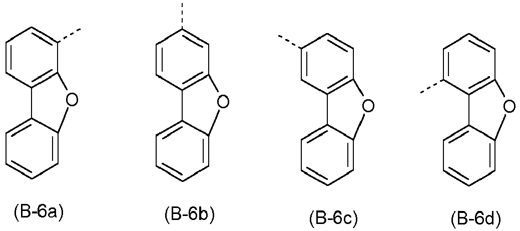 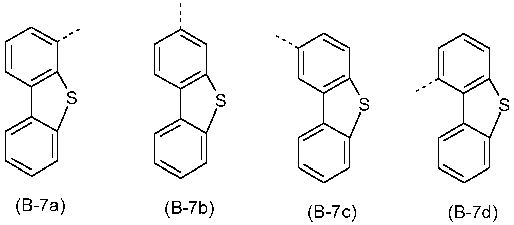 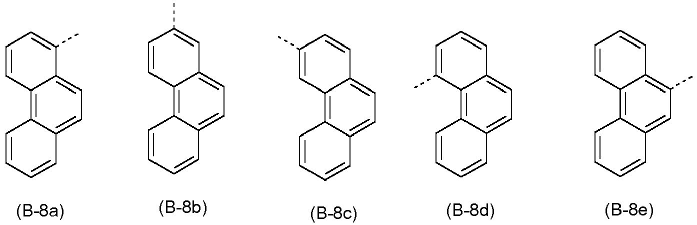 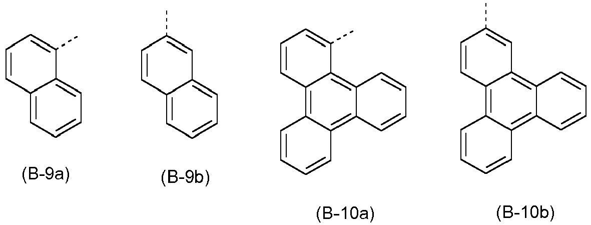 wobei die verwendeten Symbole die oben genannten Bedeutungen haben.

In einer weiteren bevorzugten Ausführungsform der Erfindung ist R gleich oder verschieden bei jedem Auftreten ausgewählt aus der Gruppe bestehend aus H, D, F, N(R²)₂, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenyl- oder Alkinylgruppe mit 2 bis 10 C-Atomen, die jeweils mit einem oder mehreren Resten R² substituiert sein kann, wobei eine oder mehrere nicht-benachbarte CH2-Gruppen durch O ersetzt sein können und wobei ein oder mehrere H-Atome durch D oder F ersetzt sein können, oder einem aromatischen oder heteroaromatischen Ringsystem mit 5 bis 30 aromatischen Ringatomen, das jeweils mit einem oder mehreren Resten R² substituiert sein kann. Besonders bevorzugt ist R gleich oder verschieden bei jedem Auftreten ausgewählt aus der Gruppe bestehend aus H, N(R²)₂, einer geradkettigen Alkylgruppe mit 1 bis 6 C-Atomen, insbesondere mit 1 bis 4 C-Atomen, oder einer verzweigten oder cyclischen Alkylgruppe mit 3 bis 8 C-Atomen, insbesondere mit 3 bis 6 C-Atomen, die jeweils mit einem oder mehreren Resten R² substituiert sein kann, bevorzugt aber unsubstiutiert ist, oder einem aromatischen oder heteroaromatischen Ringsystem mit 5 bis 24 aromatischen Ringatomen, das jeweils mit einem oder mehreren Resten R² substituiert sein kann, bevorzugt aber unsubstituiert ist.

In einer weiteren bevorzugten Ausführungsform der Erfindung ist R¹ gleich oder verschieden bei jedem Auftreten ausgewählt aus der Gruppe bestehend aus einer geradkettigen Alkylgruppe mit 1 bis 10 C-Atomen oder einer verzweigten oder cyclischen Alkylgruppe mit 3 bis 10 C-Atomen oder einer Alkenyl- oder Alkinylgruppe mit 2 bis 10 C-Atomen, die jeweils mit einem oder mehreren Resten R² substituiert sein kann, wobei eine oder mehrere nicht-benachbarte CH2-Gruppen durch O ersetzt sein können und wobei ein oder mehrere H-Atome durch D oder F ersetzt sein können, oder einem aromatischen oder heteroaromatischen Ringsystem mit 5 bis 30 aromatischen Ringatomen, das jeweils mit einem oder mehreren Resten R² substituiert sein kann; dabei können die beiden Reste R¹ auch miteinander ein aliphatisches, aromatisches oder heteroaromatisches Ringsystem bilden. Besonders bevorzugt ist R gleich oder verschieden bei jedem Auftreten ausgewählt aus der Gruppe bestehend aus einer geradkettigen Alkylgruppe mit 1 bis 6 C-Atomen, bevorzugt mit 1 bis 4 C-Atomen, insbesondere Methyl, oder einer verzweigten oder cyclischen Alkylgruppe mit 3 bis 8 C-Atomen, insbesondere mit 3 bis 6 C-Atomen, die jeweils mit einem oder mehreren Resten R² substituiert sein kann, bevorzugt aber unsubstiutiert ist, oder einem aromatischen Ringsystem mit 6 bis 12 aromatischen Ringatomen, das jeweils mit einem oder mehreren Resten R² substituiert sein kann, bevorzugt aber unsubstituiert ist; dabei können die beiden Reste R¹ auch miteinander ein aliphatisches oder aromatisches Ringsystem bilden.

Wenn R bzw. R¹ für ein aromatisches oder heteroaromatisches Ringsystem steht, dann ist dieser Rest R bevorzugt gleich oder verschieden bei jedem Auftreten aus denselben Gruppen ausgewählt, wie oben als geeignete Gruppen für Ar angegeben.

Dabei haben in Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Wenn die Verbindung der Formel (1) bzw. die bevorzugten Ausführungsformen als Matrixmaterial für einen phosphoreszierenden Emitter oder in einer Schicht, die direkt an eine phosphoreszierende Schicht angrenzt, verwendet werden, ist es weiterhin bevorzugt, wenn die Triplettenergie der Verbindung gleich hoch oder höher ist als die des phosphoreszierenden Emitters. Dies kann insbesondere für grün und blau phosphoreszierende Emitter, welche eine höhere Triplettenergie als rot phosphoreszierende Emitter aufweisen, dadurch erreicht werden, dass die erfindungsgemäße Verbindung keine kondensierten Aryl- bzw. Heteroarylgruppen enthält, in denen mehr als zwei Sechsringe direkt aneinander ankondensiert sind. Solche Verbindungen sind daher eine weitere bevorzugte Ausführungsform der Erfindung. Insbesondere ist es für diese Verwendung bevorzugt, dass die Reste R, R¹, R² und Ar keine kondensierten Aryl- bzw. Heteroarylgruppen enthalten, in denen zwei oder mehr Sechsringe direkt aneinander ankondensiert sind.

Die oben genannten bevorzugten Ausführungsformen können beliebig miteinander kombiniert werden. In einer besonders bevorzugten Ausführungsform der Erfindung treten die oben genannten Bevorzugungen gleichzeitig auf.

Beispiele für bevorzugte Verbindungen gemäß den oben aufgeführten Ausführungsformen sind die in der folgenden Tabelle aufgeführten Verbindungen.

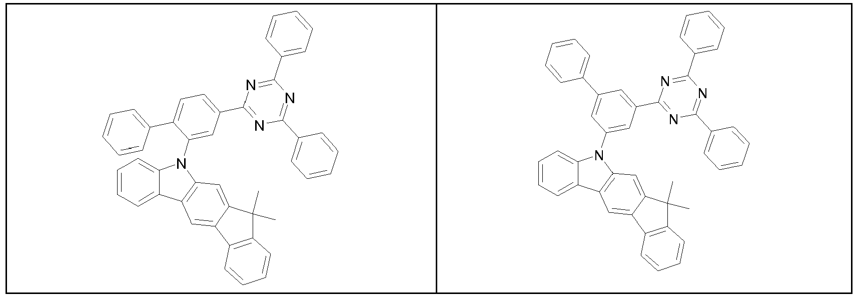

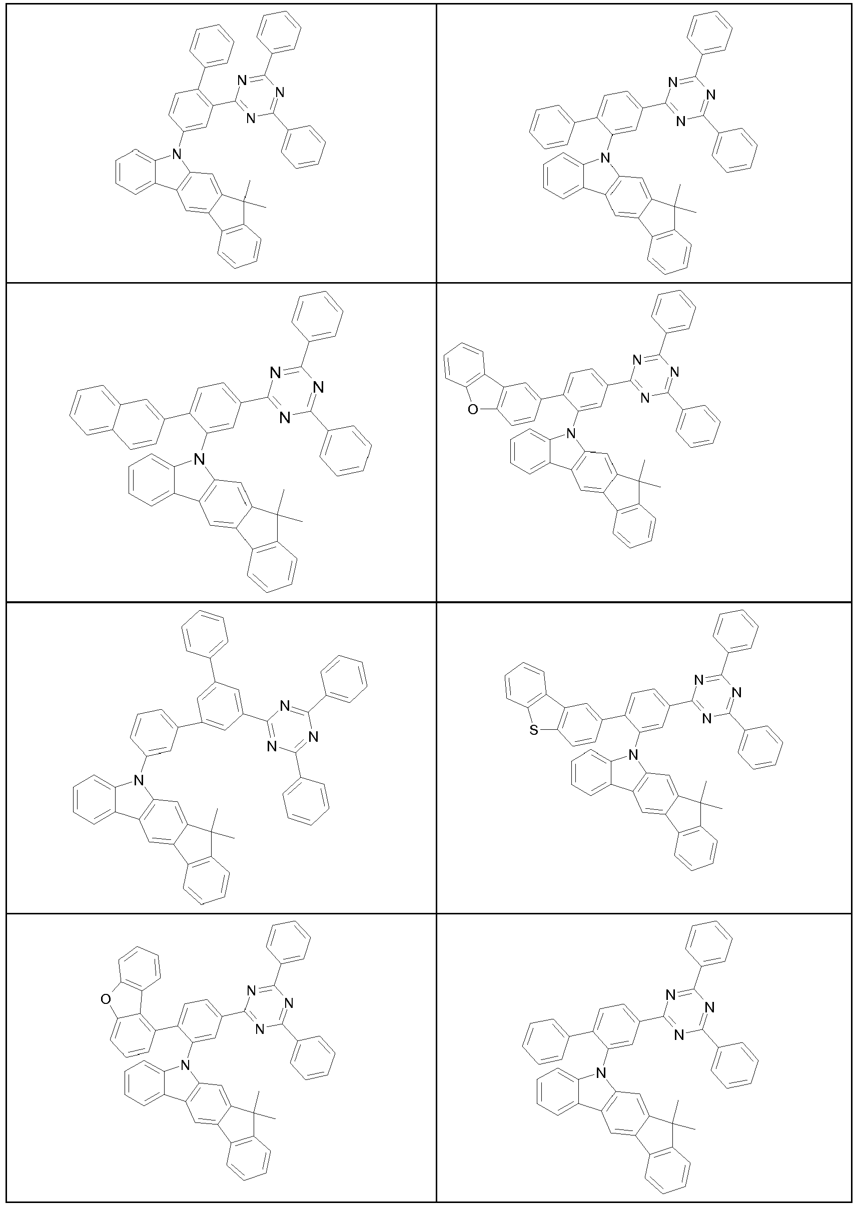

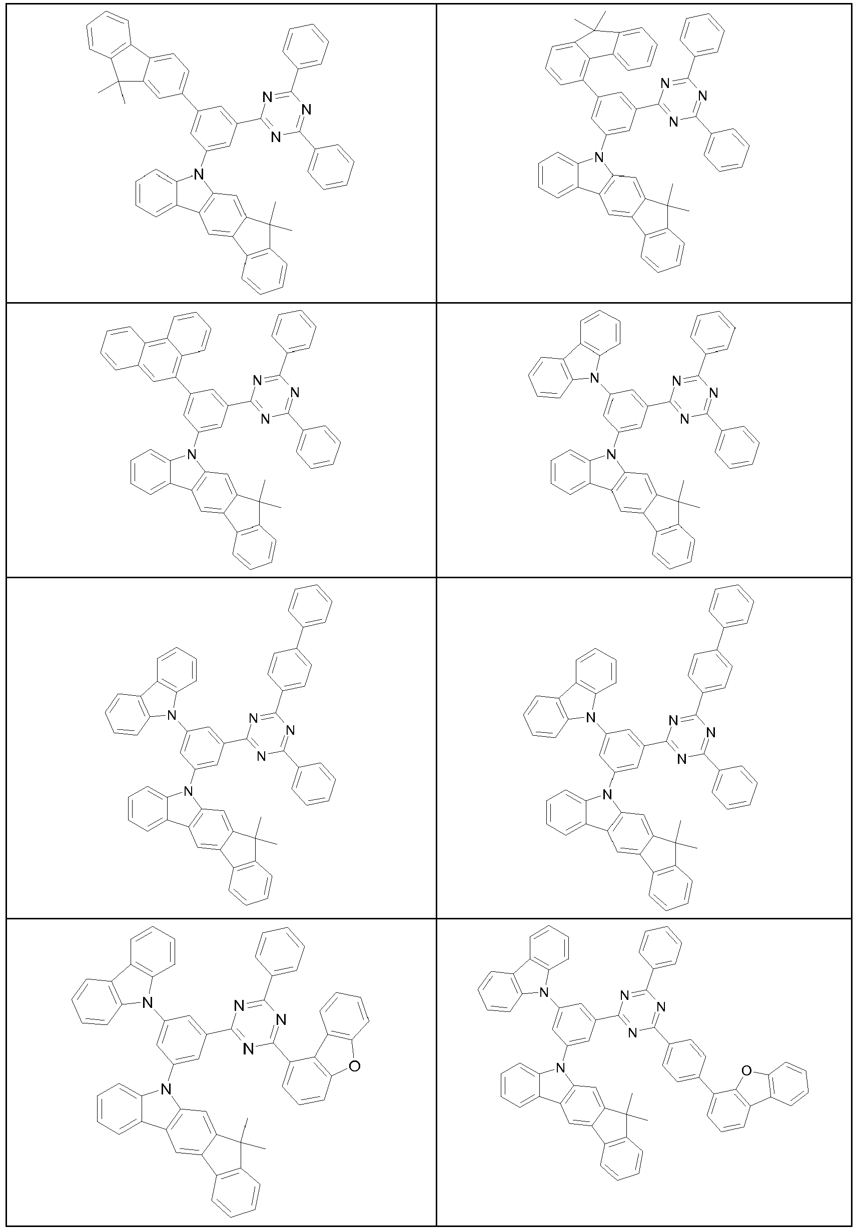

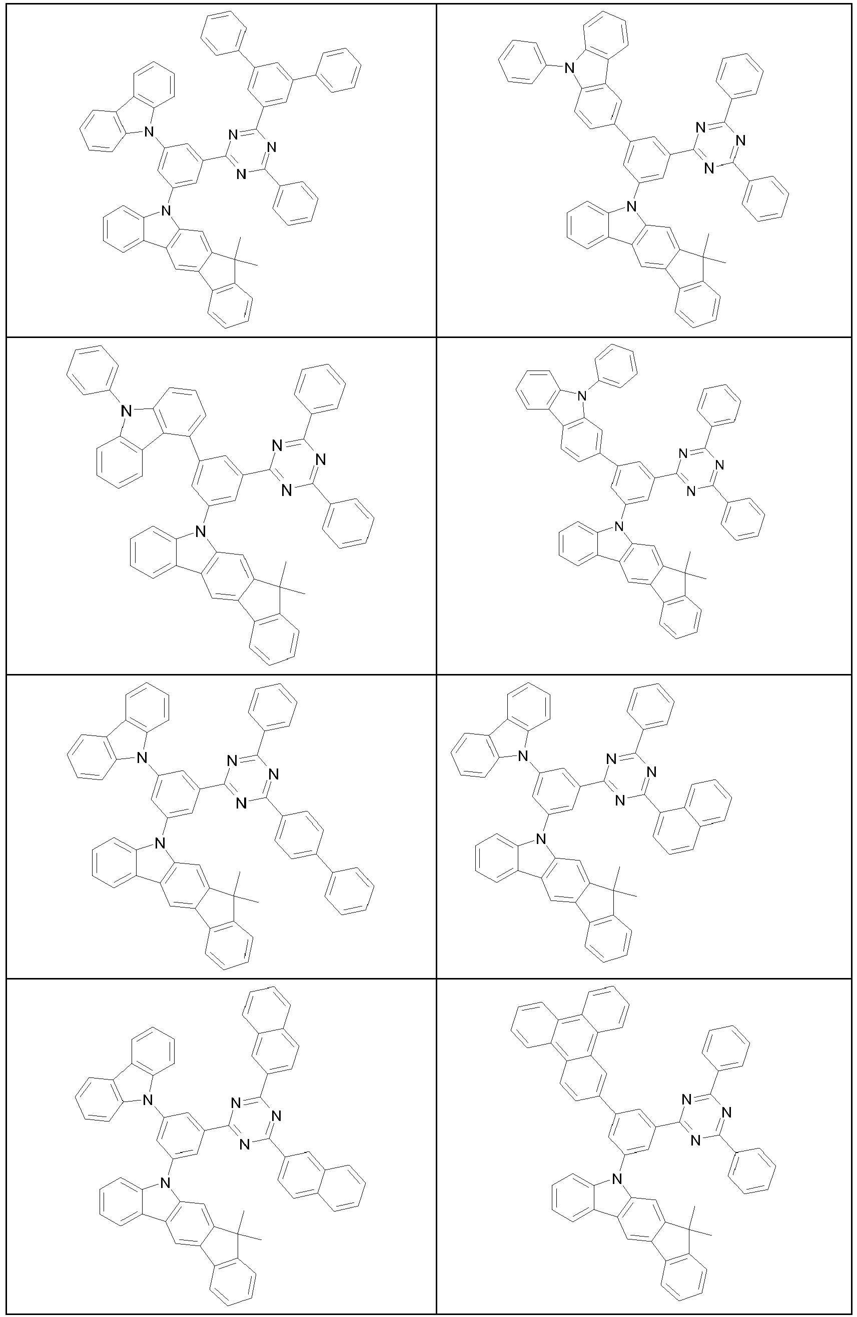

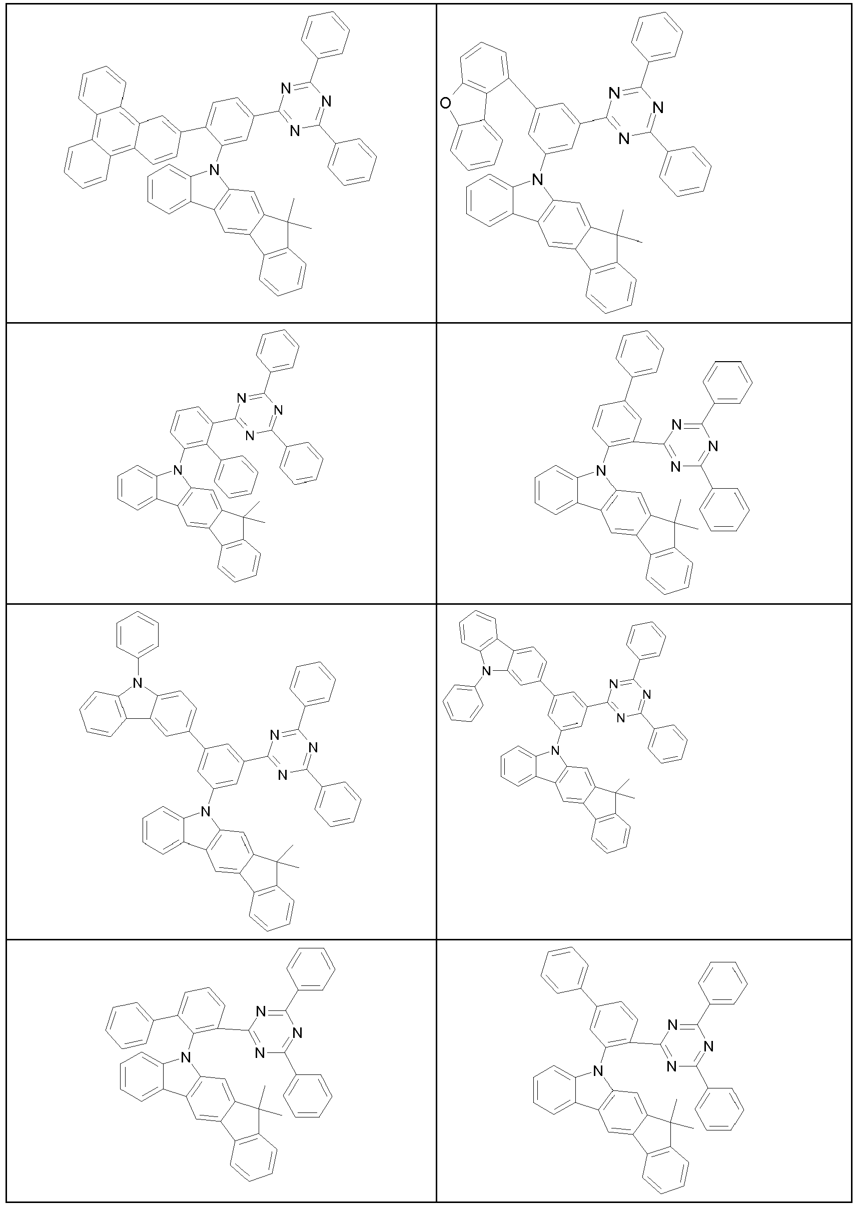

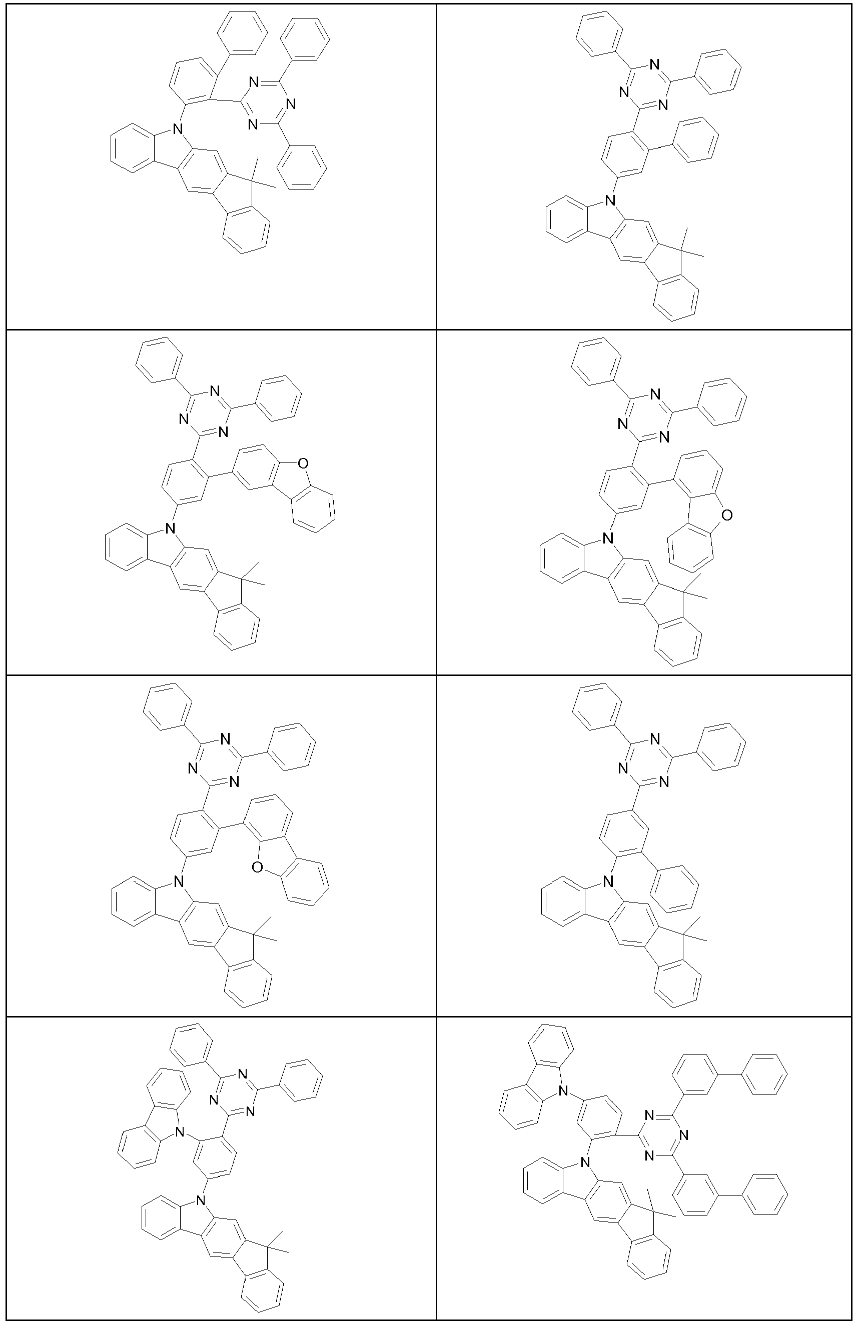

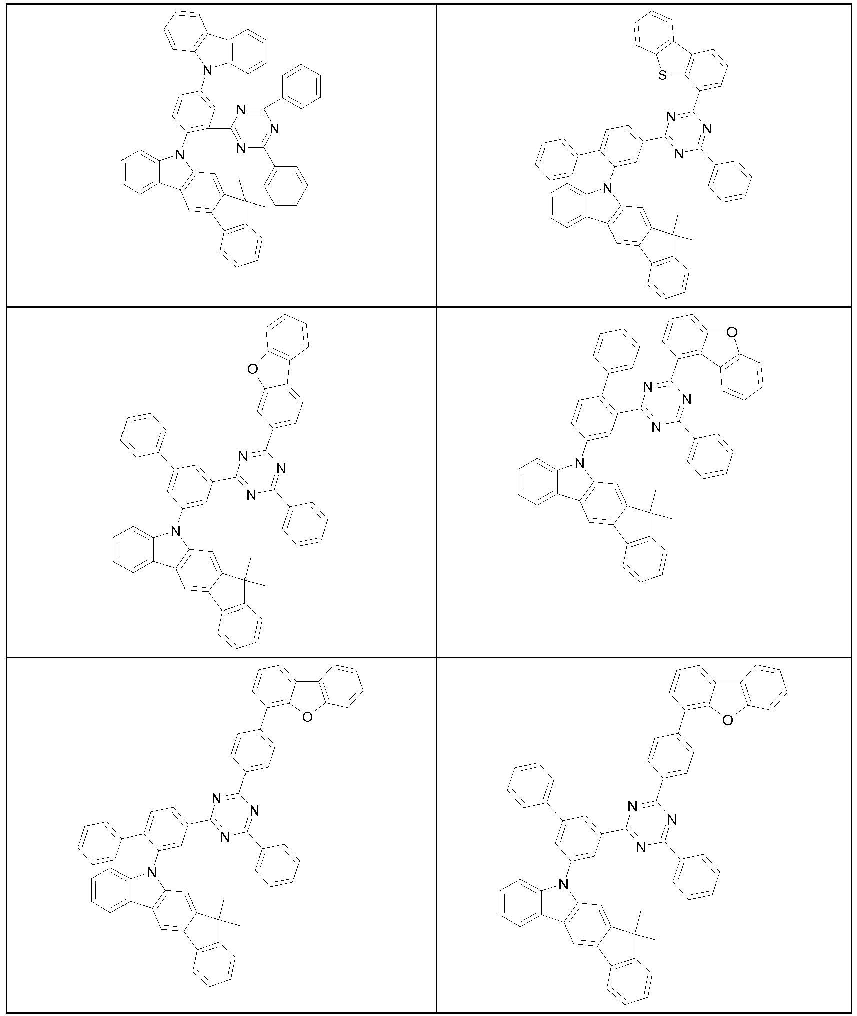

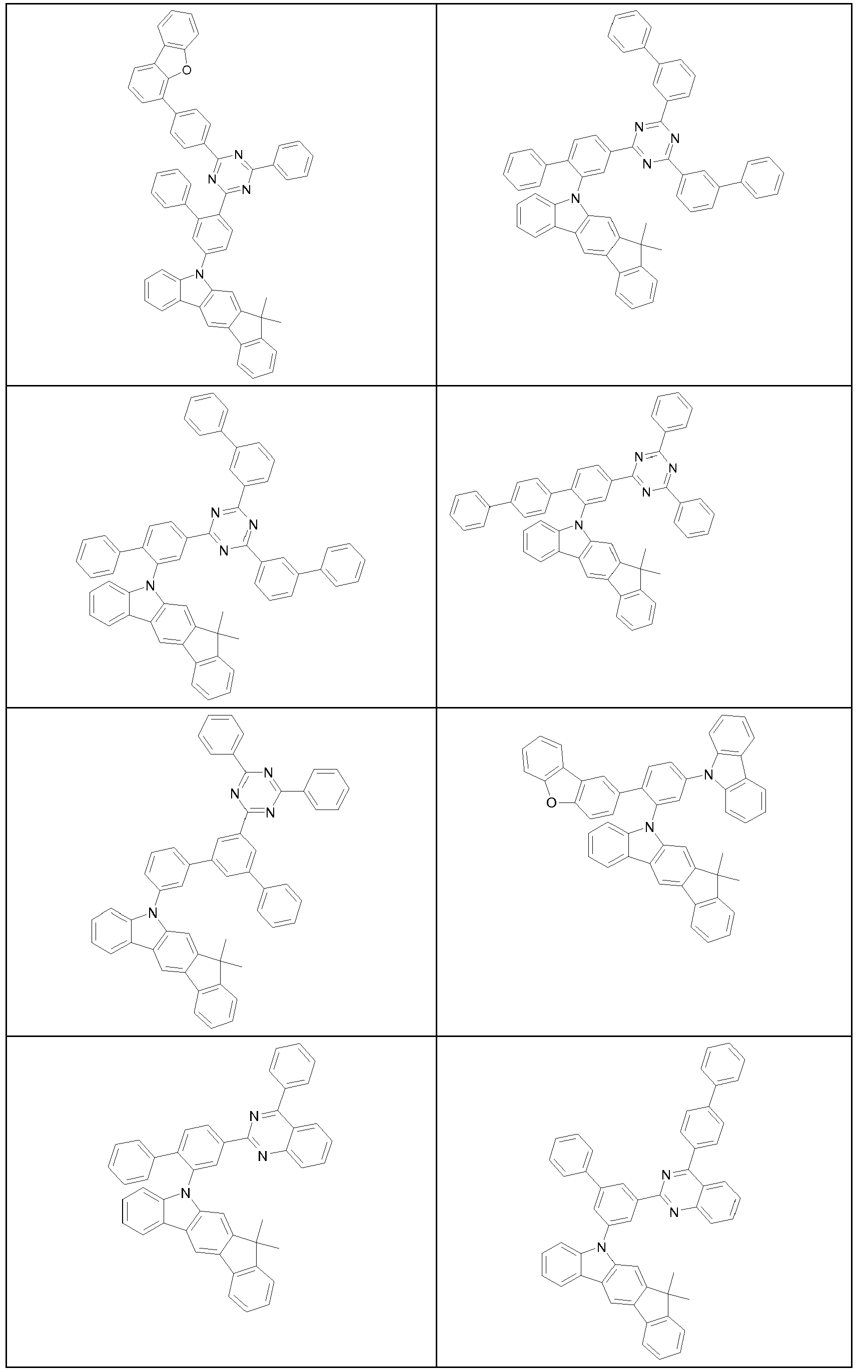

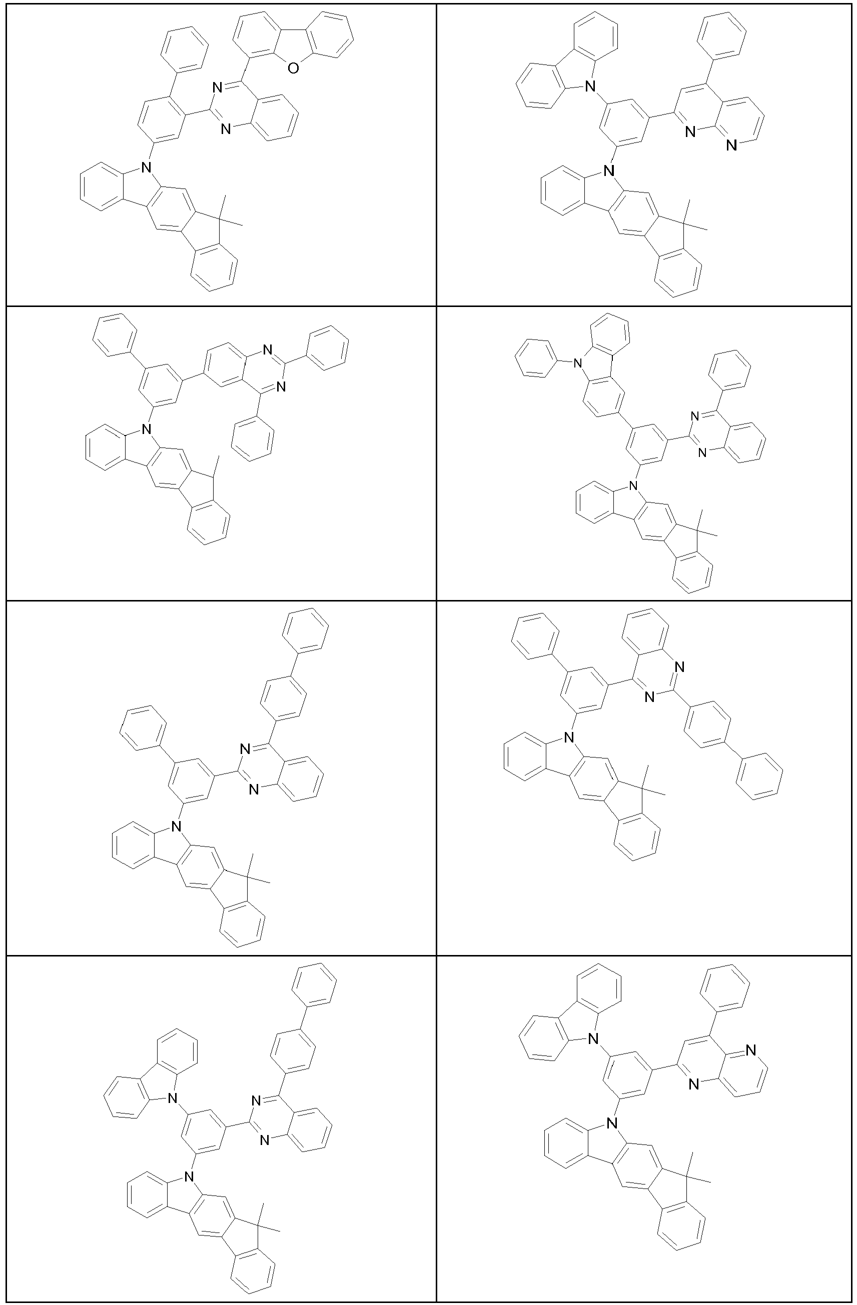

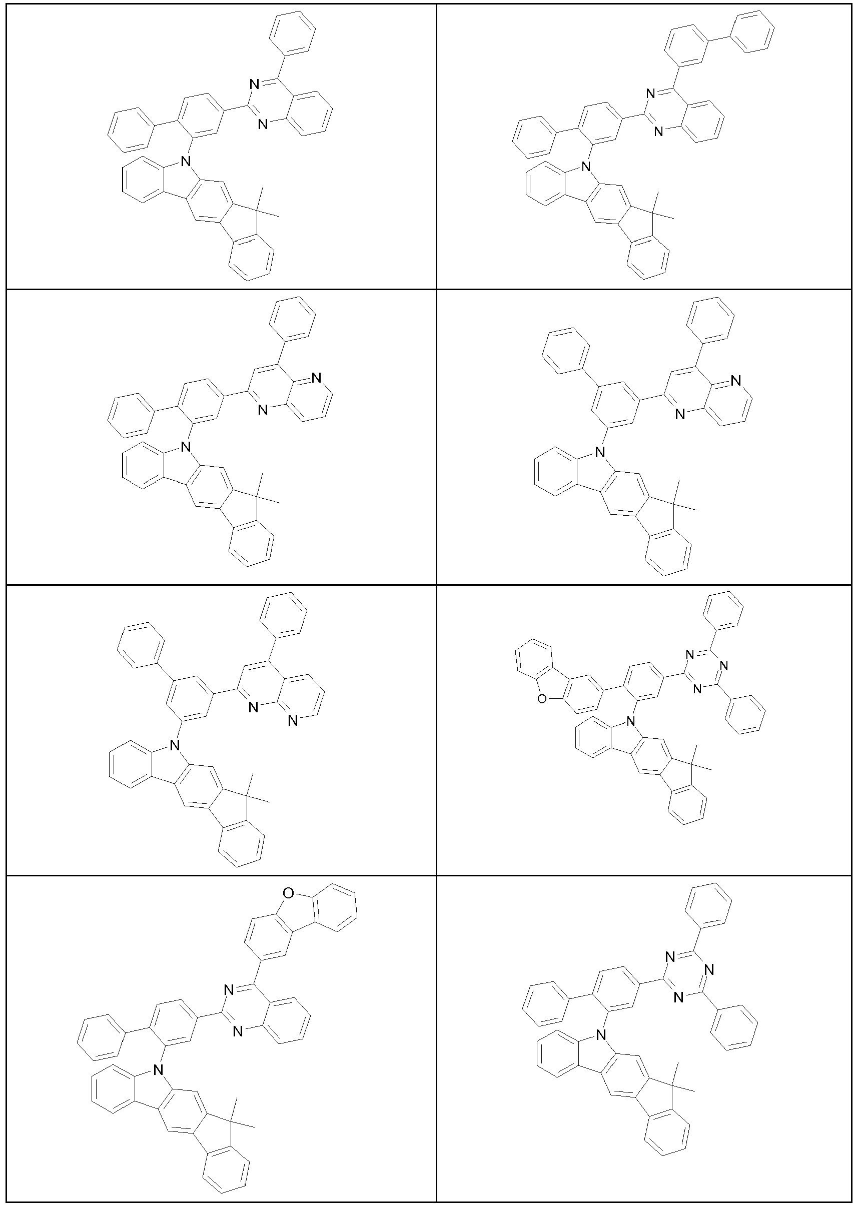

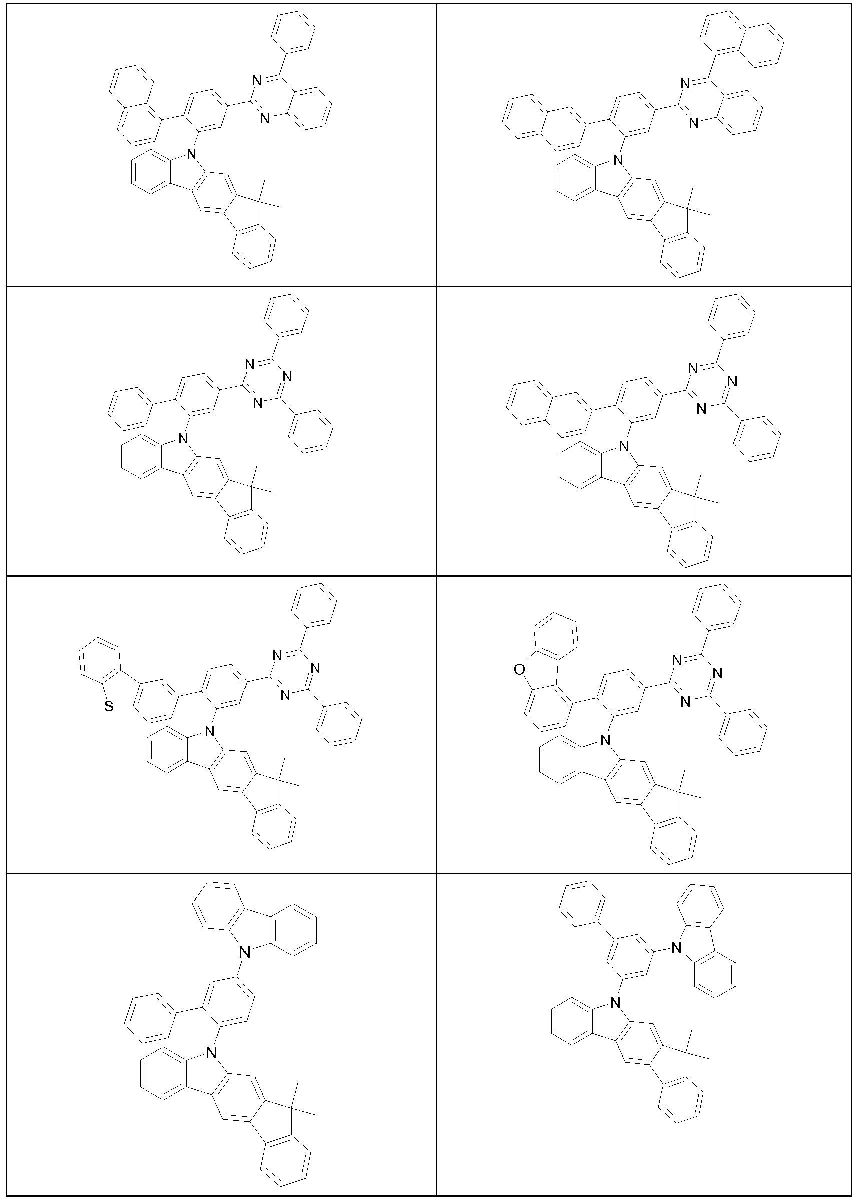

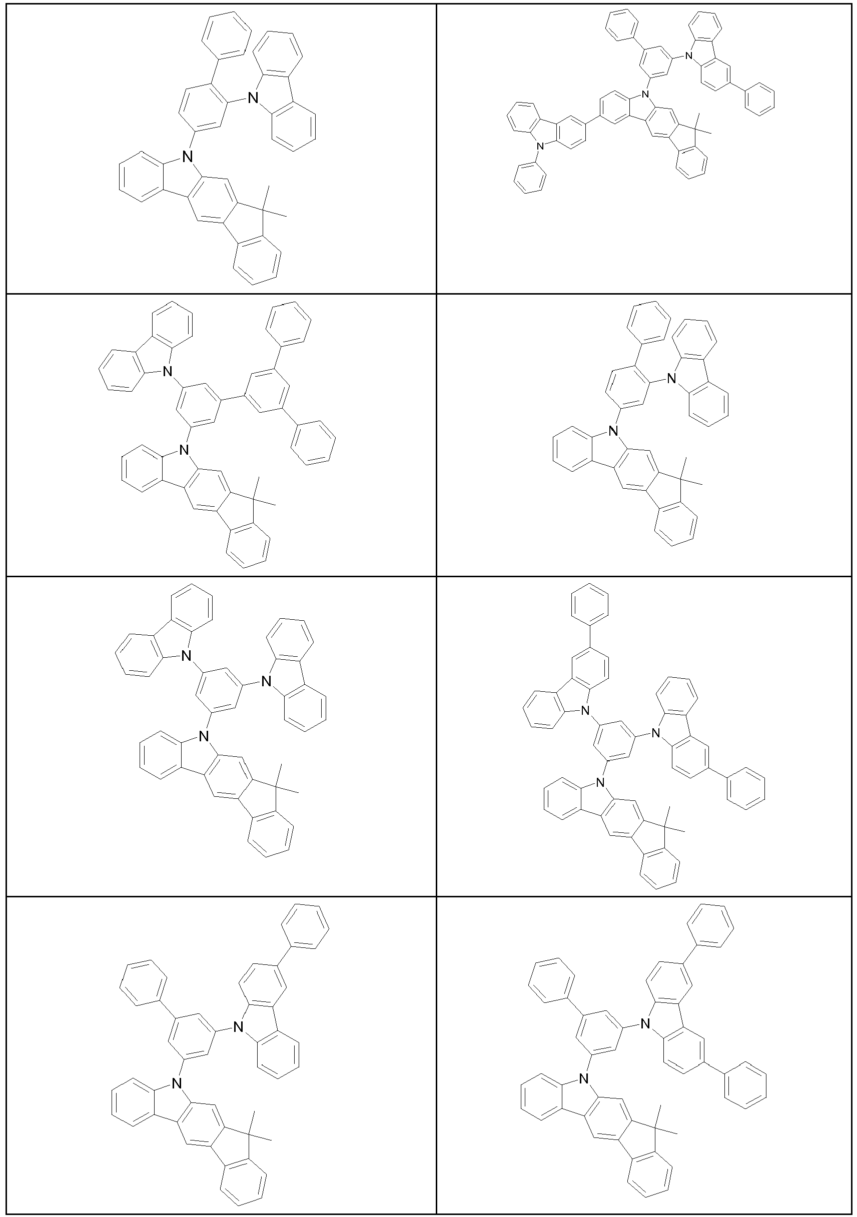

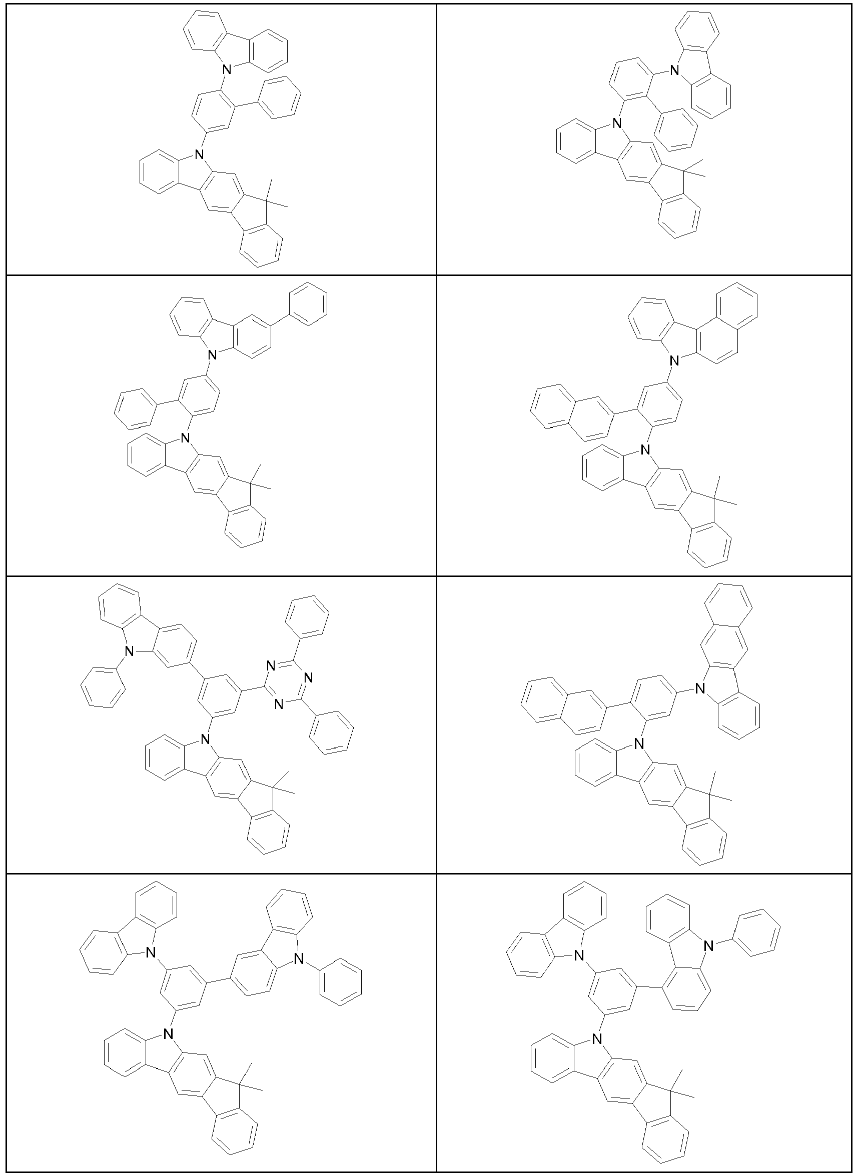

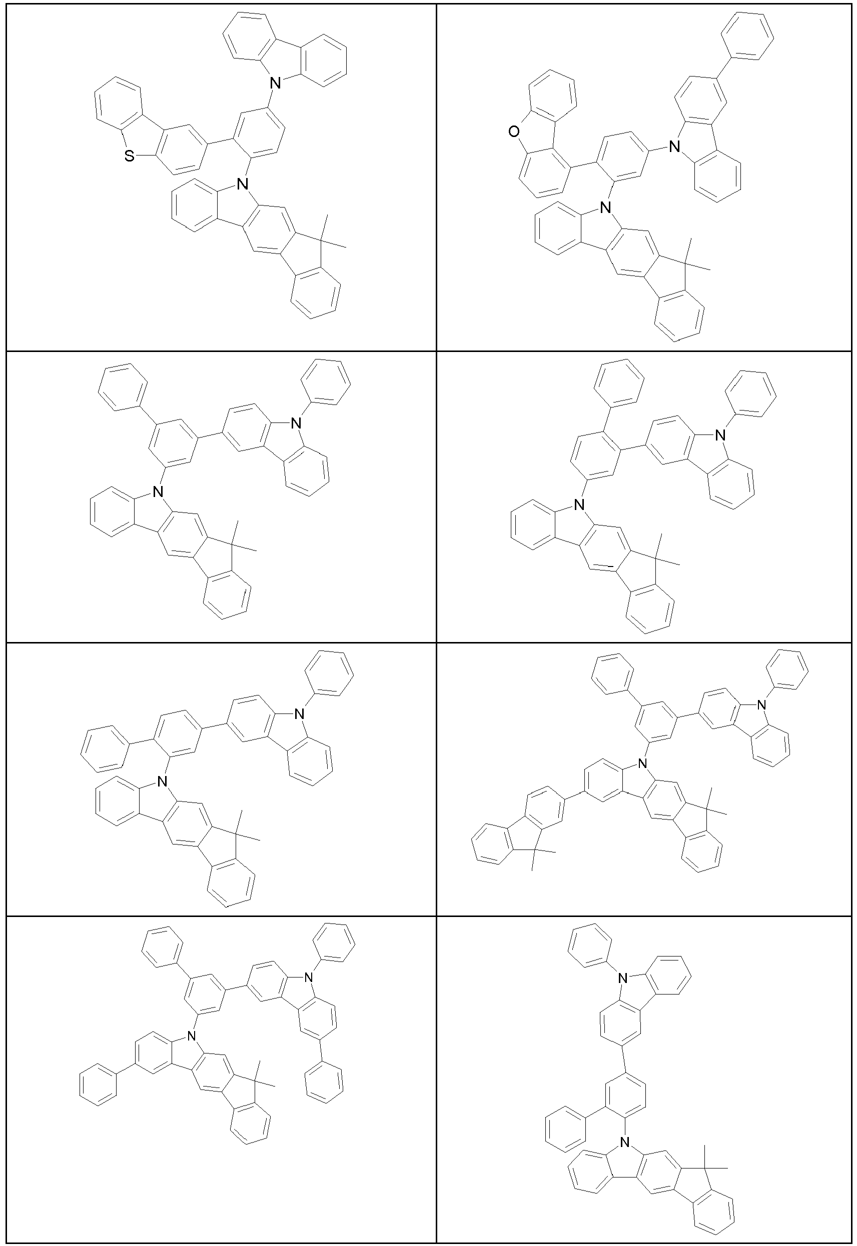

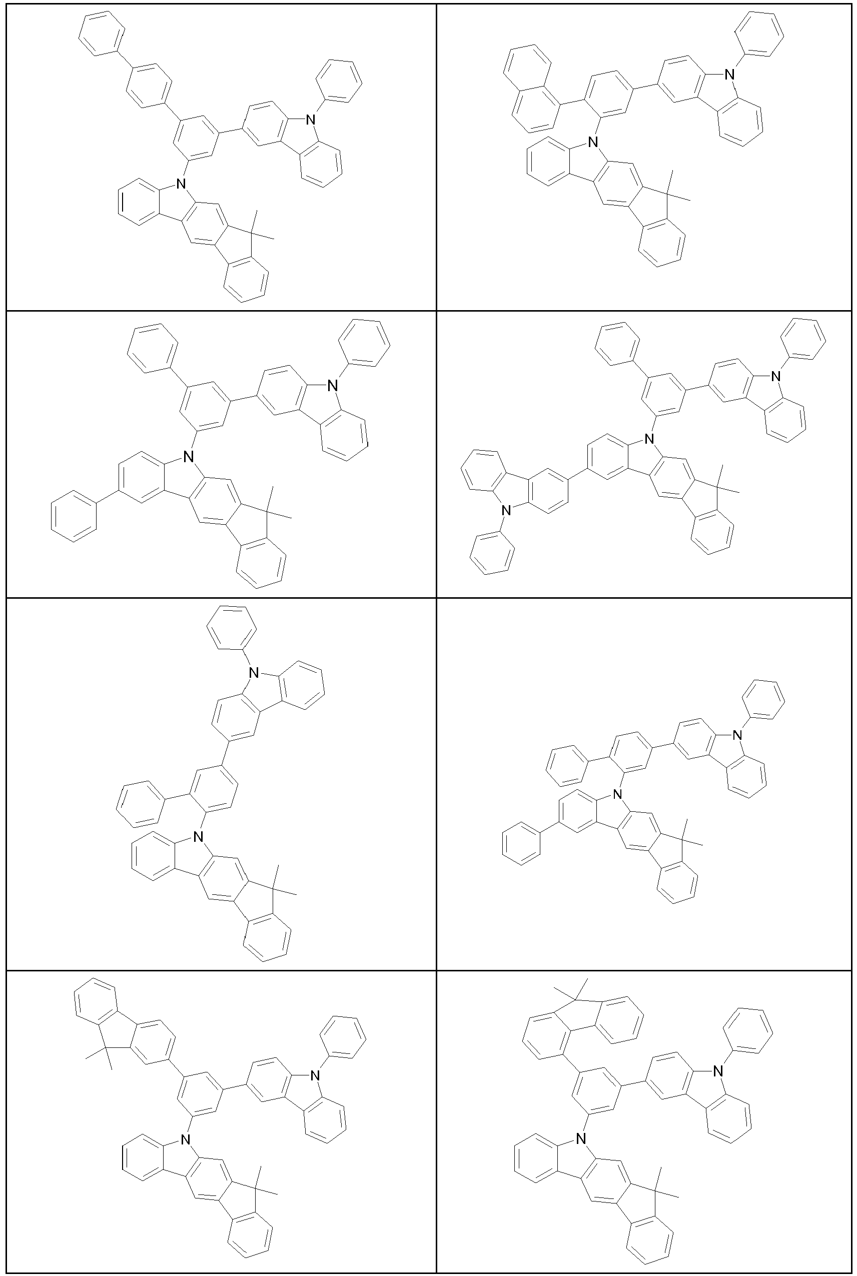

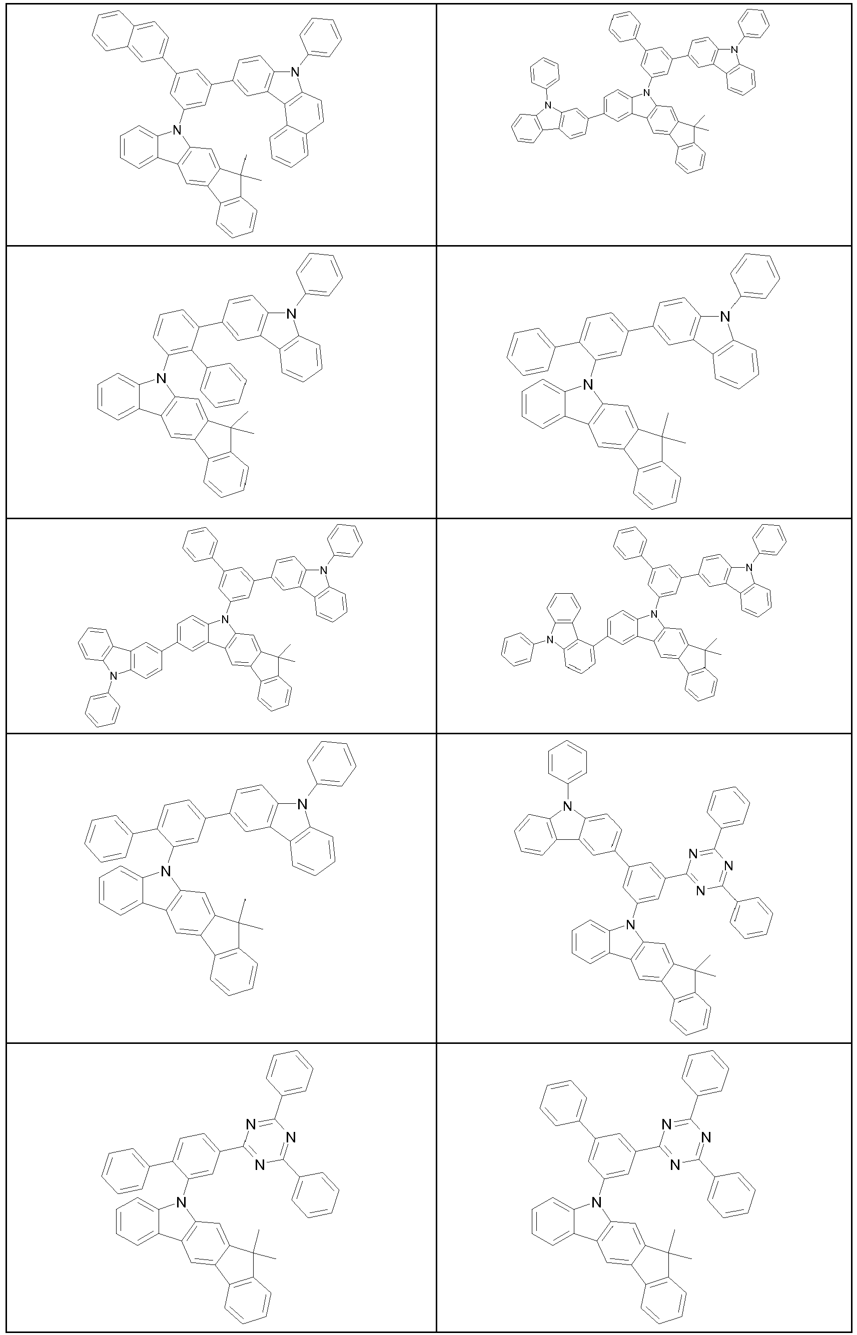

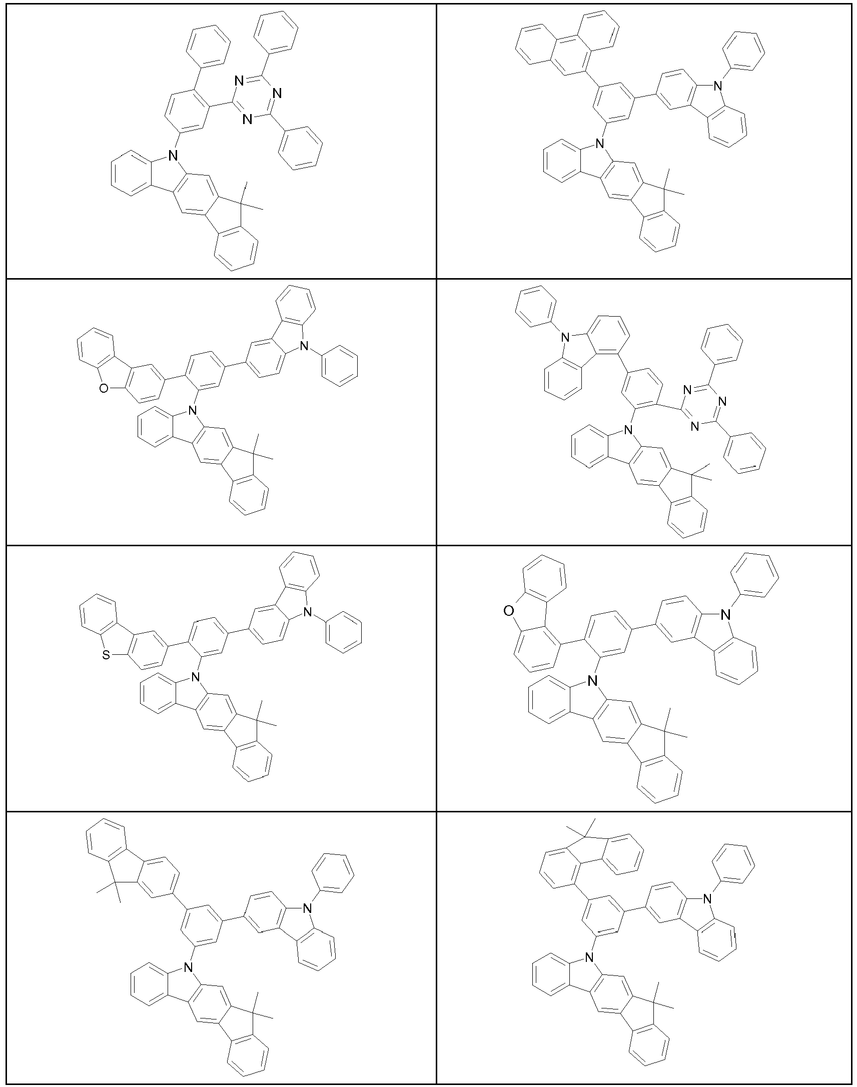

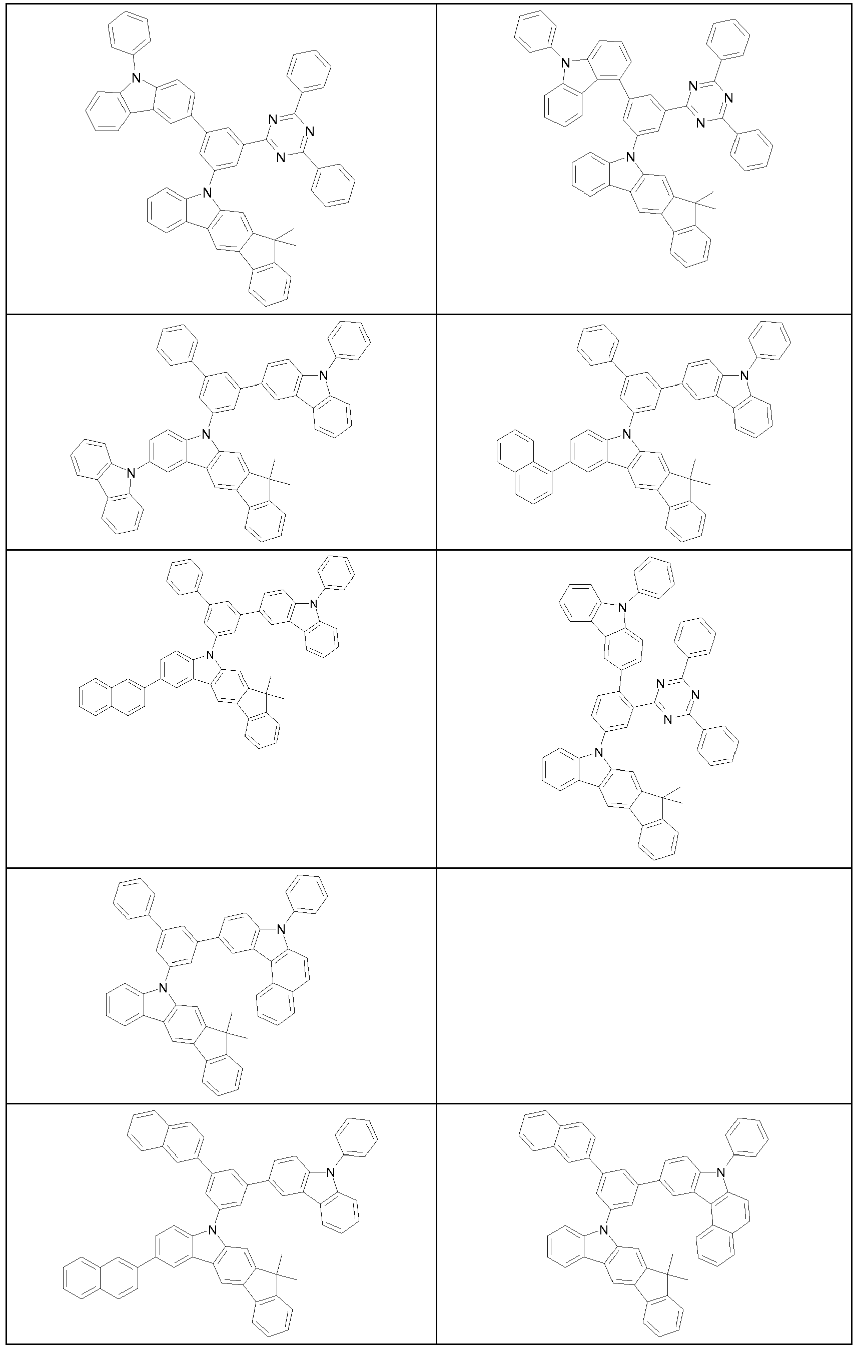

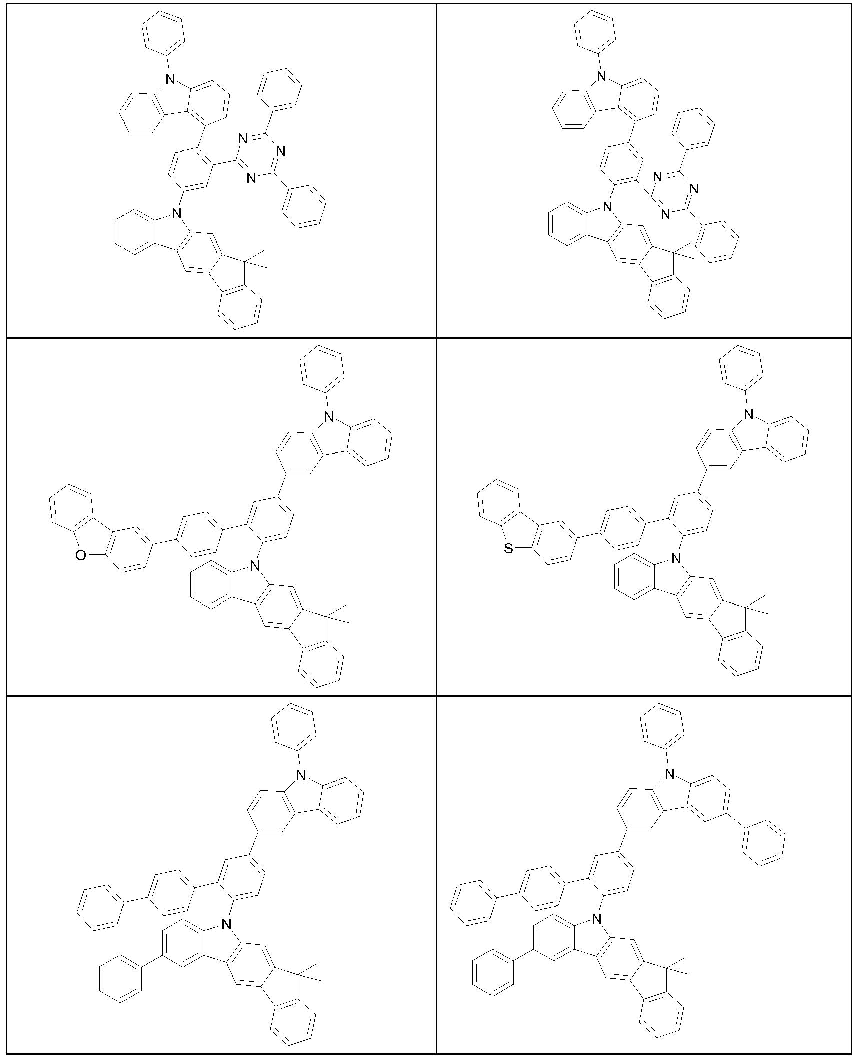

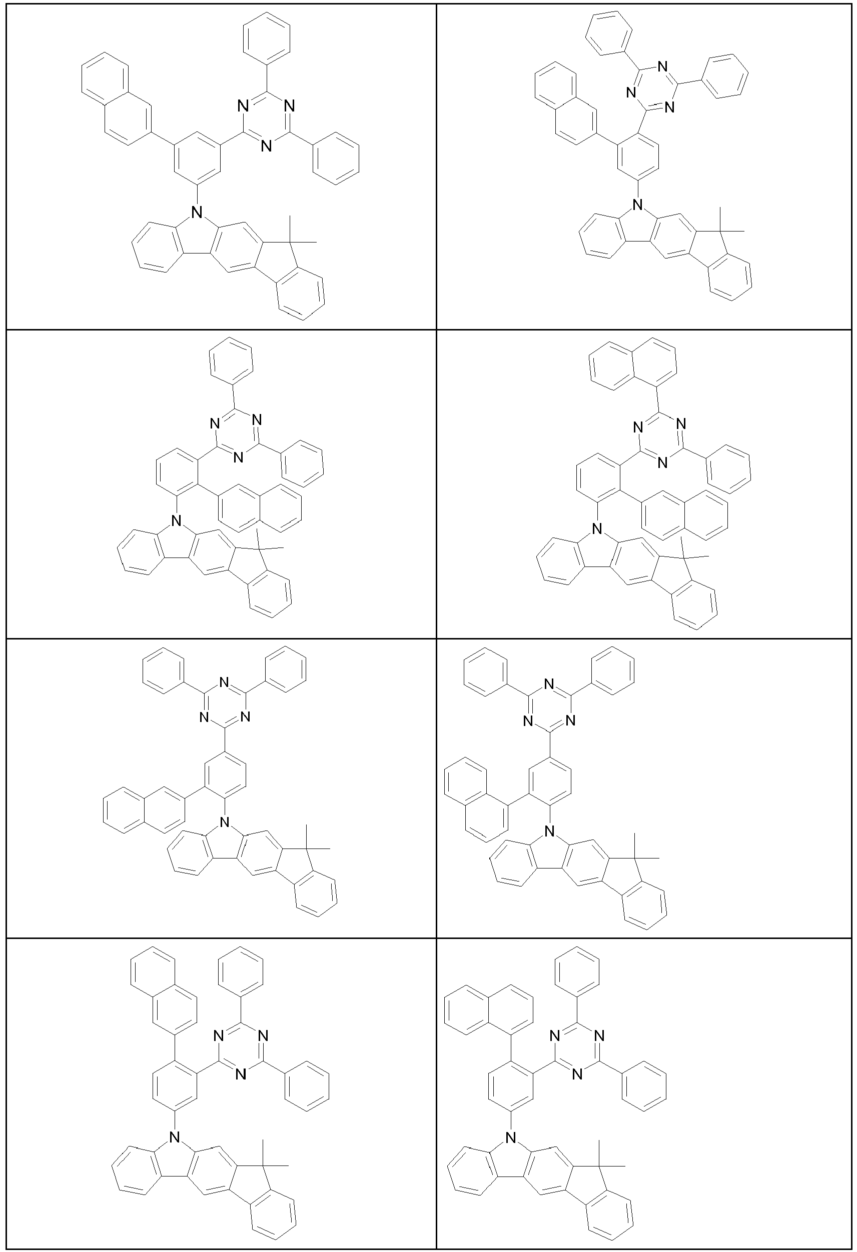

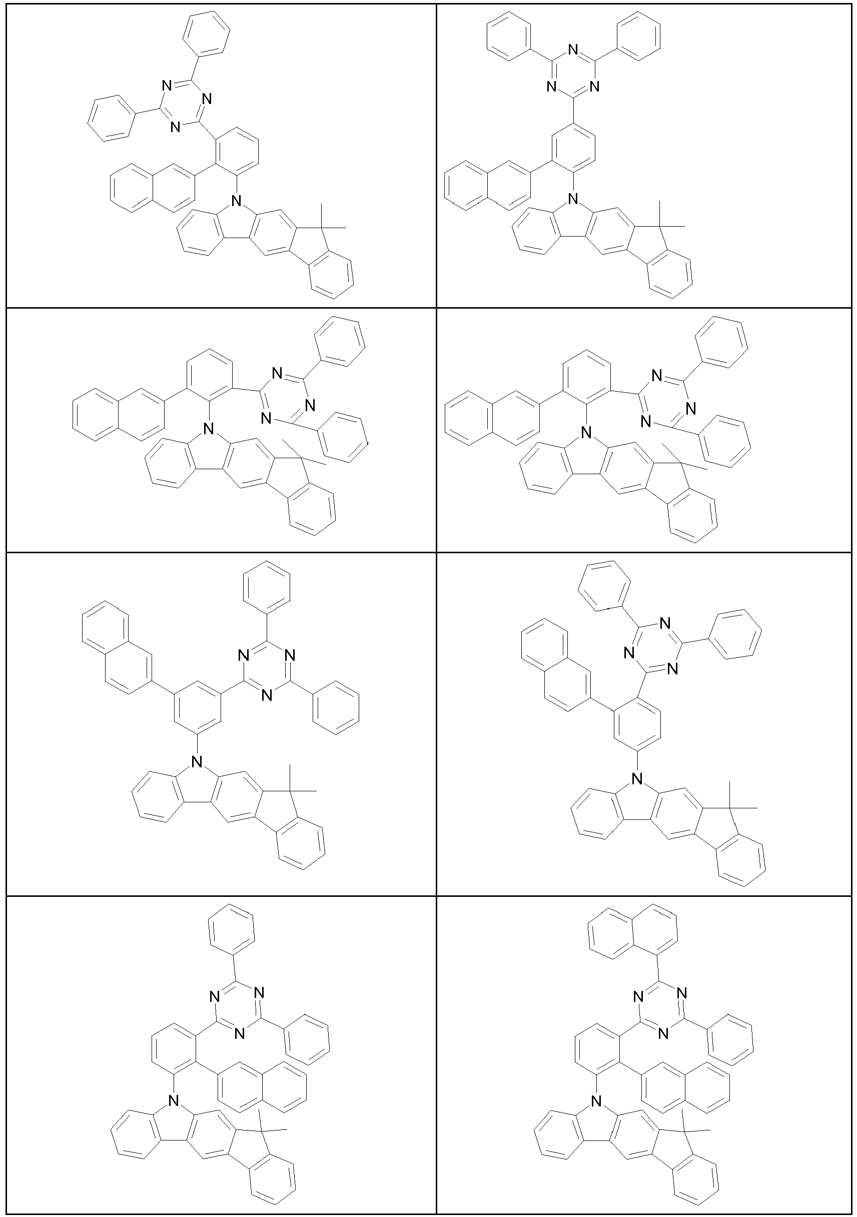

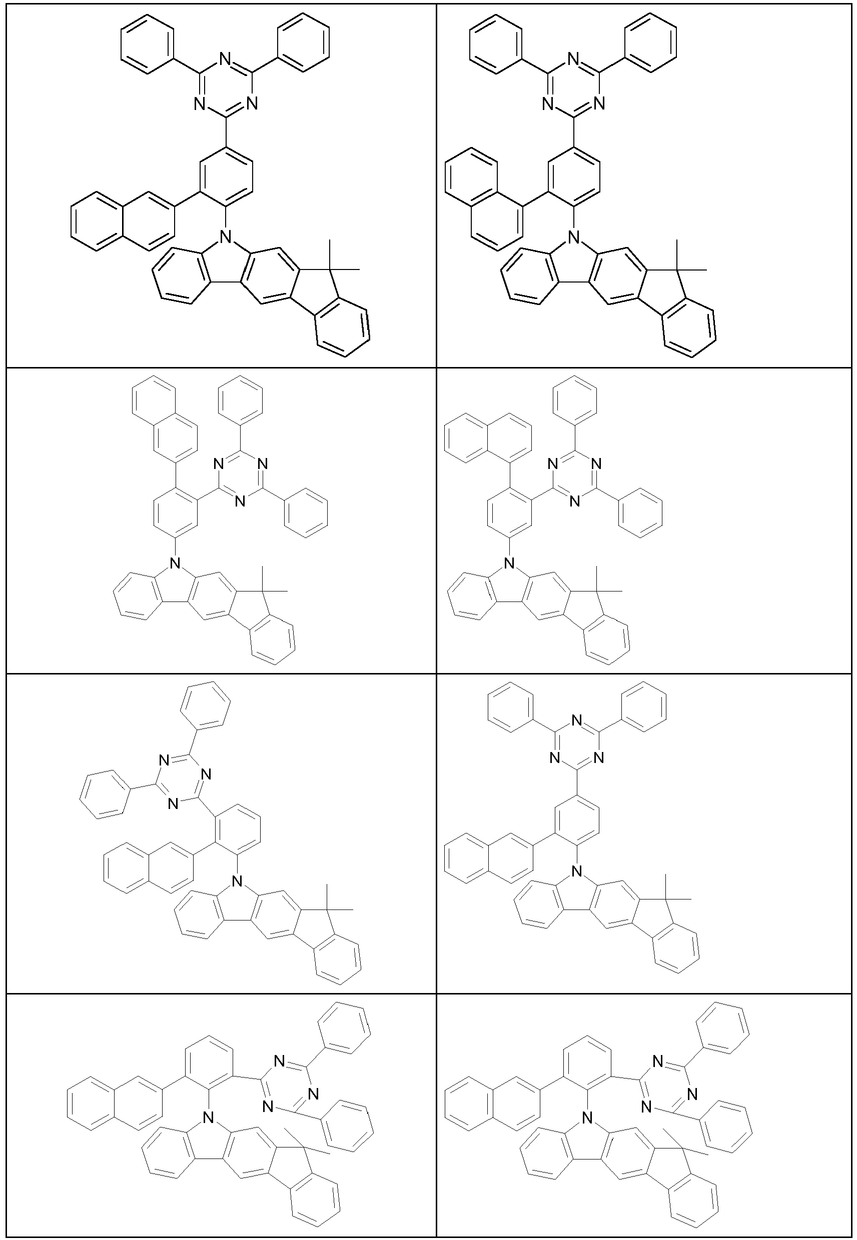

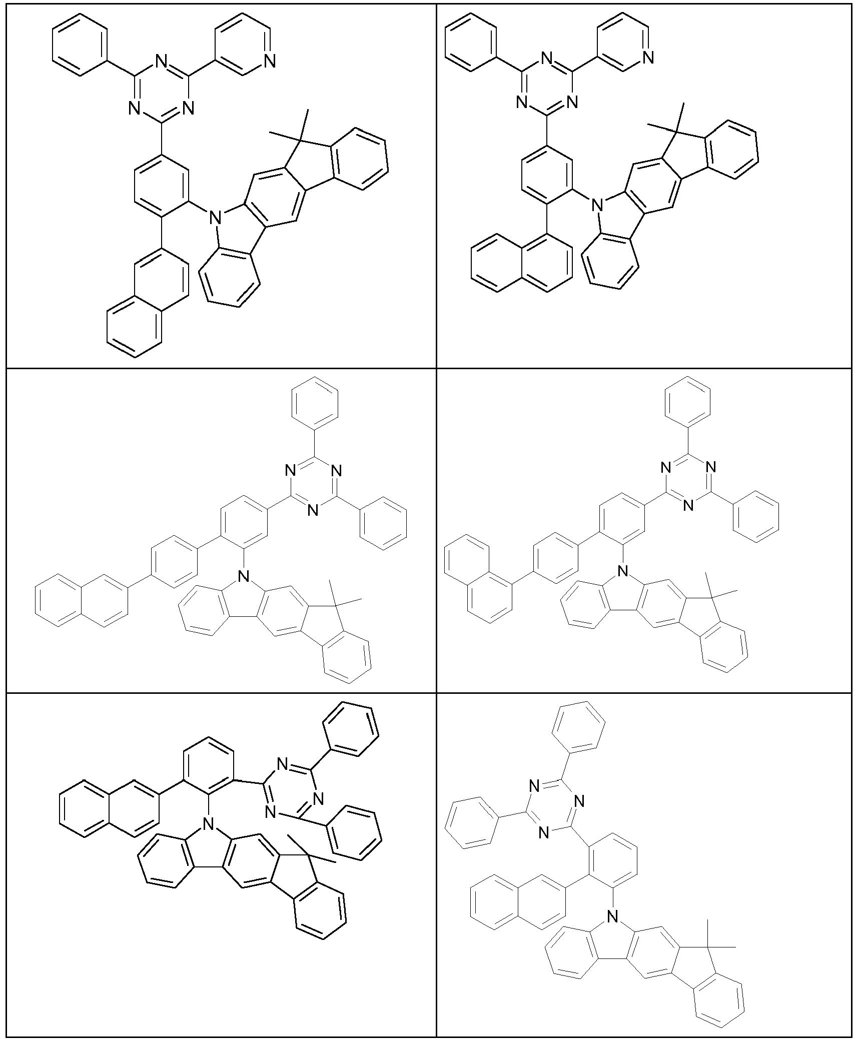

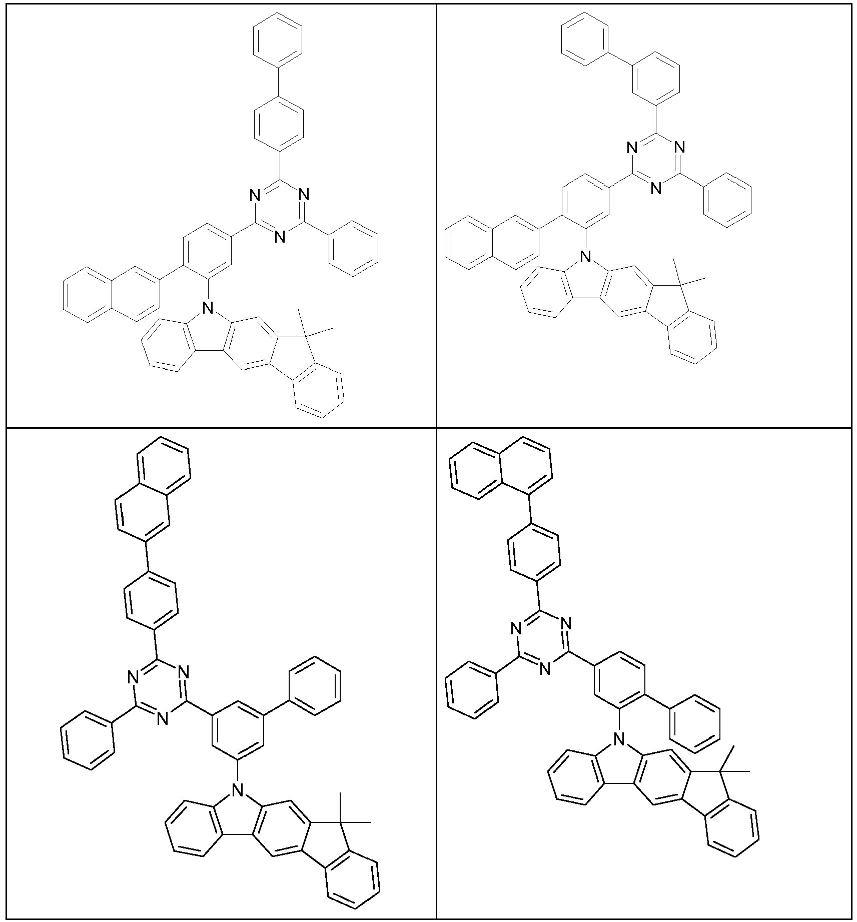

Die Synthese der erfindungsgemäßen Verbindungen kann auf den in den folgenden Schemata abgebildeten Synthesewegen erfolgen.

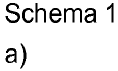

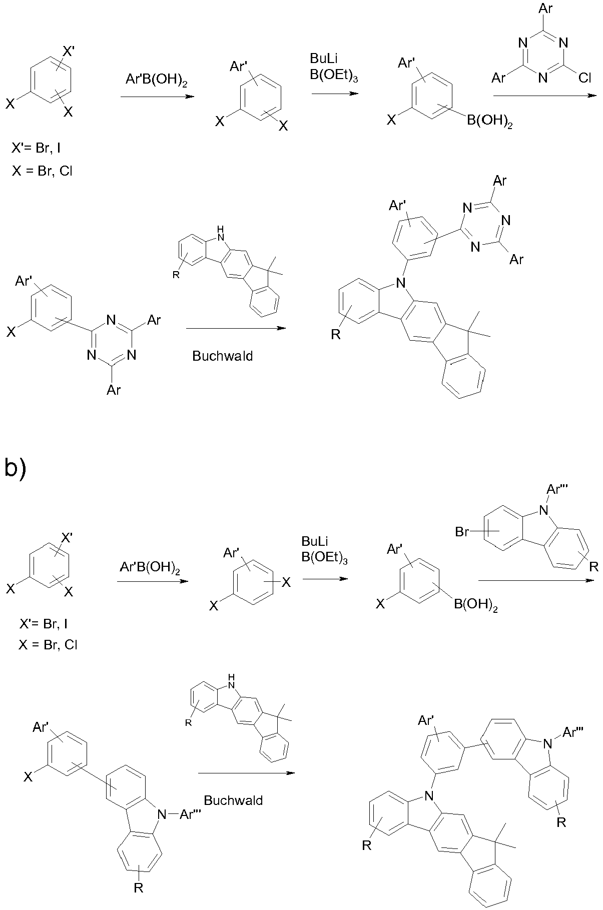

Dabei steht Ar' für eine Gruppe B. Statt der Triazingruppe können in Schema 1a) analog auch andere Gruppen (A-1) bis (A-4) eingesetzt werden. Ebenso können in Schema 1b) analog auch andere Carbazolgruppen bzw. Benzocarbazolgruppen eingesetzt werden.

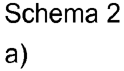

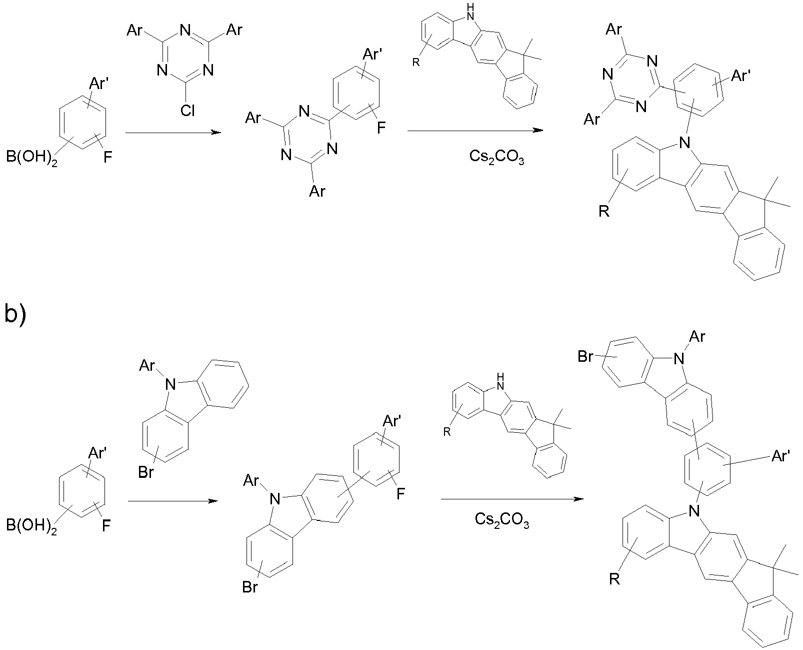

Dabei steht Ar' für eine Gruppe B. Statt der Triazingruppe können in Schema 2a) analog auch andere Gruppen (A-1) bis (A-4) eingesetzt werden. Ebenso können in Schema 2b) analog auch andere Carbazolgruppen bzw. Benzocarbazolgruppen eingesetzt werden.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Hexamethylindan, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung und/oder ein weiteres Matrixmaterial. Geeignete emittierende Verbindungen und weitere Matrixmaterialien sind hinten im Zusammenhang mit der organischen Elektrolumineszenzvorrichtung aufgeführt. Diese weitere Verbindung kann auch polymer sein.

Die erfindungsgemäßen Verbindungen eignen sich für die Verwendung in einer elektronischen Vorrichtung, insbesondere in einer organischen Elektrolumineszenzvorrichtung.

Ein weiterer Gegenstand der vorliegenden Erfindung ist daher die Verwendung einer erfindungsgemäßen Verbindung in einer elektronischen Vorrichtung, insbesondere in einer organischen Elektrolumineszenzvorrichtung.

Ein nochmals weiterer Gegenstand der vorliegenden Erfindung ist eine elektronische Vorrichtung enthaltend mindestens eine erfindungsgemäße Verbindung.

Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), farbstoffsensibilisierten organischen Solarzellen (DSSCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices", bevorzugt aber organischen Elektrolumineszenzvorrichtungen (OLEDs), welche phosphoreszierend oder fluoreszierend sein können.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Es kann sich bei der erfindungsgemäßen organischen Elektrolumineszenzvorrichtung auch um eine Tandem-OLED handeln, insbesondere auch für weiß emittierende OLEDs.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. die oben ausgeführten bevorzugten Ausführungsformen als Matrixmaterial für phosphoreszierende Emitter in einer emittierenden Schicht. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Wenn die erfindungsgemäße Verbindung als Matrixmaterial für eine phosphoreszierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der erfindungsgemäßen Verbindung und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der erfindungsgemäßen Verbindung bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 30 Vol.-% des Emitters bezogen auf die Gesamtmischung aus Emitter und Matrixmaterial.

Eine weitere bevorzugte Ausführungsform der vorliegenden Erfindung ist der Einsatz der erfindungsgemäßen Verbindung als Matrixmaterial für einen phosphoreszierenden Emitter in Kombination mit einem weiteren Matrixmaterial. Wenn die erfindungsgemäße Verbindung in Kombination mit einem weiteren Matrixmaterial eingesetzt wird, liegt deren Anteil bevorzugt bei 20 bis 50 Vol.-%, bezogen auf die Gesamtmischung. Geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Bis-carbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, oder Triphenylenderivate, z. B. gemäß WO 2012/048781. Ebenso kann ein weiterer phosphoreszierender Emitter, welcher kürzerwellig als der eigentliche Emitter emittiert, als Co-Host in der Mischung vorhanden sein oder eine Verbindung, die nicht oder nicht in wesentlichem Umfang am Ladungstransport teilnimmt, wie beispielsweise in WO 2010/108579 beschrieben.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 2005/033244, WO 2005/019373, US 2005/0258742, WO 2010/086089, WO 2011/044988, WO 2011/157339, WO 2012/007086, WO 2012/163471, WO 2013/000531 und WO 2013/020631, WO 2014/008982, WO 2014/023377, WO 2014/094962, WO 2014/094961, WO 2014/094960 und WO 2015/039723 entnommen werden. Weiterhin eignen sich beispielsweise die in den nicht offen gelegten Anmeldungen EP 14000345.0, EP 14000417.7, EP 14002623.8 und EP 15000307.7 offenbarten Metallkomplexe. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Beispiele für geeignete phosphoreszierende Verbindungen sind die in der folgenden Tabelle aufgeführten Metallkomplexe.

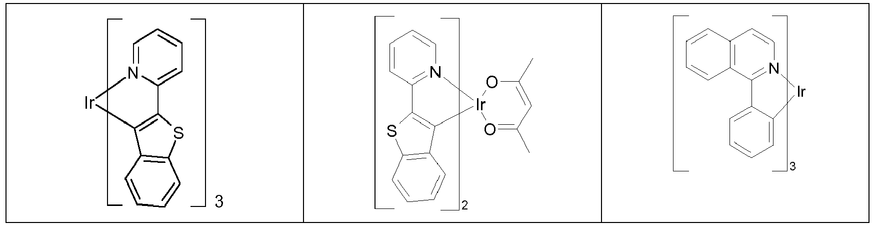

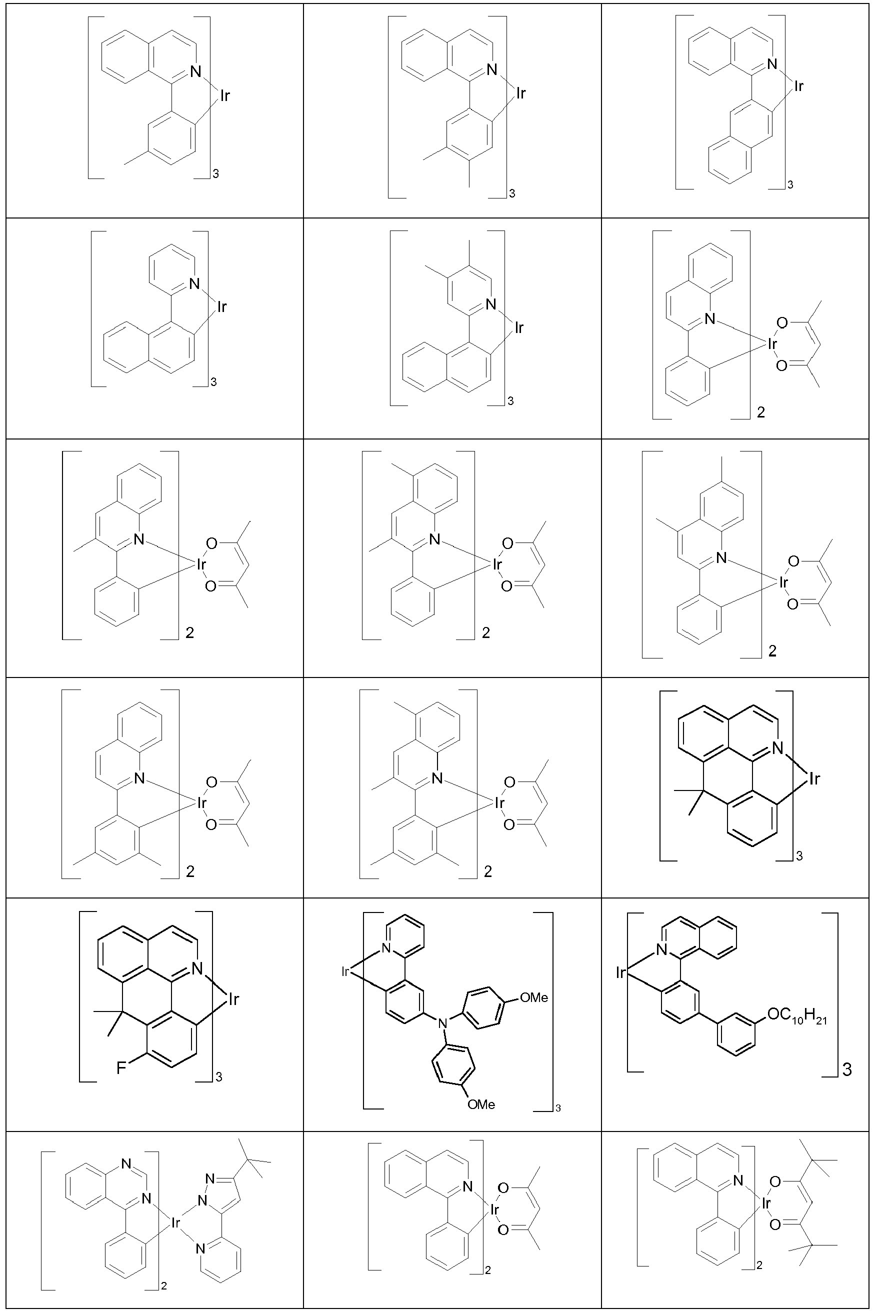

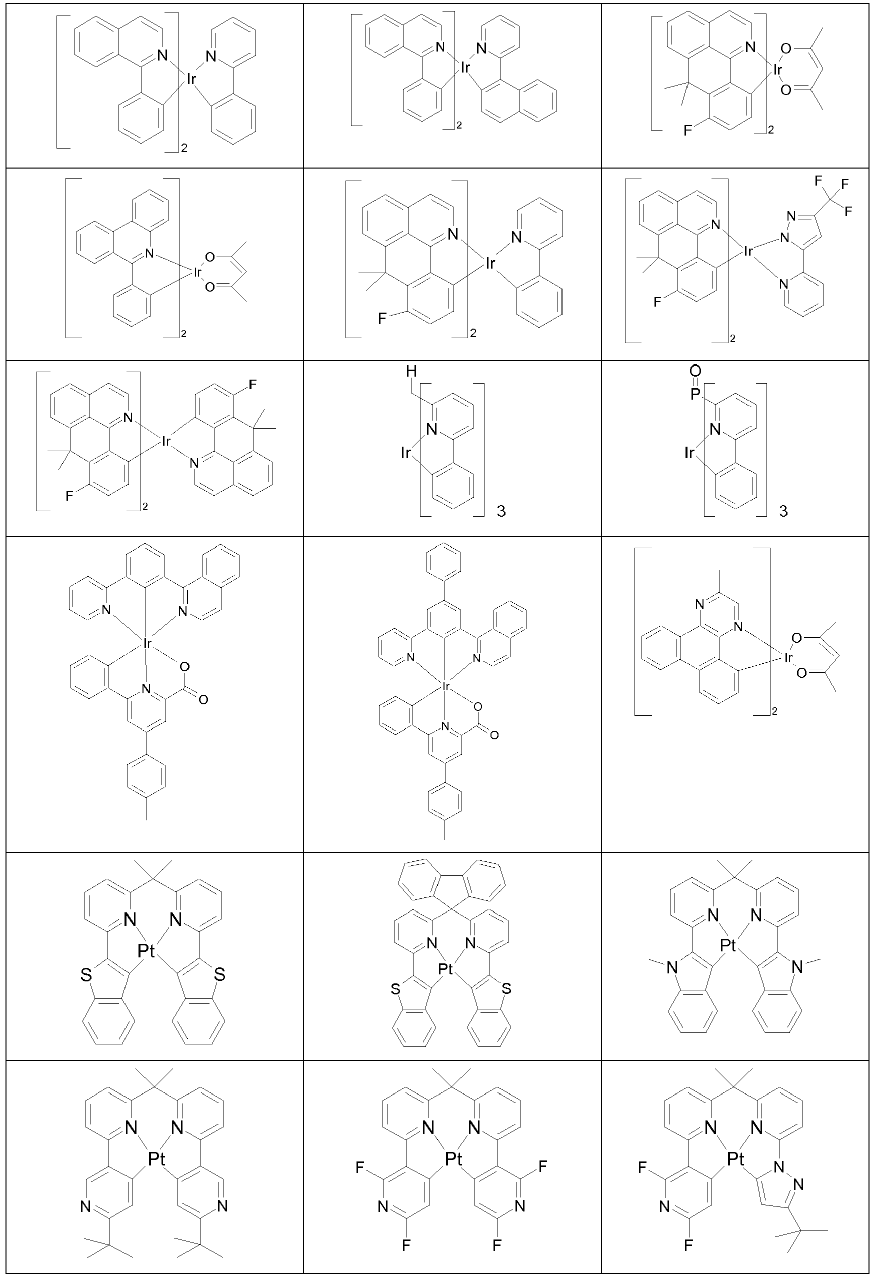

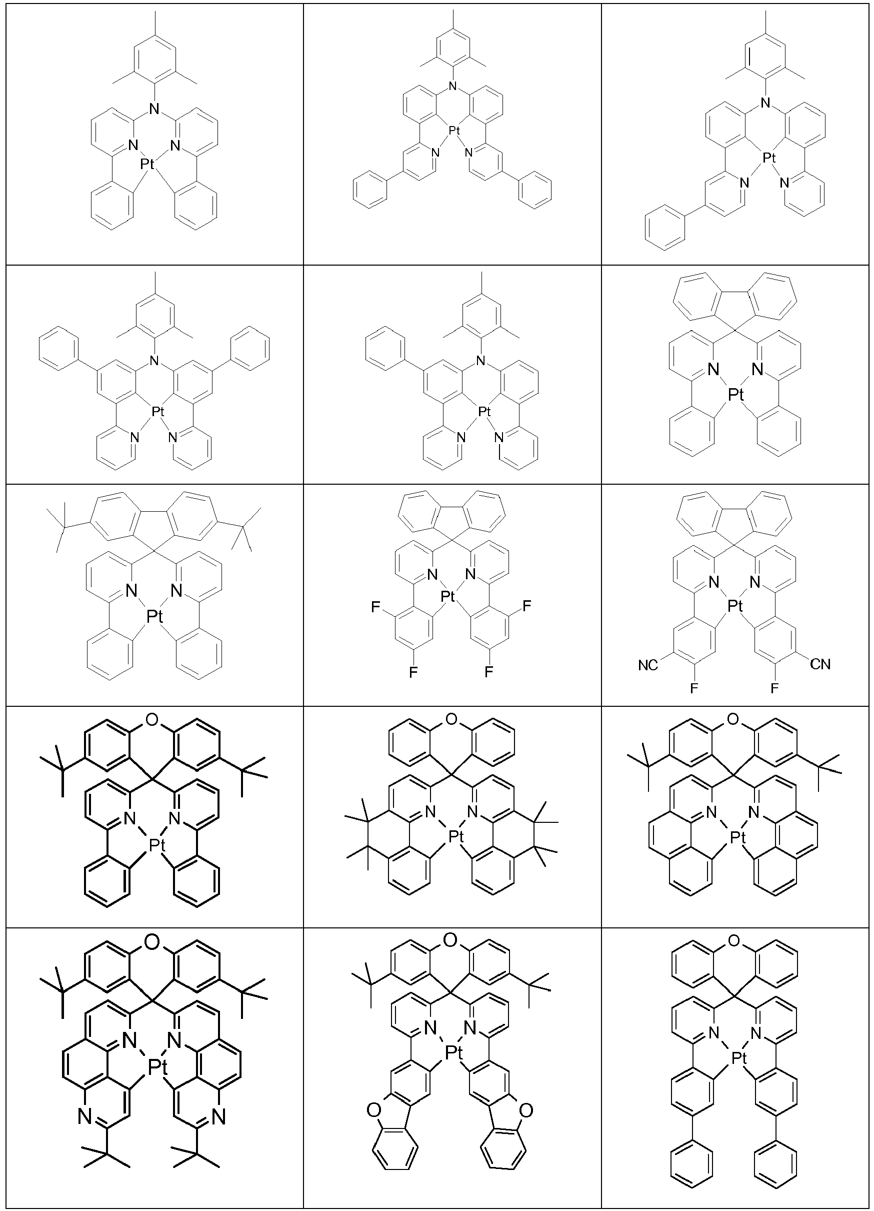

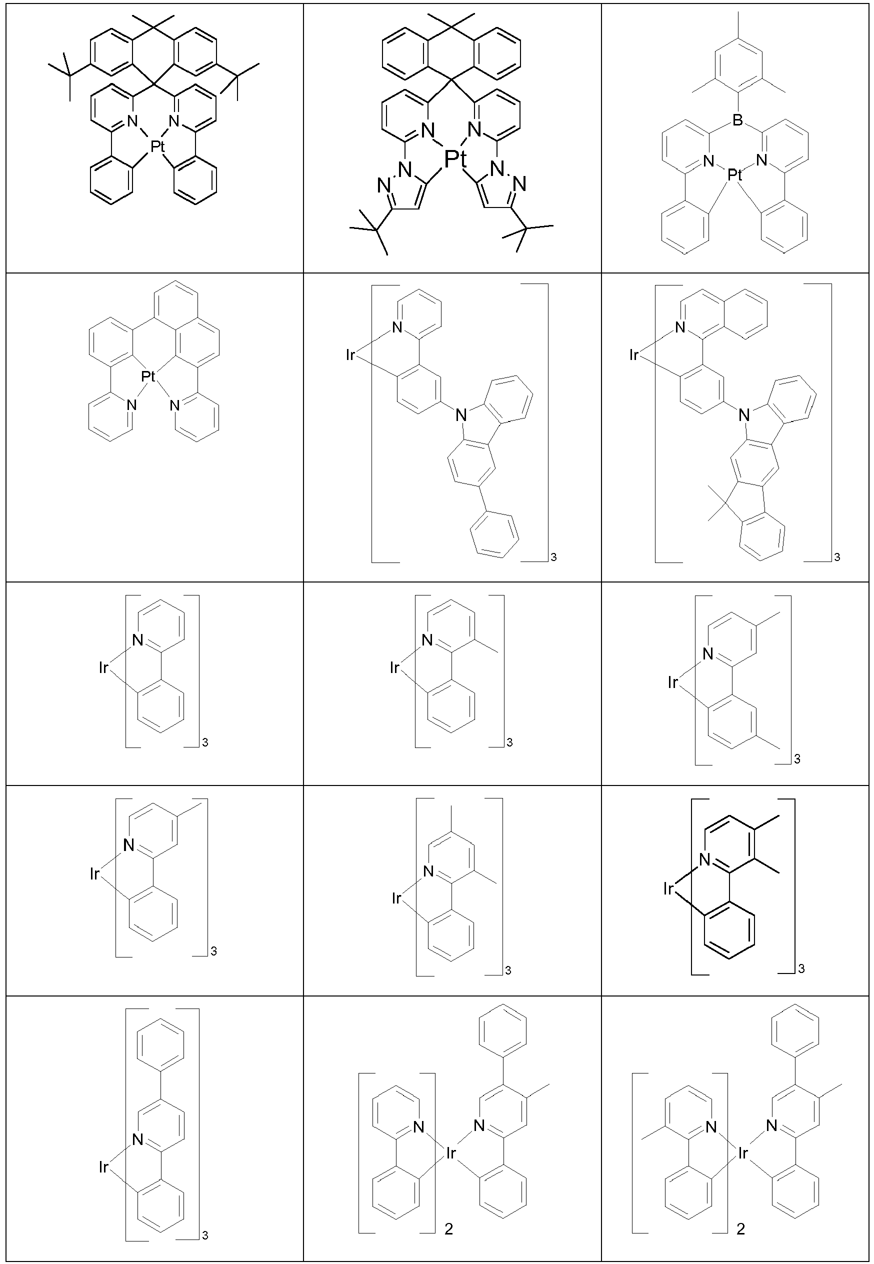

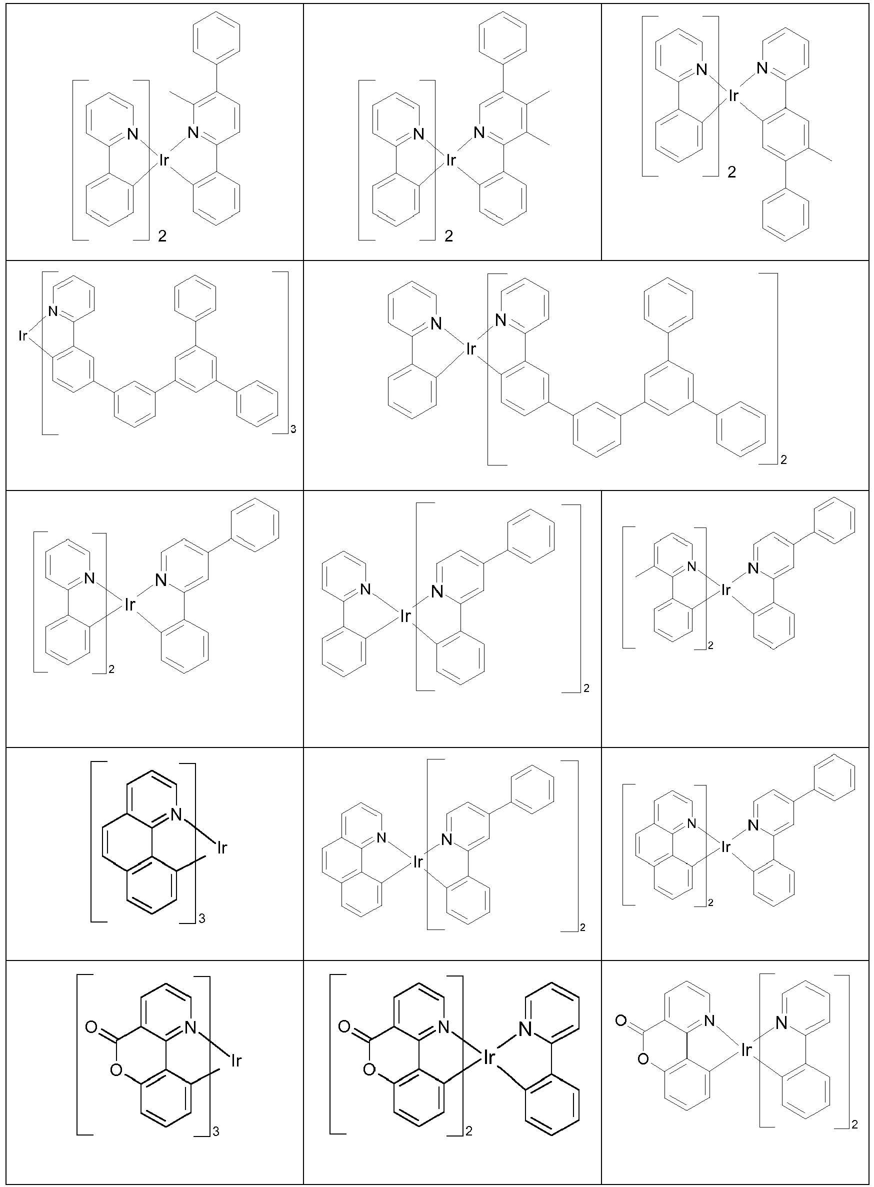

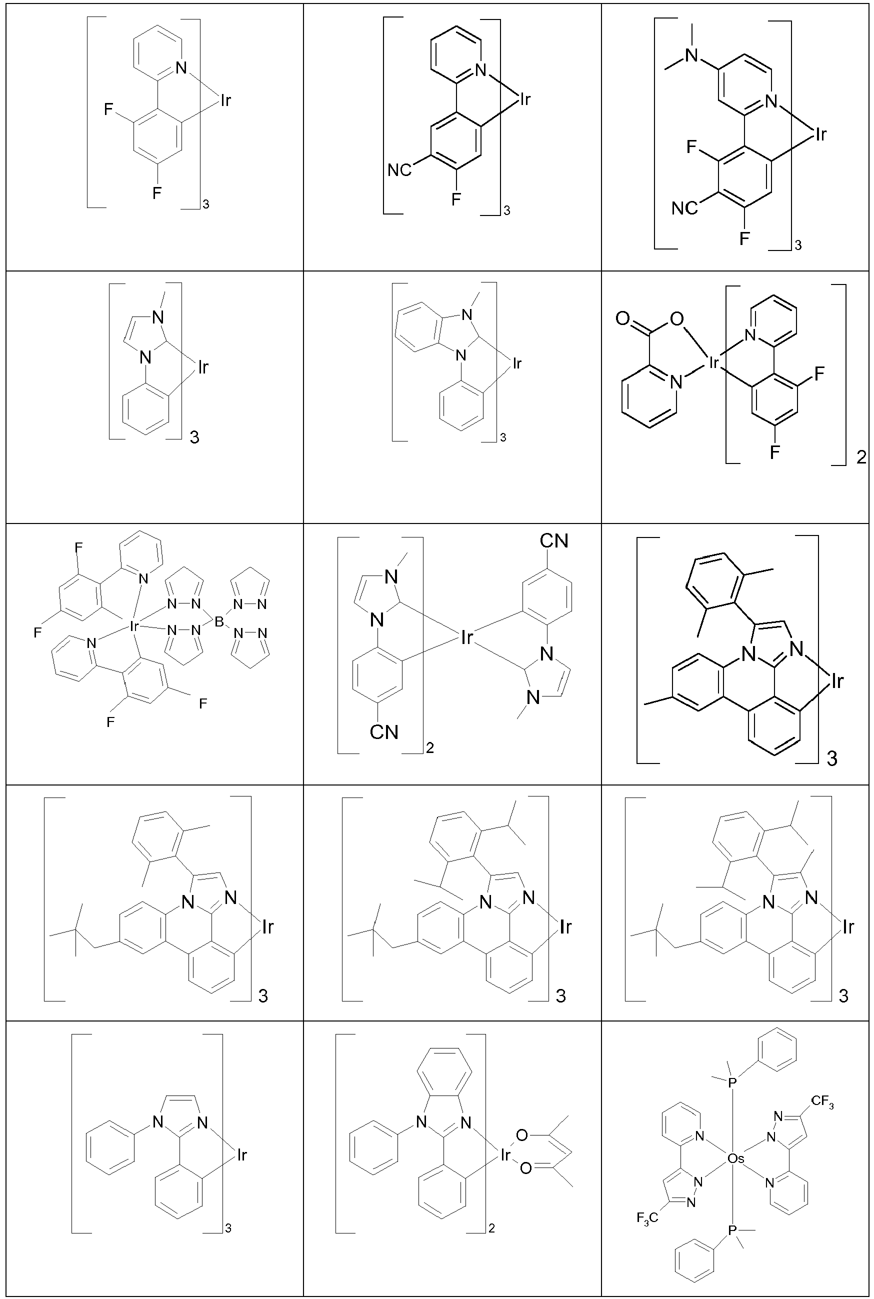

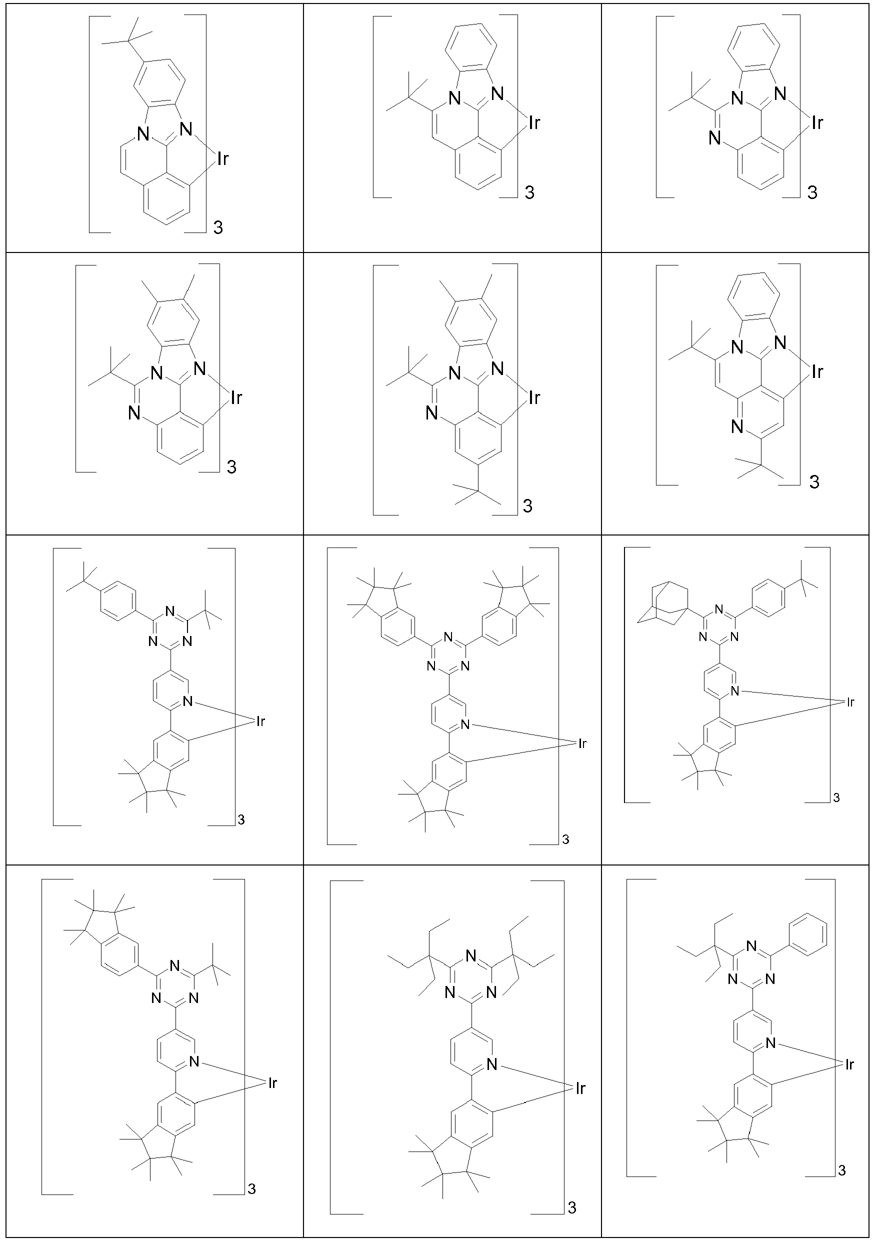

Die erfindungsgemäßen Verbindungen sind insbesondere auch geeignet als Matrixmaterialien für phosphoreszierende Emitter in organischen Elektrolumineszenzvorrichtungen, wie sie z. B. in WO 98/24271, US 2011/0248247 und US 2012/0223633 beschrieben sind. In diesen mehrfarbigen Display-Bauteilen wird eine zusätzliche blaue Emissionsschicht vollflächig auf alle Pixel, auch diejenigen mit einer von Blau verschiedenen Farbe, aufgedampft.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In einer weiteren bevorzugten Ausführungsform der Erfindung wird die erfindungsgemäße Verbindung in einer Lochblockierschicht oder eine Elektronentransportschicht eingesetzt. Dies gilt insbesondere für Materialien, in denen die Gruppe A ausgewählt ist aus den Gruppen (A-1) bis (A-4) bzw. den entsprechenden bevorzugten Ausführungsformen. Dabei kann es sich um eine fluoreszierende oder phosphoreszierende OLED handeln. Wenn die erfindungsgemäßen Verbindungen als Elektronentransportmaterial eingesetzt werden, werden sie bevorzugt entweder als Reinschicht oder in Mischung mit einem Lithiumsalz, insbesondere LiQ (Lithiumchinonlinat) eingesetzt. Wenn die erfindungsgemäße Verbindung als Reinschicht verwendet wird, kann es bevorzugt sein, wenn die OLED angrenzend an diese Schicht eine weitere Schicht bestehend aus einem Lithiumsalz, insbesondere LiQ, enthält.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich durch einen oder mehrere der folgenden überraschenden Vorteile gegenüber dem Stand der Technik aus:
1. Die erfindungsgemäßen Verbindungen, eingesetzt als Matrixmaterial für phosphoreszierende Emitter, führen zu verbesserten Lebensdauern im Vergleich zu den analogen Matrixmaterialien, die statt der Gruppe B ein Wasserstoffatom enthalten oder die statt der Indenocarbazolgruppe eine einfache Carbazolgruppe enthalten.
2. Die erfindungsgemäßen Verbindungen, eingesetzt als Lochblockier- bzw. Elektronentransportmaterial, führen zu einer verringerten Spannung und einer verbesserten Leistungseffizienz im Vergleich zu den analogen Elektronentransportmaterialien, die statt der Gruppe B ein Wasserstoffatom enthalten oder die statt der Indenocarbazolgruppe eine einfache Carbazolgruppe enthalten. Dies gilt insbesondere für erfindungsgemäße Verbindungen, in denen die Gruppe A eine elektronenarme Gruppe, also eine Gruppe gemäß einer der Formeln (A-1) bis (A-4) darstellt.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

### Synthesebeispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können von ALDRICH bzw. ABCR bezogen werden. Die bei den nicht kommerziell erhältlichen Edukten angegeben Nummern sind die entsprechenden CAS-Nummern. Die Verbindungen (I), (II), (IX), XIII), (XIV), (XIX), (XXI) und (XXVI) sind kommerziell erhältlich. Die Verbindungen (XV), (XXII) und (XXVIII) können gemäß Syn. Let, 2011, 12, 1761 synthetisiert werden. Die Verbindungen (XII), (XVI), (XXIII) und (XXIX) können gemäß Chem. Eur. J, 2009, 15, 5482 synthetisiert werden. Die Angaben in eckigen Klammern sind die CAS-Nummern der Verbindungen.

### Beispiel 1: Synthese von Verbindung (VIII)

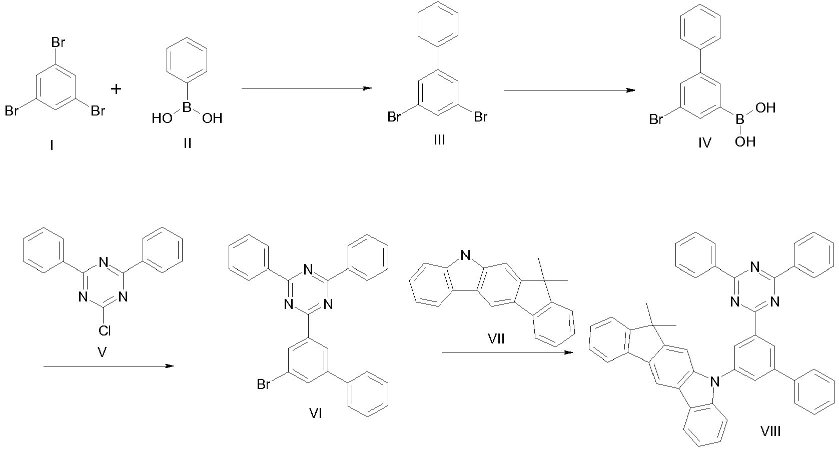

### Synthese von Verbindung (III)

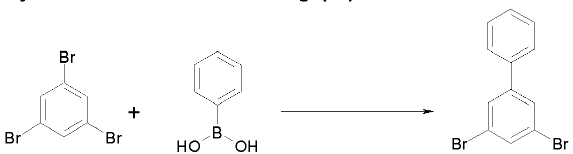

33.0 g (271.0 mmol) Phenyllboronsäure, 100 g (318 mmol) 1,3,5-Tribrombenzol und 56.2 g (530 mmol) einer wässrigen 2M Natriumcarbonatlösung werden in 540 ml Ethanol und 1620 ml Toluol unter Argonatmosphäre suspendiert. 7.3 g (6.0 mmol) Tetrakis(triphenylphosphin)-palladium(0) werden zugegeben und gerührt. Das Reaktionsgemisch wird unter Rückfluss über Nacht gerührt. Nachdem das Gemisch abgekühlt ist, wird die organische Phase abgetrennt, dreimal mit 300 ml Wasser gewaschen, über MgSO₄ getrocknet, filtriert und das Lösungsmittel im Vakuum entfernt. Der Rückstand wird durch Säulenchromatographie über Kieselgel (Eluent: Heptan) gereinigt. Die Ausbeute beträgt 45.9 g (147 mmol), entsprechend 46 % der Theorie.

### Synthese von Verbindung (IV)

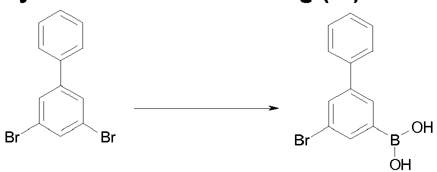

45.9 g (147 mmol) des Moleküls (III) werden in 920 ml Diethylether unter Argonatmosphäre suspendiert. Das Gemisch wird auf -78 °C gekühlt, wobei 59.4 ml (149 mmol/ 2.5M in Hexan) Butyllithium tropfenweise zugegeben werden, und das Gemisch wird 2 h bei -78 °C gerührt. 22.9 g (221 mmol) Trimethylborat werden anschließend bei -78 °C tropfenweise zugegeben, und die Mischung wird über Nacht bei Raumtemperatur gerührt. 200 ml 2N- Salzsäure werden zugegeben und 1 h gerührt. Die organische Phase wird abgetrennt, dreimal mit 300 ml Wassergewaschen, über MgSO₄ getrocknet, filtriert und das Lösungsmittel im Vakuum entfernt. Die Ausbeute beträgt 29.8 g (108 mmol), entsprechend 41 % der Theorie.

Analog dazu werden die folgenden Verbindungen hergestellt:

| | **Edukt 1** | **Produkt** | **Ausbeute** |
|---|---|---|---|
| **(IV)a** | 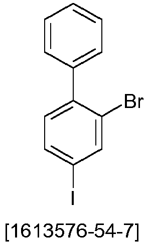 | 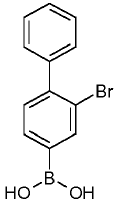 | 50% |
| **(IV)b** | 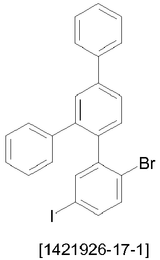 | 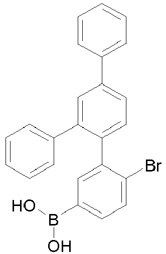 | 43% |
| **(IV)c** | 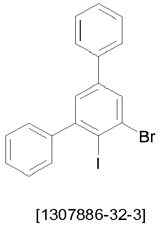 | 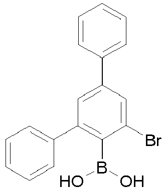 | 42% |
| **(IV)d** | 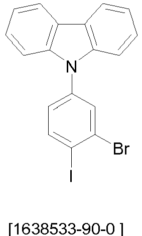 | 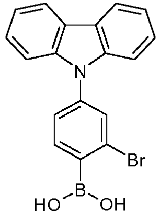 | 41% |

### Synthese von Verbindung (VI)

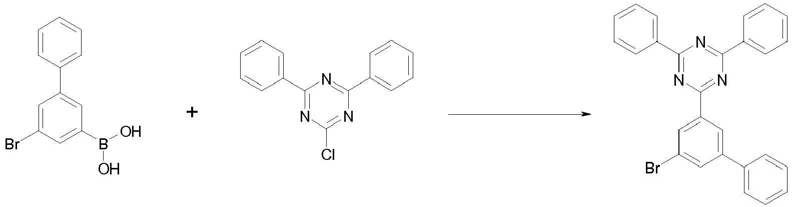

28 g (101 mmol) des Moleküls (IV), 27.1 g (101 mmol) 2-Chlor-4,6-diphenyl-1,3,5-triazin und 11.8 g (111 mmol) Natriumcarbonat werden in 800 ml 1,4-Dioxan, 800 ml Wasser und 250 ml Toluol gelöst und unter Argonatmosphäre gerührt. 1.2 g (1 mmol) Tetrakis(triphenylphosphin)-palladium werden zugegeben. Das Reaktionsgemisch wird unter Rückfluss über Nacht gerührt. Nach dem Abkühlen wird das Gemisch gequecht. Die organische Phase wird abgetrennt, dreimal mit 300 ml Wasser gewaschen, über MgSO₄ getrocknet, filtriert und das Lösungsmittel im Vakuum entfernt. Der Rückstand wird durch Säulenchromatographie über Kieselgel (Eluent: DCM/ Heptan (1:10) gereinigt. Die Ausbeute beträgt 24.6 g (53 mmol), entsprechend 52 % der Theorie.

Analog dazu werden die folgenden Verbindungen hergestellt:

| | **Edukt 1** | **Edukt 2** | **Produkt** | **Ausbeute** |
|---|---|---|---|---|
| **(VI)a** | 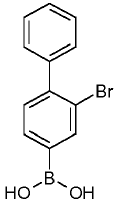 | 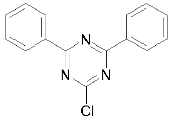 | 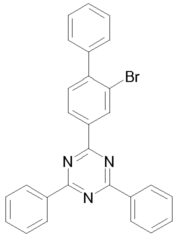 | 61% |
| **(VI)b** | 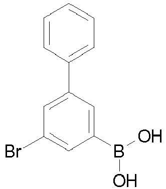 | 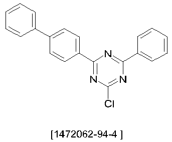 | 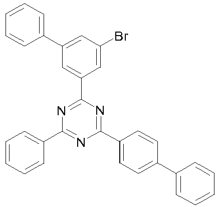 | 53% |
| **(VI)c** | 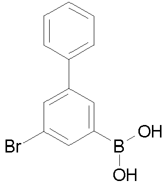 | 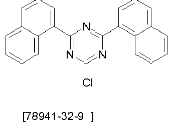 | 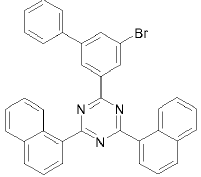 | 57% |
| **(VI)d** | 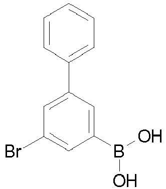 | 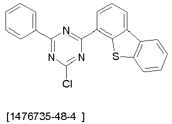 | 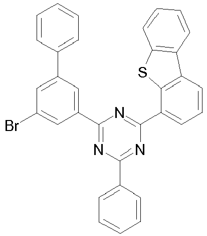 | 56% |
| **(VI)e** | 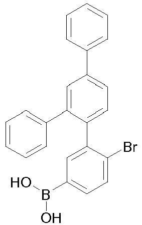 | 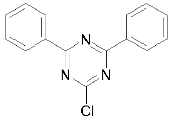 | 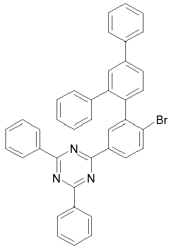 | 54% |
| **(VI)f** | 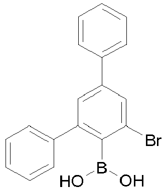 | 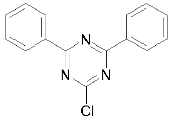 | 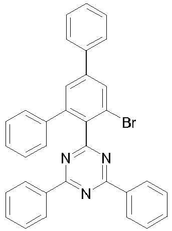 | 51% |
| **(VI)g** | 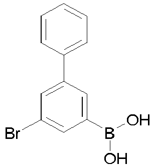 | 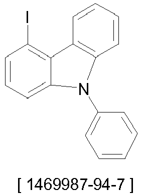 | 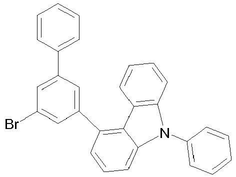 | 58% |
| **(VI)h** | 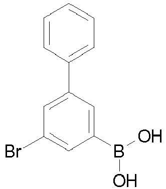 | 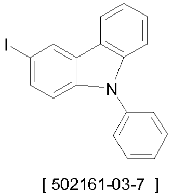 | 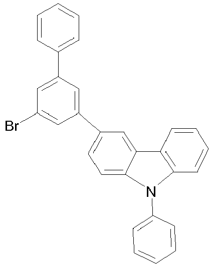 | 60% |
| **(VI)j** | 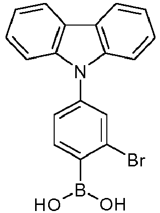 | 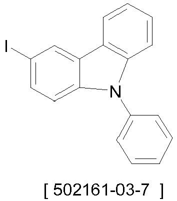 | 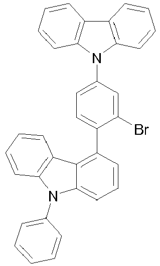 | 59% |

### Synthese von Verbindung (VIII)

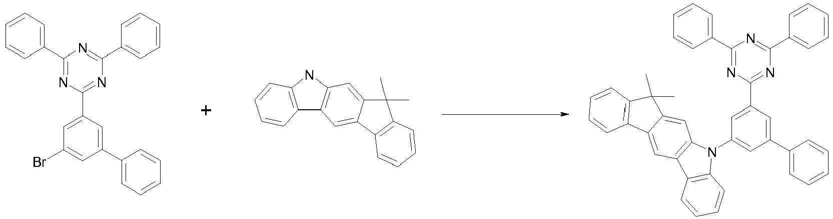

23 g (50 mmol) des Moleküls (VI) und 15.4 g (54 mmol) 5,7-Dihydro-7,7-dimethylindeno-[2,1-b]carbazol werden in 400 ml Toluol unter Argonatmosphäre gelöst. 1.0 g (5 mmol) Tri-tert-butylphosphin werden zugegeben und unter Argonatmosphäre gerührt. 0.6 g (2 mmol) Pd(OAc)₂ werden zugegeben und unter Argonatmosphäre gerührt, dann werden 9.5 g (99 mmol) Natium-tert-butanolat zugegeben. Das Reaktionsgemisch wird unter Rückfluss 24 h gerührt. Nach dem Abkühlen wird die organische Phase abgetrennt, dreimal mit 200 ml Wasser gewaschen, über MgSO₄ getrocknet, filtriert und das Lösungsmittel im Vakuum entfernt. Der Rückstand wird durch Säulenchromatographie über Kieselgel (Eluent:
DCM/Heptan (1:3)) gereinigt. Die Ausbeute beträgt 32 g (48 mmol), entsprechend 97 % der Theorie.

Analog dazu werden die folgenden Verbindungen hergestellt:

| | **Edukt 1** | **Edukt 2** | **Produkt** | **Ausbeute** |
|---|---|---|---|---|
| **(VIII)a** | 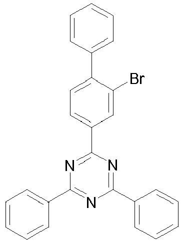 | 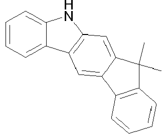 | 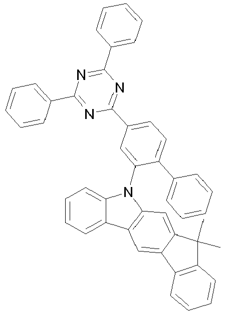 | 72% |
| **(VIII)b** | 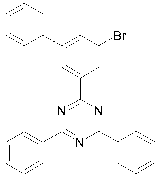 | 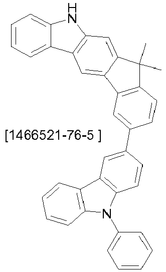 | 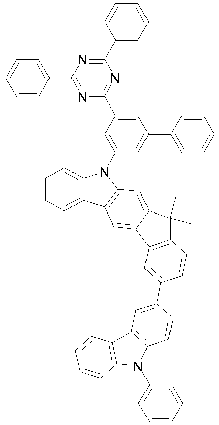 | 76% |
| **(VIII)c** | 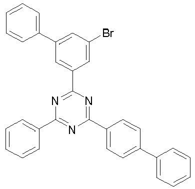 | 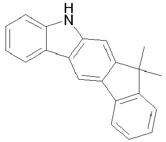 | 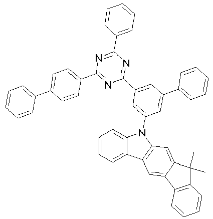 | 71% |
| **(VIII)d** | 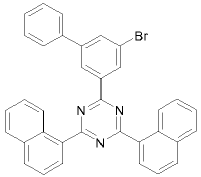 | 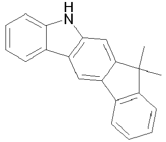 | 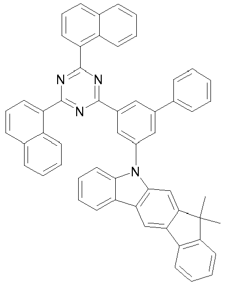 | 75% |
| **(VIII)e** | 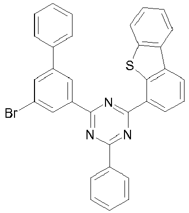 | 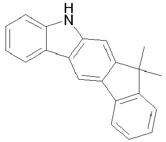 | 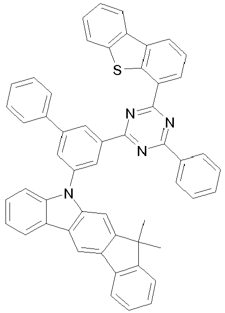 | 74% |
| **(VIII)f** | 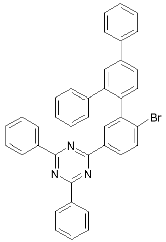 | 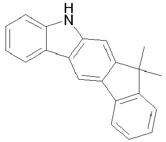 | 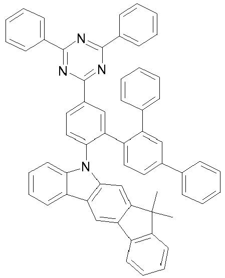 | 67% |
| **(VIII)h** | 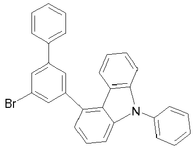 | 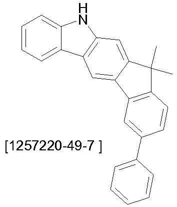 | 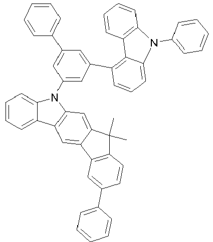 | 73% |
| **(VIII)j** | 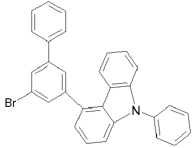 | 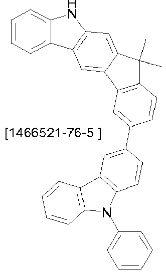 | 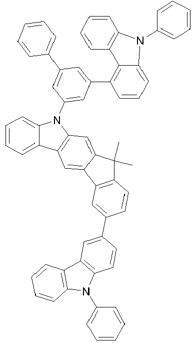 | 77% |
| **(VIII)i** | 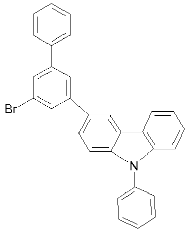 | 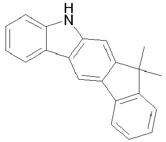 | 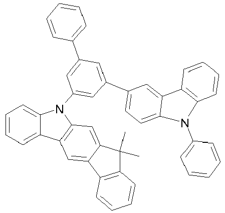 | 78% |
| **(VIII)k** | 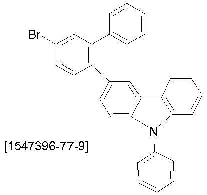 | 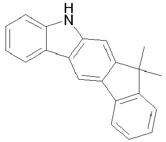 | 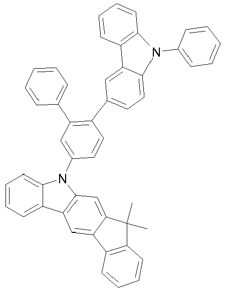 | 75% |
| **(VIII)l** | 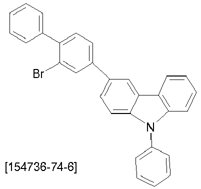 | 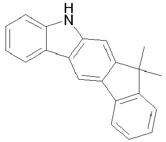 | 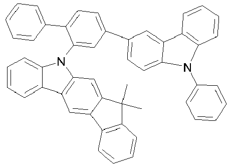 | 69% |
| **(VIII)m** | 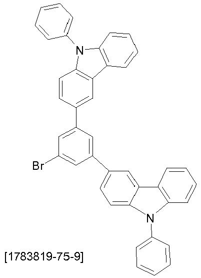 | 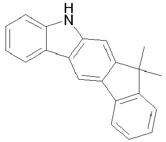 | 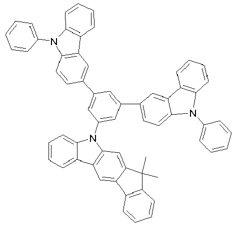 | 73% |
| **(VIII)n** | 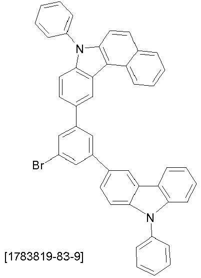 | 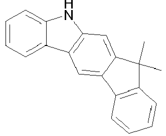 | 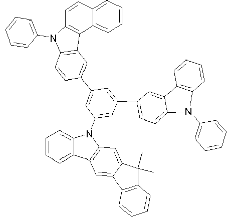 | 70% |
| **(VIII)o** | 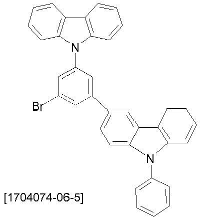 | 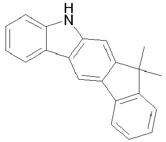 | 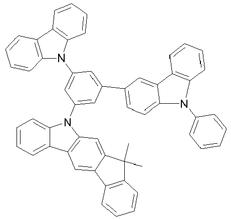 | 76% |
| **(VIII)p** | 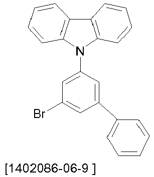 | 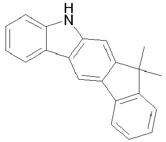 | 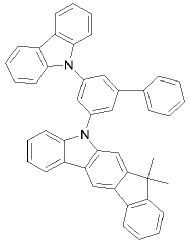 | 79% |
| **(VIII)q** | 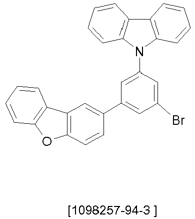 | 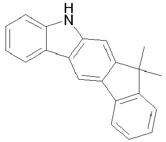 | 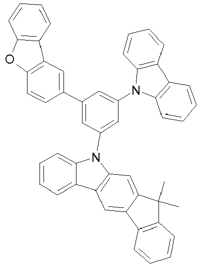 | 76% |
| **(VIII)r** | 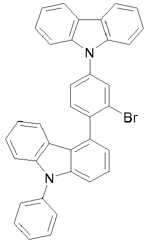 | 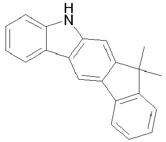 | 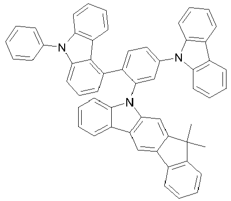 | 68% |
| **(VIII)s** | 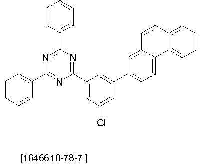 | 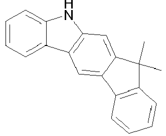 | 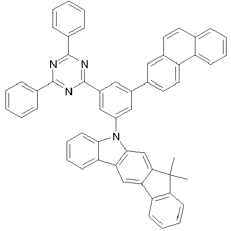 | 60% |
| **(VIII)t** | 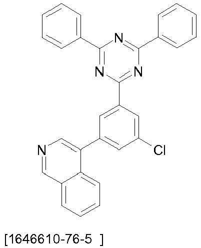 | 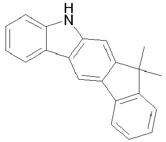 | 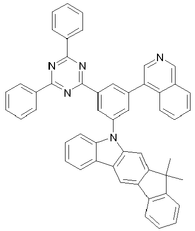 | 62% |
| **(VIII)u** | 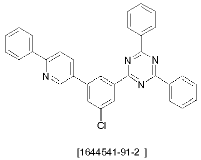 | 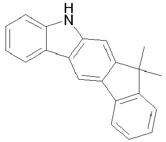 | 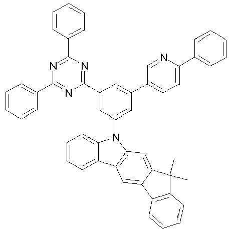 | 58% |
| **(VIII)v** | 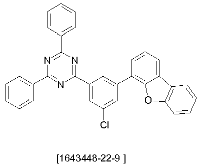 | 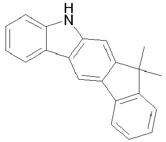 | 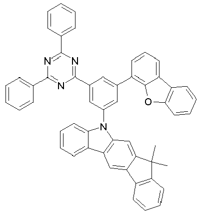 | 52% |
| **(VIII)w** | 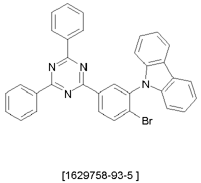 | 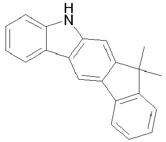 | 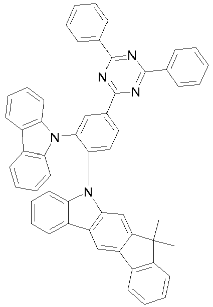 | 56% |
| **(VIII)z** | 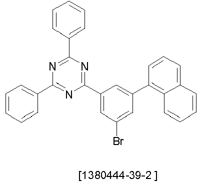 | 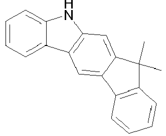 | 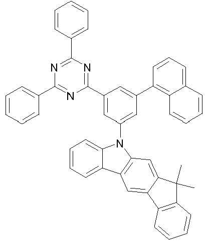 | 71% |
| **(VIII)za** | 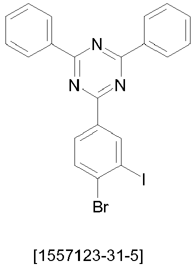 | 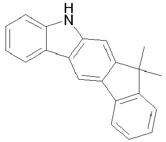 | 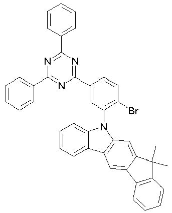 | 57% |
| **(VIII)zb** | 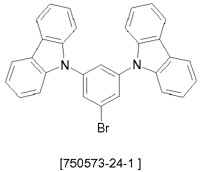 | 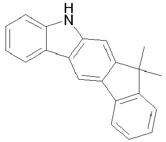 | 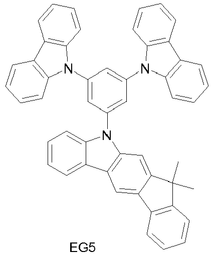 | 67% |
| **(VIII)zc** | 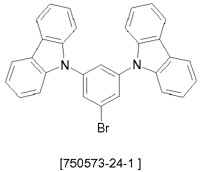 | 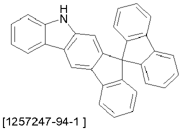 | 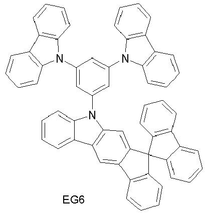 | 61% |
| **(VIII)zd** | 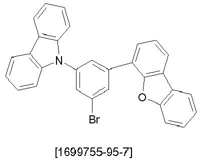 | 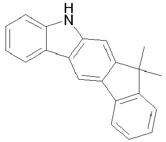 | 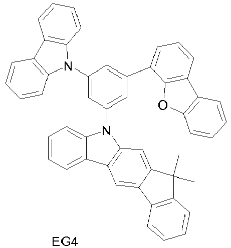 | 70% |

### Beispiel 2: Synthese von Verbindung (XII)

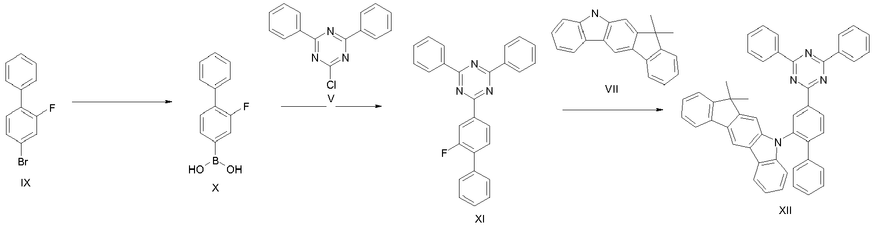

### Synthese von Verbindung (X)

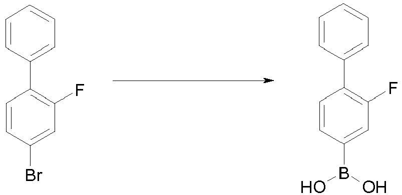

49 g (195 mmol) 4-Brom-2-fluor-1,1'-biphenyl werden in 1000ml THF unter Argonatmosphäre gelöst und auf -78 °C gekühlt. 89.4 ml (215 mmol/2.5M in Hexan) Buthyllithium werden bei -78 °C tropfenweise zugegeben und das Gemisch 2 h bei -78 °C gerührt. 30.4 g (293 mmol) Trimethylborat werden anschließend tropfenweise bei -78 °C zugegeben und das Gemisch über Nacht bei Raumtemperatur gerührt. 200 ml 2N-Salzsäure werden zugegeben und 1 h gerührt. Die organische Phase wird abgetrennt, dreimal mit 300 ml Wasser gewaschen, über MgSO₄ getrocknet, filtriert und das Lösungsmittel im Vakuum entfernt. Der Rückstand wird mit 500 ml Heptan gewaschen, filtriert und getrocknet. Die Ausbeute beträgt 16.2 g (75 mmol), entsprechend 38 % der Theorie.

### Synthese von Verbindung (XI)

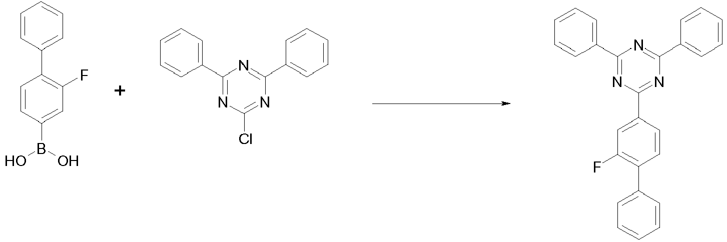

18 g (83 mmol) des Moleküls (X), 22.3 g (83 mmol) 2-Chlor-4,6-diphenyl-1,3,5-triazin und 9.7 g (91.6 mmol) Natriumcarbonat werden in 144 ml 1,4-Dioxan, 144 ml Wasser und 24 ml Toluol gelöst und unter Argonatmosphäre gerührt. 0.9 g (1 mmol) Tetrakis(triphenylphosphin)-palladium werden zugegeben. Das Reaktionsgemisch wird unter Rückfluss über Nacht gerührt. Nach dem Abkühlen wird die organische Phase getrennt, dreimal mit 300 ml Wasser gewaschen, über MgSO₄ getrocknet, filtriert und das Lösungsmittel im Vakuum entfernt. Der Rückstand wird durch Säulenchromatographie über Kieselgel (Eluent: DCM/Heptan (1:10) gereinigt. Die Ausbeute beträgt 25.1 g (62 mmol), entsprechend 34 % der Theorie.

Analog dazu werden die folgenden Verbindungen hergestellt:

| | **Edukt 1** | **Edukt 2** | **Produkt** | **Ausbeute** |
|---|---|---|---|---|
| **(XI)a** | 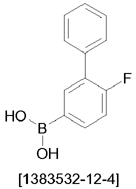 | 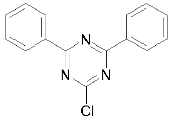 | 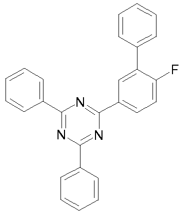 | 72% |
| **(XI)b** | 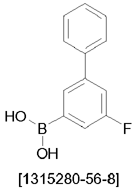 | 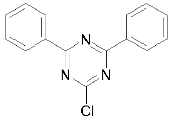 | 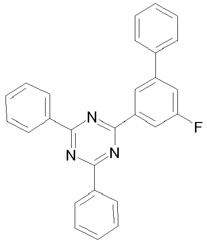 | 70% |
| **(XI)c** | 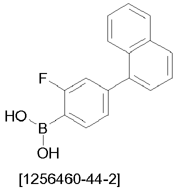 | 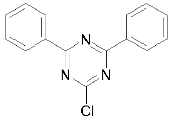 | 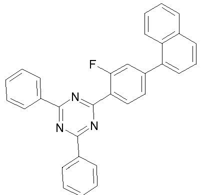 | 67% |
| **(XI)d** | 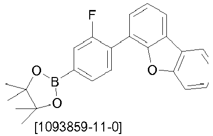 | 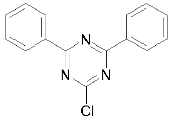 | 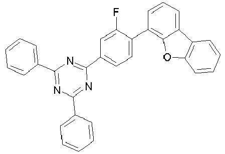 | 68% |
| **(XI)e** | 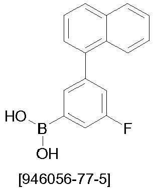 | 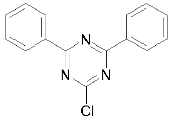 | 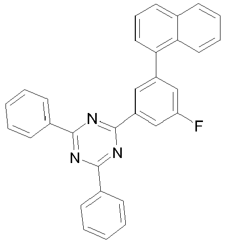 | 60% |

### Synthese von Verbindung (XII)

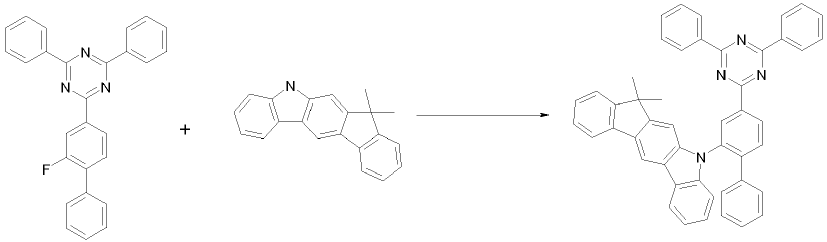

24.0 g (60 mmol) des Moleküls (XI), 16.8 g (60 mmol) von 5,7-Dihydro-7,7-dimethylindeno-[2,1-b]carbazol und 27.1 g (77 mmol) Caesiumcarbonat werden in 320 ml DMSO unter Argonatmosphäre gelöst. Das Reaktionsgemisch wird unter Rückfluss 24 h gerührt. Nach dem Abkühlen wird die organische Phase getrennt, dreimal mit 200 ml Wasser gewaschen, über MgSO₄ getrocknet, filtriert und das Lösungsmittel im Vakuum entfernt. Der Rückstand wird durch Säulenchromatographie über Kieselgel (Eluent: DCM und Heptan (1:3)) gereinigt. Die Ausbeute beträgt 33.2 g (48 mmol), entsprechend 84 % der Theorie.

Analog dazu werden die folgenden Verbindungen hergestellt:

| | **Edukt 1** | **Edukt 2** | **Produkt** | **Ausbeute** |
|---|---|---|---|---|
| **(XII)a** | 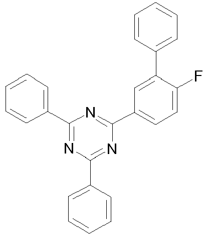 | 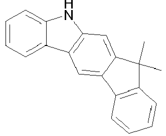 | 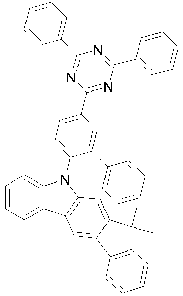 | 72% |
| **(XII)b** | 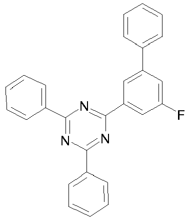 | 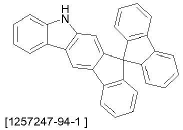 | 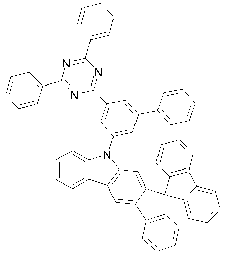 | 62% |
| **(XII)c** | 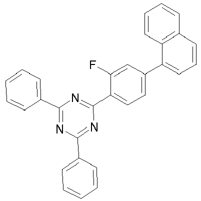 | 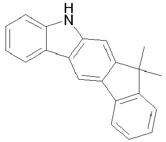 | 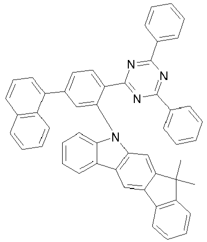 | 68% |
| **(XII)d** | 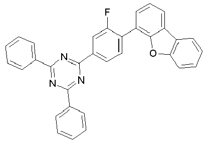 | 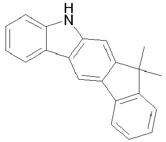 | 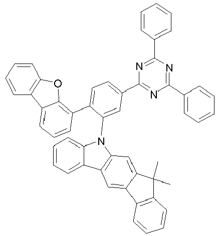 | 72% |
| **(XII)e** | 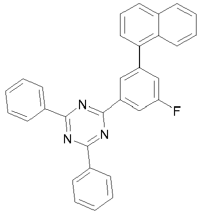 | 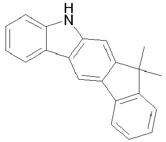 | 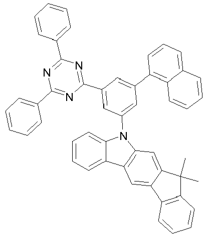 | 73% |

### Beispiel 3: Synthese von Verbindung (XVIII)

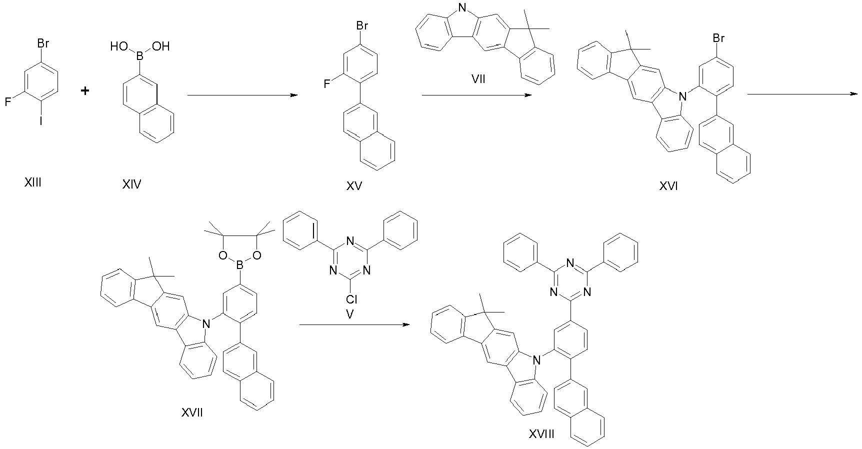

### Synthese von Verbindung (XV)

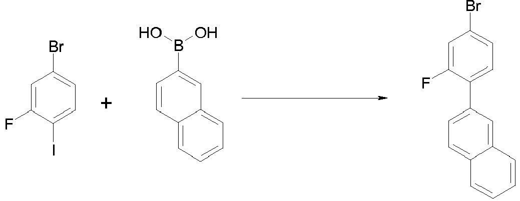

20 g (67 mmol) 4-Brom-2-fluor-1-iodbenzol, 10.5 g (61 mmol) 2-Naphthylboronsäure und 12.8 g (122 mmol) Natriumsulfat werden in 150 ml Ethanol, 50 ml Wasser und 240 ml Toluol gelöst und unter Argonatmosphäre gerührt. 2.1 g (2 mmol) Tetrakis(triphenylphosphin)-palladium werden zugegeben. Das Reaktionsgemisch wird unter Rückfluss über Nacht gerührt. Nach dem Abkühlen wird die organische Phase getrennt, dreimal mit 300 ml Wasser gewaschen, über MgSO₄ getrocknet, filtriert und das Lösungsmittel im Vakuum entfernt. Der Rückstand wird durch Säulenchromatographie über Kieselgel (Eluent: DCM und Heptan (1:10)) gereinigt. Die Ausbeute beträgt 11.7 g (39 mmol), entsprechend 64 % der Theorie.

Analog dazu wird die folgende Verbindung hergestellt:

| | **Edukt 1** | **Edukt 2** | **Produkt** | **Ausbeute** |
|---|---|---|---|---|
| **(XV)a** | 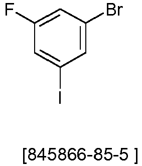 | 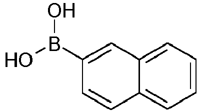 | 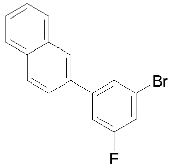 | 50% |

### Synthese von Verbindung (XVI)

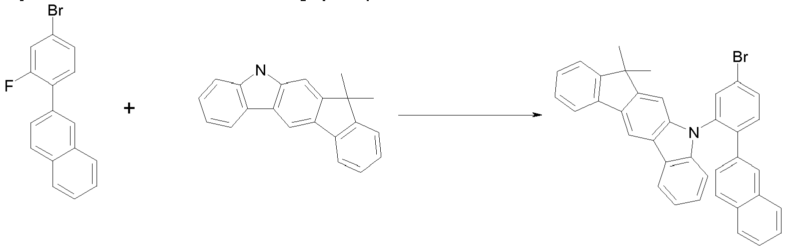

20 g (66 mmol) des Moleküls (XV), 20.7 g (73 mmol) 5,7-Dihydro-7,7-dimethyl-indeno[2,1-b]carbazol und 30 g (73 mmol) Caesiumcarbonat werden in 320 ml DMSO unter Argonatmosphäre gelöst. Das Reaktionsgemisch wird unter Rückfluss 24 h gerührt. Nach dem Abkühlen wird die organische Phase getrennt, dreimal mit 200 ml Wasser gewaschen, über MgSO₄ getrocknet, filtriert und das Lösungsmittel im Vakuum entfernt. Der Rückstand wird durch Säulenchromatographie über Kieselgel (Eluent: DCM und Heptan (1:3)) gereinigt. Die Ausbeute beträgt 35 g (62 mmol), entsprechend 93 % der Theorie.

Analog dazu wird die folgende Verbindung hergestellt:

| | **Edukt 1** | **Edukt 2** | **Produkt** | **Ausbeute** |
|---|---|---|---|---|
| **(XVI)a** | 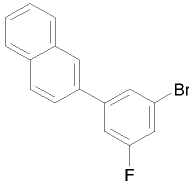 | 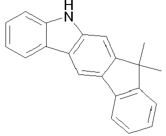 | 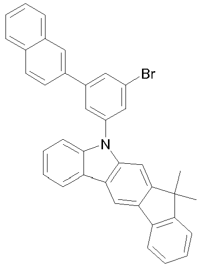 | 87% |

### Synthese von Verbindung (XVII)

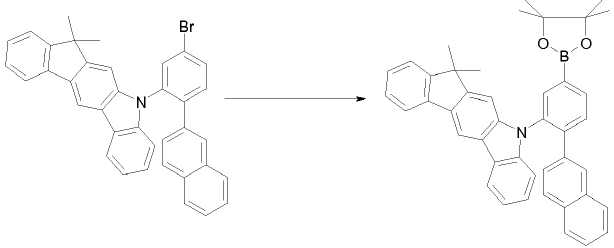

28.0 g (50 mmol) des Moleküls (XVI), 13.8 g (54 mmol) Bis(pinacolato)-diboran und 14.1 g (144 mmol) Kaliumacetat werden in 1000 ml DMSO gelöst und unter Argonatmosphäre gerührt. 1.2 g (1 mmol) 1,1-Bis(diphenylphosphino)ferrocen-dichloropalladium(II) werden zugegeben. Das Reaktionsgemisch wird unter Rückfluss über Nacht gerührt. Nach dem Abkühlen wird die organische Phase abgetrennt, dreimal mit 300 ml Wasser gewaschen, über MgSO₄ getrocknet, filtriert und das Lösungsmittel im Vakuum entfernt. Der Rückstand wird durch Säulenchromatographie auf Kieselgel (Eluent: EA und Heptan (1:100)) gereinigt. Die Ausbeute beträgt 24.1 g (39 mmol), entsprechend 79 % der Theorie.

Analog dazu werden die folgenden Verbindungen hergestellt:

| | **Edukt 1** | **Produkt** | **Ausbeute** |
|---|---|---|---|
| **(XVI)a** | 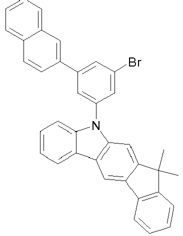 | 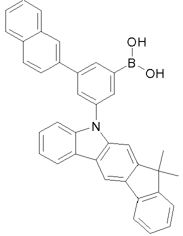 | 65% |
| **(XVI)b** | 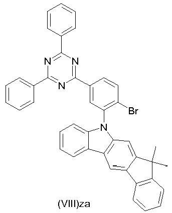 | 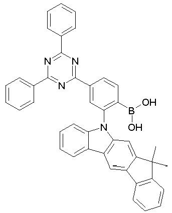 | 76% |

### Synthese von Verbindung (XVIII)

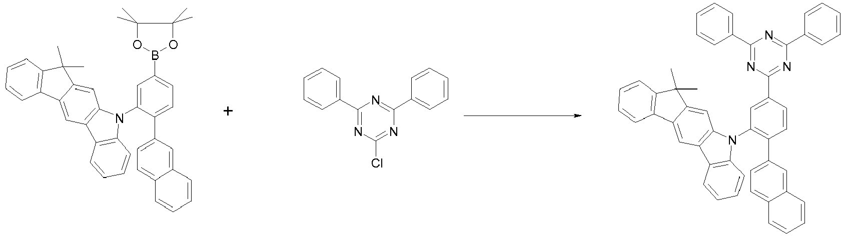

24 g (39 mmol) des Moleküls (XVII), 10.5 g (39 mmol) 2-Chlor-4,6-diphenyl-1,3,5-triazin und 8.3 g (78.4 mmol) Natriumcarbonat werden in 70 ml Ethanol, 80 ml Wasser und 170 ml Toluol gelöst und unter Argonatmosphäre gerührt. 0.8 g (1 mmol) Tetrakis(triphenylphosphin)-palladium werden zugegeben. Das Reaktionsgemisch wird unter Rückfluss über Nacht gerührt. Nach dem Abkühlen wird die organische Phase abgetrennt, dreimal mit 200 ml Wasser gewaschen, über MgSO₄ getrocknet, filtriert und das Lösungsmittel im Vakuum entfernt. Der Rückstand wird durch Säulenchromatographie über Kieselgel (Eluent: DCM und Heptan (1:3)) gereinigt. Die Ausbeute beträgt 24 g (33 mmol), entsprechend 85 % der Theorie.

Analog dazu werden die folgenden Verbindungen hergestellt:

| | **Edukt 1** | **Edukt 2** | **Produkt** | **Ausbeute** |
|---|---|---|---|---|
| **(XVIII)a** | 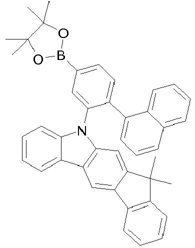 | 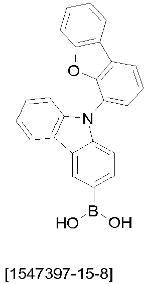 | 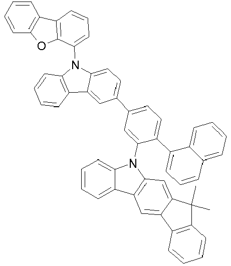 | 70% |
| **(XVIII)b** | 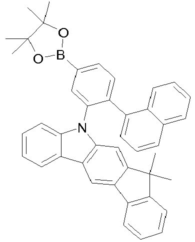 | 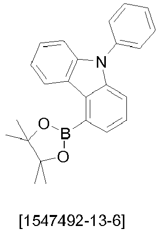 | 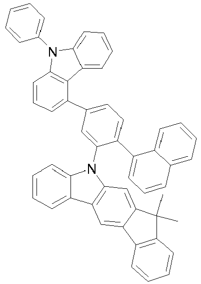 | 73% |
| **(XVIII)c** | 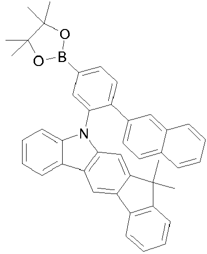 | 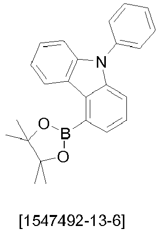 | 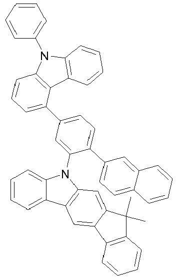 | 76% |
| **(XVIII)d** | 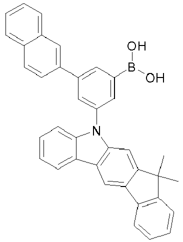 | 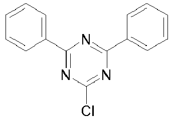 | 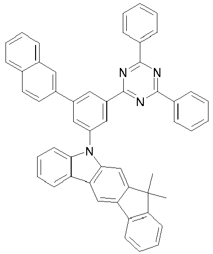 | 71% |
| **(XVIII)e** | 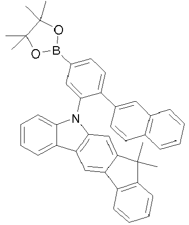 | 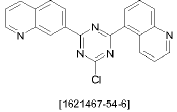 | 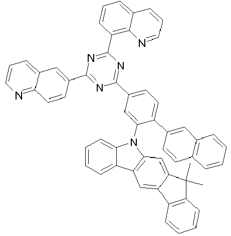 | 69% |
| **(XVIII)f** | 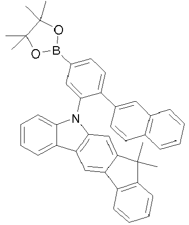 | 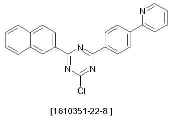 | 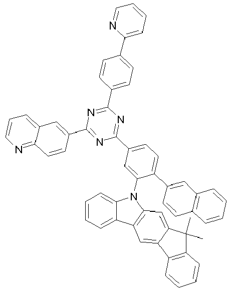 | 70% |
| **(XVIII)g** | 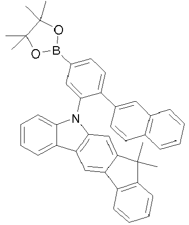 | 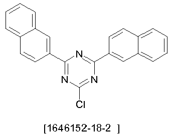 | 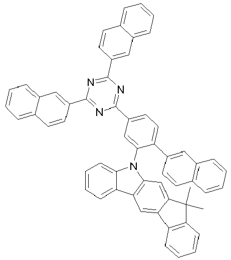 | 73% |
| **(XVIII)h** | 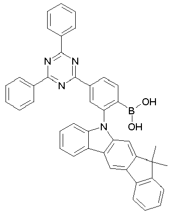 | 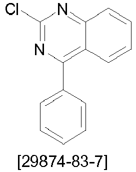 | 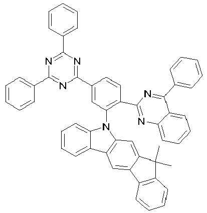 | 70% |
| **(XVIII)i** | 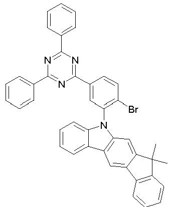 | 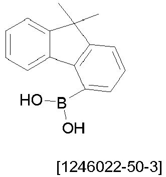 | 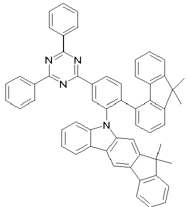 | 77% |
| **(XVIII)j** | 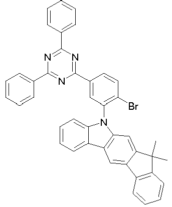 | 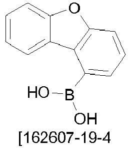 | 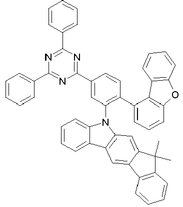 | 71% |
| **(XVIII)k** | 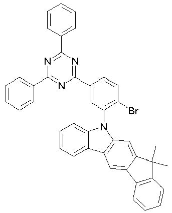 | 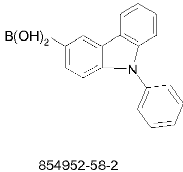 | 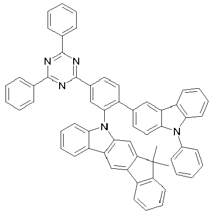 | 74% |
| **(XVIII)m** | 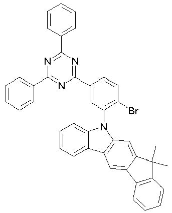 | 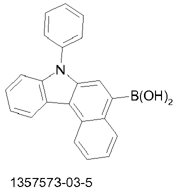 | 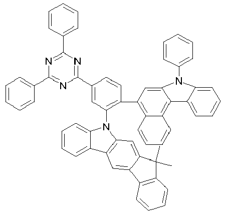 | 73% |

### Beispiel 4: Synthese von Verbindung (XXV)

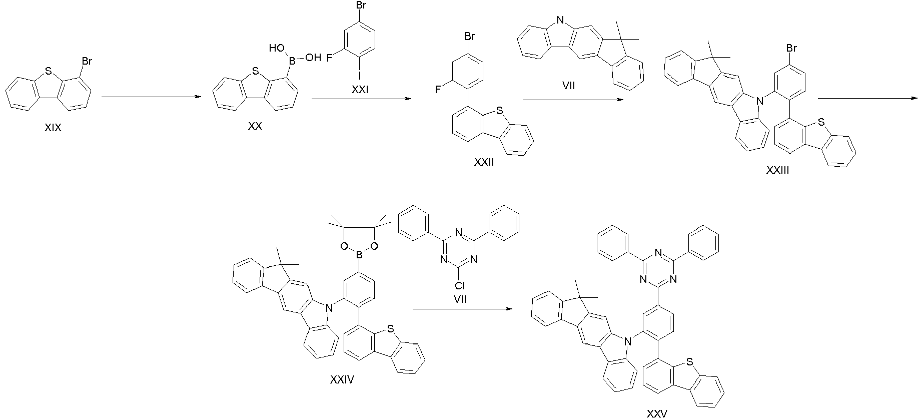

### Synthese von Verbindung (XX)

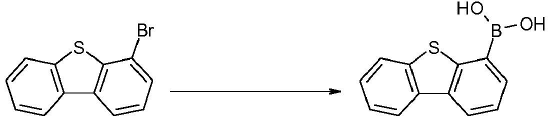

25.0 g (95 mmol) 4-Bromdibenzothiophen werden in 500 ml THF unter Argonatmosphäre gelöst und auf -78 °C gekühlt. 10 ml (104 mmol, 2.5M in Hexan) Buthyllithium werden bei -78 °C tropfenweise zugegeben und das Gemisch 2 h bei -78 °C gerührt. 14.8 g (143 mmol) Trimethylborat werden anschließend tropenweise bei -78 °C zugegeben und das Gemisch über Nacht bei Raumtemperatur gerührt. 200 ml 2N-Salzsäure werden zugegeben und 1 h gerührt. Die organische Phase wird abgetrennt, dreimal mit 300 ml Wasser gewaschen, über MgSO₄ getrocknet, filtriert und das Lösungsmittel im Vakuum entfernt. Der Rückstand wird mit 500 ml Heptan gewaschen, gefiltert und getrocknet. Die Ausbeute beträgt 20.1 g (88 mmol), entsprechend 77 % der Theorie.

### Synthese von Verbindung (XXII)

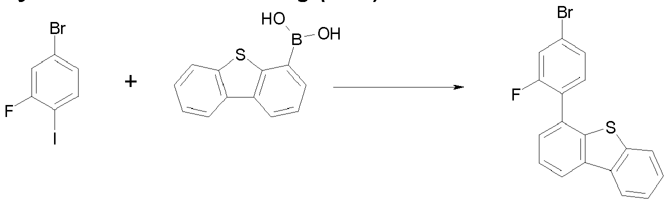

29 g (96 mmol) 4-Brom-2-fluor-1-iobenzol, 20 g (88 mmol) des Moleküls (XX) und 19.2 g (175 mmol) Natriumcarbonat werden in 300 ml Ethanol, 100 ml Wasser und 480 ml Toluol gelöst und unter Argonatmosphäre gerührt. 1 g (3 mmol) Tetrakis(triphenylphosphin)-palladium werden zugegeben. Das Reaktionsgemisch wird unter Rückfluss über Nacht gerührt. Nach dem Abkühlen wird das Reaktionsgemisch gequencht. Die organische Phase wird abgetrennt, dreimal mit 200 ml Wasser gewaschen, über MgSO₄ getrocknet, filtriert und das Lösungsmittel im Vakuum entfernt. Der Rückstand wird durch Säulenchromatographie über Kieselgel (Eluent: DCM und Heptan (1:10) gereinigt. Die Ausbeute beträgt 22.5 g (63 mmol), entsprechend 72 % der Theorie.

Analog dazu wird die folgende Verbindung hergestellt:

| | **Edukt 1** | **Edukt 2** | **Produkt** | **Ausbeute** |
|---|---|---|---|---|
| **(XXII)a** | 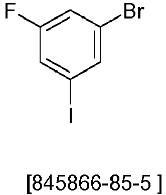 | 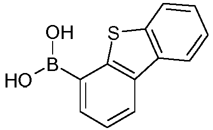 | 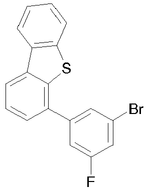 | 56% |

### Synthese von Verbindung (XXIII)

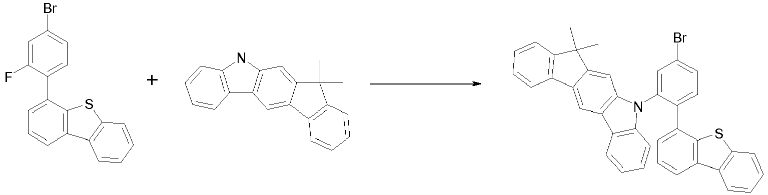

22.5 g (63 mmol) des Moleküls (XXII), 19.6 g (69 mmol) 5,7-Dihydro-7,7-dimethyl-indeno[2,1-b]carbazol und 28.4 g (69 mmol) Caesiumcarbonat werden in 350 ml DMSO unter Argonatmosphäre gelöst. Das Reaktionsgemisch wird unter Rückfluss 24 h gerührt. Nach dem Abkühlen wird die organische Phase abgetrennt, dreimal mit 200 ml Wasser gewaschen, über MgSO₄ getrocknet, filtriert und das Lösungsmittel im Vakuum entfernt. Der Rückstand wird durch Säulenchromatographie über Kieselgel (Eluent: DCM und Heptan (1:3)) gereinigt. Die Ausbeute beträgt 30 g (49 mmol), entsprechend 77 % der Theorie.

Analog dazu wird die folgende Verbindung hergestellt:

| | **Edukt 1** | **Edukt 2** | **Produkt** | **Ausbeute** |
|---|---|---|---|---|
| **(XXIII)a** | 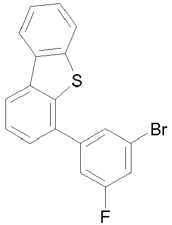 | 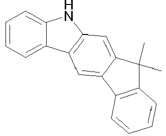 | 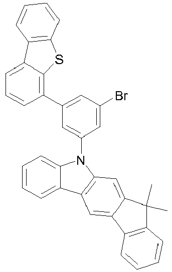 | 72% |

### Synthese von Verbindung (XXIV)

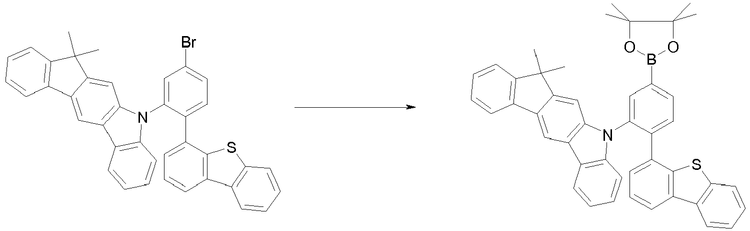

30 g (49 mmol) des Moleküls (XXIII), 13.5 g (53 mmol) Bis(pinocolato)-diboran und 13.8 g (140 mmol) Kaliumacetat werden in 1000 ml DMSO gelöst und unter Argonatmosphäre gerührt. 1.2 g (1 mmol) 1,1-Bis(di-phenylphosphino)ferrocen-dichloropalladium(II) werden zugegeben. Das Reaktionsgemisch wird unter Rückfluss über Nacht gerührt. Nach dem Abkühlen wird das Gemisch gequencht. Die organische Phase wird abgetrennt, dreimal mit 300 ml Wasser gewaschen, über MgSO₄ getrocknet, filtriert und das Lösungsmittel im Vakuum entfernt. Der Rückstand wird durch Säulenchromatographie über Kieselgel (Eluent: EA und Heptan (1:3)) gereinigt. Die Ausbeute beträgt 25.5 g (38 mmol), entsprechend 79 % der Theorie.

Analog dazu wird die folgende Verbindung hergestellt:

| | **Edukt 1** | **Produkt** | **Ausbeute** |
|---|---|---|---|
| **(XXIV)a** | 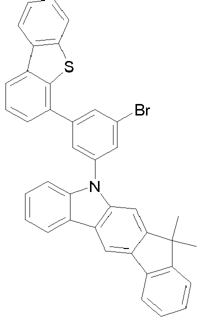 | 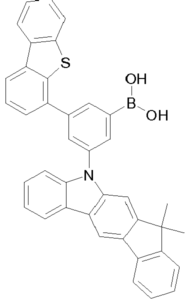 | 66% |

### Synthese von Verbindung (XXV)

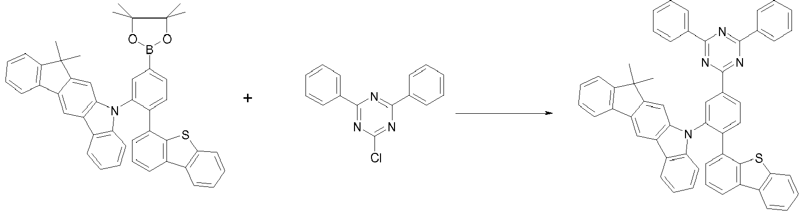

25.5 g (38 mmol) des Moleküls (XXIV), 10.2 g (38 mmol) 2-Chlor-4,6-diphenyl-1,3,5-triazin und 8.1 g (76.4 mmol) Natriumcarbonat werden in 90 ml Ethanol, 100 ml Wasser und 200 ml Toluol gelöst und unter Argonatmosphäre gerührt. 0.8 g (1 mmol) Tetrakis(triphenylphosphin)-palladium werden zugegeben. Das Reaktionsgemisch wird unter Rückfluss über Nacht gerührt. Nach dem Abkühlen wird die organische Phase abgetrennt, dreimal mit 200 ml Wasser gewaschen, über MgSO₄ getrocknet, filtriert und das Lösungsmittel im Vakuum entfernt. Der Rückstand wird durch Säulenchromatographie über Kieselgel (Eluent: DCM und Heptan (1:3)) gereinigt. Die Ausbeute beträgt 25 g (32 mmol), entsprechend 85 % der Theorie.

Analog dazu werden die folgenden Verbindungen hergestellt:

| | **Edukt 1** | **Edukt 2** | **Produkt** | **Ausbeute** |
|---|---|---|---|---|
| **(XXV)a** | 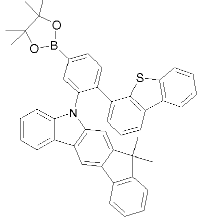 | 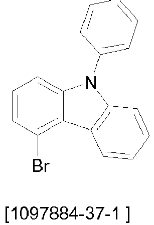 | 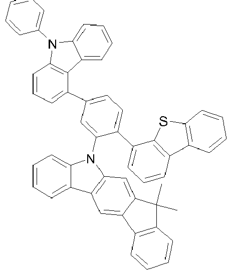 | 70% |
| **(XXV)b** | 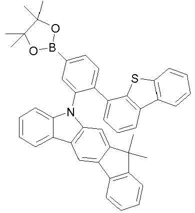 | 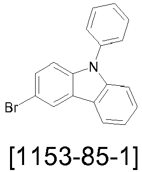 | 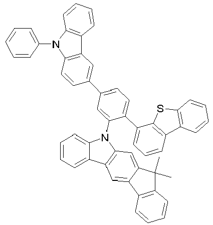 | 72% |
| **(XXV)c** | 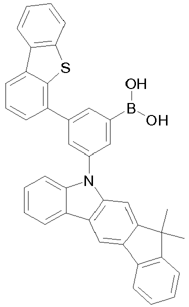 | 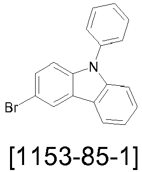 | 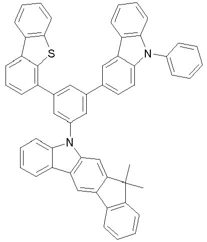 | 71% |
| **(XXV)d** | 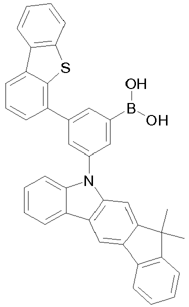 | 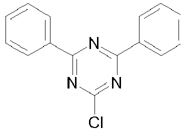 | 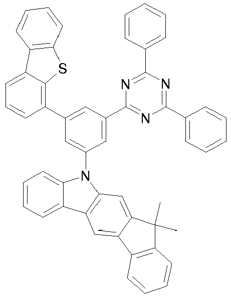 | 68% |

### Beispiel 5: Synthese von Verbindung (XXXI)

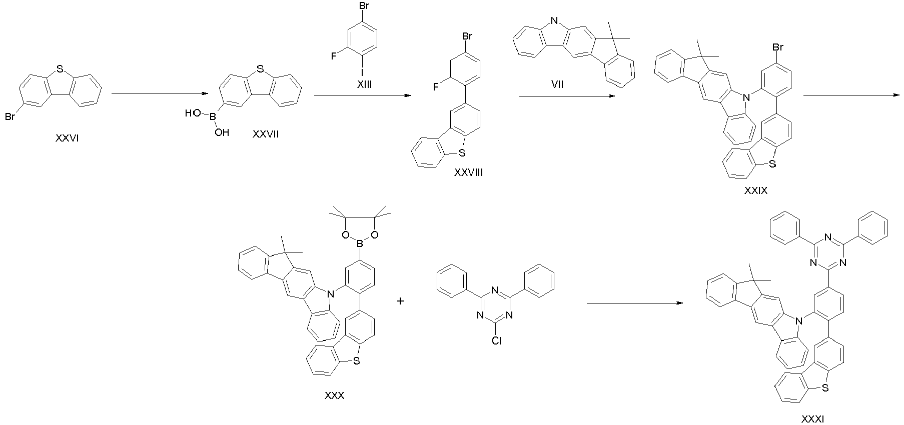

### Synthese von Verbindung (XXVII)

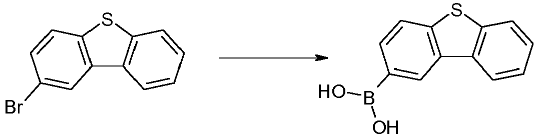

25.0 g (95 mmol) 2-Bromdibenzothiophen werden in 500 ml THF unter Argonatmosphäre gelöst und auf -78 °C gekühlt. 10 ml (104 mmol, 2.5M in Hexan) Buthyllithium werden bei -78 °C tropfenweise zugegeben und das Gemisch wird 2 h bei -78 °C gerührt. 14.8 g (143 mmol) Trimethylborat werden anschließend tropfenweise bei -78 °C zugegeben und das Gemisch über Nacht bei Raumtemperatur gerührt. 200 ml 2N-Salzsäure werden zugegeben und 1 h gerührt. Die organische Phase wird abgetrennt, dreimal mit 300 ml Wasser gewaschen, über MgSO₄ getrocknet, filtriert und das Lösungsmittel im Vakuum entfernt. Der Rückstand wird mit 500 ml Heptan gewaschen, filtriert und getrocknet. Die Ausbeute beträgt 18.6 g (79 mmol), entsprechend 86 % der Theorie.

### Synthese von Verbindung (XXVIII)

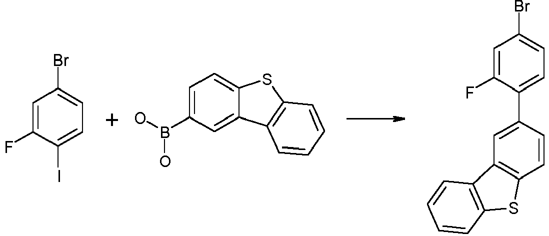

23.7 g (79 mmol) 4-Brom-2-fluor-1-iodbenzol, 18 g (79 mmol) des Moleküls (XXVII) und 16.7 g (158 mmol) Natriumcarbonat werden in 300 ml Ethanol, 100 ml Wasser und 480 ml Toluol gelöst und unter Argonatmosphäre gerührt. 1 g (3 mmol) Tetrakis(triphenylphosphin)-palladium werden zugegeben. Das Reaktionsgemisch wird unter Rückfluss über Nacht gerührt. Nach dem Abkühlen wird das Reaktionsgemisch gequencht. Die organische Phase wird abgetrennt, dreimal mit 200 ml Wasser gewaschen, über MgSO₄ getrocknet, filtriert und das Lösungsmittel im Vakuum entfernt. Der Rückstand wird durch Säulenchromatographie über Kieselgel (Eluent: DCM und Heptan (1:10) gereinigt. Die Ausbeute beträgt 24.1 g (67 mmol), entsprechend 85 % der Theorie.

### Synthese von Verbindung (XXIX)

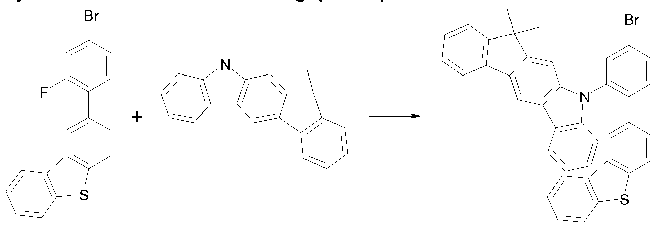

20 g (56 mmol) des Moleküls (XXVIII), 17.5 g (52 mmol) 5,7-Dihydro-7,7-dimethyl-indeno[2,1-b]carbazol und 25.3 g (61 mmol) Caesiumcarbonat werden in 350 ml DMSO unter Argonatmosphäre gelöst. Das Reaktionsgemisch wird unter Rückfluss 24 h gerührt. Nach dem Abkühlen wird die organische Phase abgetrennt, dreimal mit 200 ml Wasser gewaschen, über MgSO₄ getrocknet, filtriert und das Lösungsmittel im Vakuum entfernt. Der Rückstand wird durch Säulenchromatographie über Kieselgel (Eluent DCM : Heptan 1:3) gereinigt. Die Ausbeute beträgt 19 g (31 mmol), entsprechend 54 % der Theorie.

### Synthese von Verbindung (XXX)

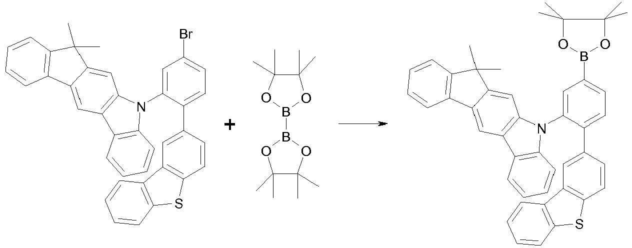

19 g (31 mmol) des Moleküls (XXIX), 8.6 g (33 mmol) Bis(pinacolato)-diboran und 8.7 g (89 mmol) Kaliumacetat werden in 500 ml DMSO gelöst und unter Argonatmosphäre gerührt. 1.2 g (1 mmol) 1,1-Bis(diphenyl-phosphino)ferrocen-dichloropalladium(II) werden zugegeben. Das Reaktionsgemisch wird unter Rückfluss über Nacht gerührt. Nach dem Abkühlen wird das Gemisch gequencht. Die organische Phase wird abgetrennt, dreimal mit 300 ml Wasser gewaschen, über MgSO₄ getrocknet, filtriert und das Lösungsmittel im Vakuum entfernt. Der Rückstand wird durch Säulenchromatographie über Kieselgel (Eluent: EA und Heptan (1:100) gereinigt. Die Ausbeute beträgt 14.5 g (22 mmol), entsprechend 71 % der Theorie.

### Synthese von Verbindung (XXXI)

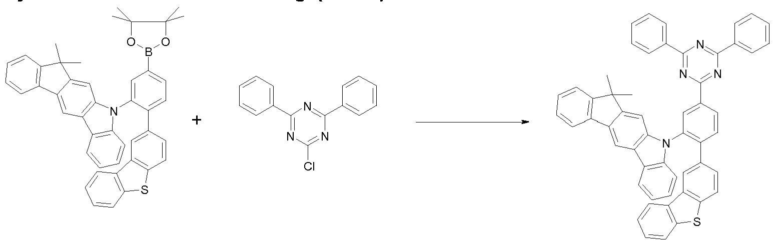

14.5 g (22 mmol) des Moleküls (XXX), 6.4 g (24 mmol) 2-Chlor-4,6-diphenyl-1,3,5-triazin und 4.6 g (43 mmol) Natriumcarbonat werden in 90 ml Ethanol, 100 ml Wasser und 200 ml Toluol gelöst und unter Argonatmosphäre gerührt. 0.8 g (1 mmol) Tetrakis(triphenylphosphin)-palladium werden zugegeben. Das Reaktionsgemisch wird unter Rückfluss über Nacht gerührt. Nach dem Abkühlen wird die organische Phase abgetrennt, dreimal mit 200 ml Wasser gewaschen, über MgSO₄ getrocknet, filtriert und das Lösungsmittel im Vakuum entfernt. Der Rückstand wird durch Säulenchromatographie über Kieselgel (Eluent: DCM und Heptan (1:3)) gereinigt. Die Ausbeute beträgt 14 g (18 mmol), entsprechend 83 % der Theorie.

Analog dazu werden die folgenden Verbindungen hergestellt:

| | **Edukt 1** | **Edukt 2** | **Produkt** | **Ausbeute** |
|---|---|---|---|---|
| **(XXXI)a** | 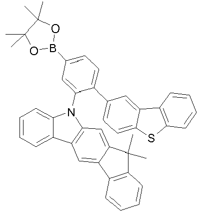 | 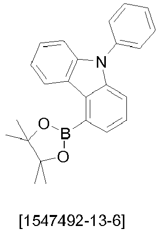 | 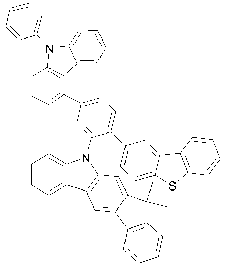 | 74% |
| **(XXXI)b** | 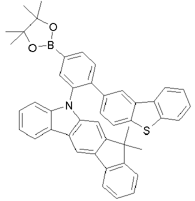 | 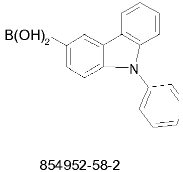 | 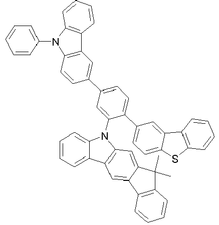 | 75% |

### Devicebeispiele

### Herstellung der OLEDs

In den folgenden Beispielen V1 bis E40 (siehe Tabellen 1 und 2) werden die Daten verschiedener OLEDs vorgestellt.

**Vorbehandlung für die Beispiele V1-E40:** Glasplättchen, die mit strukturiertem ITO (Indium Zinn Oxid) der Dicke 50 nm beschichtet sind, werden zur verbesserten Prozessierung mit 20 nm PEDOT:PSS beschichtet (Poly(3,4-ethylendioxythiophen)poly(styrolsulfonat), bezogen als CLEVIOS™ P VP AI 4083 von Heraeus Precious Metals GmbH Deutschland, aus wässriger Lösung aufgeschleudert). Diese beschichteten Glasplättchen bilden die Substrate, auf welche die OLEDs aufgebracht werden.

Die OLEDs haben prinzipiell den folgenden Schichtaufbau: Substrat / Lochtransportschicht (HTL) / optionale Zwischenschicht (IL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:IC3:TEG1 (55%:35%:10%) bedeutet hierbei, dass das Material IC1 in einem Volumenanteil von 55%, IC3 in einem Anteil von 35% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik, sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m². Als Lebensdauer LD wird die Zeit definiert, nach der die Leuchtdichte bei Betrieb mit konstantem Strom von der Startleuchtdichte auf einen gewissen Anteil L1 absinkt. Eine Angabe von L0;j0 = 4000 cd/m² und L1 = 70% in Tabelle 2 bedeutet, dass die in Spalte LD angegebene Lebensdauer der Zeit entspricht, nach der die Anfangsleuchtdichte von 4000 cd/m² auf 2800 cd/m² absinkt. Analog bedeutet L0;j0 = 20mA/cm², L1 = 80%, dass die Leuchtdichte bei Betrieb mit 20mA/cm² nach der Zeit LD auf 80% ihres Anfangswertes absinkt.

Die Daten der verschiedenen OLEDs sind in Tabelle 2 zusammengefasst. Die Beispiele V1- V11 sind Vergleichsbeispiele gemäß dem Stand der Technik, die Beispiele E1-E40 zeigen Daten von erfindungsgemäßen OLEDs.

Im Folgenden werden einige der Beispiele näher erläutert, um die Vorteile der erfindungsgemäßen OLEDs zu verdeutlichen.

### Verwendung von erfindungsgemäßen Mischungen in der Elektronentransportschicht phosphoreszenter OLEDs

Die elektronenleitenden erfindungsgemäßen Materialien ergeben bei Einsatz in der Elektronentransportschicht in phosphoreszierenden OLEDs wesentliche Verbesserungen gegenüber dem Stand der Technik, vor allem bezüglich Spannung und Leistungseffizienz. Durch Einsatz der erfindungsgemäßen Verbindungen EG1, EG2 und EG3 in Kombination mit LiQ lässt sich eine Steigerung der Spannung sowie der Leistungseffizienz um ca. 10-20 % gegenüber dem Stand der Technik SdT1-5 erzielen (Vergleich der Beispiele V1, V2, V3, V4 und V5 mit E1, E2 und E3).

### Verwendung von erfindungsgemäßen Verbindungen in der Emissionsschicht phosphoreszenter OLEDs

Die lochleitenden erfindungsgemäßen Materialien ergeben bei Einsatz in der Emissionsschicht in phosphoreszierenden OLEDs wesentliche Verbesserungen gegenüber dem Stand der Technik, vor allem bezüglich Lebensdauer. Durch Einsatz der erfindungsgemäßen Verbindungen EG4, EG5, EG6, EG7 und EG8 in Kombination mit dem grün emittierenden Dotanden TEG1 und der elektronenleitenden Matrix IC1 lässt sich eine Steigerung der Lebensdauer um ca. 20-60 % gegenüber dem Stand der Technik SdT7-11 erzielen (Vergleich der Beispiele E4 mit V7, E5 mit V8, E6 mit V9, E7 mit V10, E8 mit V11).

**Tabelle 1: Aufbau der OLEDs**

| Bsp. | HTL | IL | EBL | EML | HBL | ETL | EIL |
|---|---|---|---|---|---|---|---|
| | Dicke | Dicke | Dicke | Dicke | Dicke | Dicke | Dicke |
| V1 | SpA1 | HATCN | SpMA1 | IC1:TEG1 ( | IC1 | SdT1:LiQ (50%:50%) | --- |
| | 70nm | 5nm | 90nm | 90%:10%) 30nm | 10nm | 30nm | |
| V2 | SpA1 | HATCN | SpMA1 | IC1:TEG1 | IC1 | SdT2:LiQ (50%:50%) | --- |
| | 70nm | 5nm | 90nm | (90%:10%) 30nm | 10nm | 30nm | |
| V3 | SpA1 | HATCN | SpMA1 | IC1:TEG1 | IC1 | SdT3:LiQ (50%:50%) | --- |
| | 70nm | 5nm | 90nm | (90%:10%) 30nm | 10nm | 30nm | |
| V4 | SpA1 | HATCN | SpMA1 | IC1:TEG1 | IC1 | SdT4:LiQ (50%:50%) | --- |
| | 70nm | 5nm | 90nm | (90%:10%) 30nm | 10nm | 30nm | |
| V5 | SpA1 | HATCN | SpMA1 | IC1:TEG1 | IC1 | SdT5:LiQ (50%:50%) | --- |
| | 70nm | 5nm | 90nm | (90%:10%) 30nm | 10nm | 30nm | |
| V6 | SpA1 | HATCN | SpMA1 | IC1:TEG1 | IC1 | SdT6:LiQ (50%:50%) | --- |
| | 70nm | 5nm | 90nm | (90%:10%) 30nm | 10nm | 30nm | |
| V7 | SpA1 | HATCN | SpMA1 | IC1:SdT7:TEG1 | IC1 | ST2:LiQ (50%:50%) | --- |
| | 70nm | 5nm | 90nm | (45%:45%:10%) 30nm | 10nm | 30nm | |
| V8 | SpA1 | HATCN | SpMA1 | IC1:SdT8:TEG1 | IC1 | ST2:LiQ (50%:50%) | --- |
| | 70nm | 5nm | 90nm | (45%:45%:10%) 30nm | 10nm | 30nm | |
| V9 | SpA1 | HATCN | SpMA1 | IC1:SdT9:TEG1 | IC1 | ST2:LiQ (50%:50%) | --- |
| | 70nm | 5nm | 90nm | (45%:45%:10%) 30nm | 10nm | 30nm | |
| V10 | SpA1 | HATCN | SpMA1 | IC1:SdT10:TEG1 | IC1 | ST2:LiQ (50%:50%) | --- |
| | 70nm | 5nm | 90nm | (45%:45%:10%) 30nm | 10nm | 30nm | |
| V11 | SpA1 | HATCN | SpMA1 | IC1:SdT11:TEG1 | IC1 | ST2:LiQ (50%:50%) | --- |
| | 70nm | 5nm | 90nm | (45%:45%:10%) 30nm | 10nm | 30nm | |
| E1 | SpA1 | HATCN | SpMA1 | IC1:TEG1 | IC1 | EG1:LiQ (50%:50%) | --- |
| | 70nm | 5nm | 90nm | (90%:10%) 30nm | 10nm | 30nm | |
| E2 | SpA1 | HATCN | SpMA1 | IC1:TEG1 | IC1 | EG2:LiQ (50%:50%) | --- |
| | 70nm | 5nm | 90nm | (90%:10%) 30nm | 10nm | 30nm | |
| E3 | SpA1 | HATCN | SpMA1 | IC1:TEG1 | IC1 | EG3:LiQ (50%:50%) | --- |
| | 70nm | 5nm | 90nm | (90%:10%) 30nm | 10nm | 30nm | |
| E4 | SpA1 | HATCN | SpMA1 | IC1:EG4:TEG1 | IC1 | ST2:LiQ (50%:50%) | --- |
| | 70nm | 5nm | 90nm | (45%:45%:10%) 30nm | 10nm | 30nm | |
| E5 | SpA1 | HATCN | SpMA1 | IC1:EG5:TEG1 | IC1 | ST2:LiQ (50%:50%) | --- |
| | 70nm | 5nm | 90nm | (45%:45%:10%) 30nm | 10nm | 30nm | |
| E6 | SpA1 | HATCN | SpMA1 | IC1:EG6:TEG1 | IC1 | ST2:LiQ (50%:50%) | --- |
| | 70nm | 5nm | 90nm | (45%:45%:10%) 30nm | 10nm | 30nm | |
| E7 | SpA1 | HATCN | SpMA1 | IC1:EG7:TEG1 | IC1 | ST2:LiQ (50%:50%) | --- |
| | 70nm | 5nm | 90nm | (45%:45%:10%) 30nm | 10nm | 30nm | |
| E8 | SpA1 | HATCN | SpMA1 | IC1:EG8:TEG1 | IC1 | ST2:LiQ (50%:50%) | --- |
| | 70nm | 5nm | 90nm | (45%:45%:10%) 30nm | 10nm | 30nm | |
| E9 | SpA1 | HATCN | SpMA1 | 8b:TEG1 | IC1 | ST2:LiQ (50%:50%) | --- |
| | 70nm | 5nm | 90nm | (95%:5%) 30nm | 10nm | 30nm | |
| E10 | SpA1 | HATCN | SpMA1 | 8c:TEG1 | IC1 | ST2:LiQ (50%:50%) | --- |
| | 70nm | 5nm | 90nm | (90%:10%) 30nm | 10nm | 30nm | |
| E11 | SpA1 | HATCN | SpMA1 | 8d:TER3 | --- | ST2:LiQ (50%:50%) | --- |
| | 90nm | 5nm | 130nm | (92%:8%) 40nm | | 40nm | |
| E12 | SpA1 | HATCN | SpMA1 | 8e:TEG1 | IC1 | ST2:LiQ (50%:50%) | --- |
| | 70nm | 5nm | 90nm | (90%:10%) 30nm | 10nm | 30nm | |
| E13 | SpA1 | HATCN | SpMA1 | 8f:TEG1 | IC1 | ST2:LiQ (50%:50%) | --- |
| | 70nm | 5nm | 90nm | (90%:10%) 30nm | 10nm | 30nm | |
| E14 | SpA1 | HATCN | SpMA1 | IC1:6k:TEG1 | IC1 | ST2:LiQ (50%:50%) | --- |
| | 70nm | 5nm | 90nm | (45%:45%:10%) 30nm | 10nm | 30nm | |
| E15 | SpA1 | HATCN | SpMA1 | IC1:8h:TEG1 | IC1 | ST2:LiQ (50%:50%) | --- |
| | 70nm | 5nm | 90nm | (45%:40%:15%) 30nm | 10nm | 30nm | |
| E16 | SpA1 | HATCN | SpMA1 | IC1:8j:TEG1 | IC1 | ST2:LiQ (50%:50%) | --- |
| | 70nm | 5nm | 90nm | (55%:40%:5%) 30nm | 10nm | 30nm | |
| E17 | SpA1 | HATCN | SpMA1 | IC1:8i:TEG1 | IC1 | ST2:LiQ (50%:50%) | --- |
| | 70nm | 5nm | 90nm | (55%:40%:5%) 30nm | 10nm | 30nm | |
| E18 | SpA1 | HATCN | SpMA1 | IC1:81:TEG1 | IC1 | ST2:LiQ (50%:50%) | --- |
| | 70nm | 5nm | 90nm | (55%:40%:5%) 30nm | 10nm | 30nm | |
| E19 | SpA1 | HATCN | SpMA1 | IC1:8m:TER3 | --- | ST2:LiQ (50%:50%) | --- |
| | 90nm | 5nm | 130nm | (50%:45%:5%) 40nm | | 40nm | |
| E20 | SpA1 | HATCN | SpMA1 | IC1:8n:TER3 | --- | ST2:LiQ (50%:50%) | --- |
| | 90nm | 5nm | 130nm | (50%:45%:5%) 40nm | | 40nm | |
| E21 | SpA1 | HATCN | SpMA1 | IC1:8o:TEG1 | IC1 | ST2:LiQ (50%:50%) | --- |
| | 70nm | 5nm | 90nm | (45%:45%:10%) 30nm | 10nm | 30nm | |
| E22 | SpA1 | HATCN | SpMA1 | IC1:8q:TEG1 | IC1 | ST2:LiQ (50%:50%) | --- |
| | 70nm | 5nm | 90nm | (45%:45%:10%) 30nm | 10nm | 30nm | |
| E23 | SpA1 | HATCN | SpMA1 | IC1:8r:TEG1 | IC1 | ST2:LiQ (50%:50%) | --- |
| | 70nm | 5nm | 90nm | (45%:45%:10%) 30nm | 10nm | 30nm | |
| E24 | SpA1 | HATCN | SpMA1 | 12a:TEG1 | IC1 | ST2:LiQ (50%:50%) | --- |
| | 70nm | 5nm | 90nm | (90%:10%) 30nm | 10nm | 30nm | |
| E25 | SpA1 | HATCN | SpMA1 | 12b:TEG1 | IC1 | ST2:LiQ (50%:50%) | --- |
| | 70nm | 5nm | 90nm | (90%:10%) 30nm | 10nm | 30nm | |
| E26 | SpA1 | HATCN | SpMA1 | IC1:TEG1 (90%:10%) | IC1 | 12c:LiQ (50%:50%) | --- |
| | 70nm | 5nm | 90nm | 30nm | 10nm | 30nm | |
| E27 | SpA1 | HATCN | SpMA1 | 12d:TEG1 | IC1 | ST2:LiQ (50%:50%) | --- |
| | 70nm | 5nm | 90nm | (90%:10%) 30nm | 10nm | 30nm | |
| E28 | SpA1 | HATCN | SpMA1 | IC1:TEG1 | IC1 | 12e:LiQ (50%:50%) | --- |
| | 70nm | 5nm | 90nm | (90%:10%) 30nm | 10nm | 30nm | |
| E29 | SpA1 | HATCN | SpMA1 | IC1:18a:TER3 | --- | ST2:LiQ (50%:50%) | --- |
| | 90nm | 5nm | 130nm | (50%:45%:5%) 40nm | | 40nm | |
| E30 | SpA1 | HATCN | SpMA1 | IC1:18b:TER3 | --- | ST2:LiQ (50%:50%) | --- |
| | 90nm | 5nm | 130nm | (50%:45%:5%) 40nm | | 40nm | |
| E31 | SpA1 | HATCN | SpMA1 | 18d:TER3 | --- | ST2:LiQ (50%:50%) | --- |
| | 90nm | 5nm | 130nm | (92%:8%) 40nm | | 40nm | |
| E32 | SpA1 | HATCN | SpMA1 | 25:TEG1 | IC1 | ST2:LiQ (50%:50%) | --- |
| | 70nm | 5nm | 90nm | (90%:10%) 30nm | 10nm | 30nm | |
| E33 | SpA1 | HATCN | SpMA1 | IC1:25a:TEG1 | IC1 | ST2:LiQ (50%:50%) | --- |
| | 70nm | 5nm | 90nm | (45%:45%:10%) 30nm | 10nm | 30nm | |
| E34 | SpA1 | HATCN | SpMA1 | IC1:25b:TEG1 | IC1 | ST2:LiQ (50%:50%) | --- |
| | 70nm | 5nm | 90nm | (45%:45%:10%) 30nm | 10nm | 30nm | |
| E35 | SpA1 | HATCN | SpMA1 | IC1:25c:TEG1 | IC1 | ST2:LiQ (50%:50%) | --- |
| | 70nm | 5nm | 90nm | (45%:45%:10%) 30nm | 10nm | 30nm30nm | |
| E36 | SpA1 | HATCN | SpMA1 | IC1:TEG1 | IC1 | 25d:LiQ (50%:50%) | --- |
| | 70nm | 5nm | 90nm | (90%:10%) 30nm | 10nm | 30nm | |
| E37 | SpA1 | HATCN | SpMA1 | IC1:TEG1 | IC1 | 31:LiQ (50%:50%) | --- |
| | 70nm | 5nm | 90nm | (90%:10%) 30nm | 10nm | 30nm | |
| E38 | SpA1 | HATCN | SpMA1 | IC1:31a:TEG1 | IC1 | ST2:LiQ (50%:50%) | -- |
| | 70nm | 5nm | 90nm | (45%:45%:10%) 30nm | 10nm | 30nm | |
| E39 | SpA1 | HATCN | SpMA1 | IC1:31b:TEG1 | IC1 | ST2:LiQ (50%:50%) | --- |
| | 70nm | 5nm | 90nm | (45%:45%:10%) 30nm | 10nm | 30nm | |

**Tabelle 2: Daten der OLEDs**

| Bsp. | U1000 (V) | SE1000 (cd/A) | LE1000 (Im/W) | EQE 1000 | CIE x/y bei 1000 cd/m² | L₀; j₀ | L1 % | LD (h) |
|---|---|---|---|---|---|---|---|---|
| V1 | 3.7 | 64 | 54 | 17.2% | 0.33/0.63 | 20 mA/cm² | 80 | 125 |
| V2 | 3.8 | 64 | 53 | 17.3% | 0.30/0.65 | 20 mA/cm² | 80 | 120 |
| V3 | 3.8 | 64 | 53 | 17.4% | 0.32/0.64 | 20 mA/cm² | 80 | 130 |
| V4 | 3.6 | 63 | 55 | 17.2% | 0.31/0.64 | 20 mA/cm² | 80 | 90 |
| V5 | 3.6 | 63 | 55 | 17.1% | 0.33/0.63 | 20 mA/cm² | 80 | 135 |
| V6 | 3.7 | 62 | 53 | 17.3% | 0.31/0.64 | 20 mA/cm² | 80 | 115 |
| V7 | 3.4 | 59 | 55 | 15.9% | 0.32/0.64 | 20 mA/cm² | 80 | 105 |
| V8 | 3.4 | 58 | 54 | 15.6% | 0.33/0.63 | 20 mA/cm² | 80 | 150 |
| V9 | 3.5 | 56 | 50 | 15.5% | 0.32/0.63 | 20 mA/cm² | 80 | 140 |
| V10 | 3.4 | 59 | 55 | 15.7% | 0.32/0.63 | 20 mA/cm² | 80 | 165 |
| V11 | 3.5 | 58 | 52 | 15.6% | 0.34/0.62 | 20 mA/cm² | 80 | 195 |
| E1 | 3.3 | 64 | 61 | 17.4% | 0.32/0.64 | 20 mA/cm² | 80 | 125 |
| E2 | 3.3 | 63 | 60 | 17.5% | 0.32/0.63 | 20 mA/cm² | 80 | 120 |
| E3 | 3.1 | 64 | 65 | 17.5% | 0.31/0.64 | 20 mA/cm² | 80 | 130 |
| E4 | 3.5 | 56 | 50 | 15.8% | 0.32/0.63 | 20 mA/cm² | 80 | 170 |
| E5 | 3.5 | 58 | 52 | 15.7% | 0.33/0.63 | 20 mA/cm² | 80 | 185 |
| E6 | 3.6 | 58 | 51 | 15.8% | 0.31/0.64 | 20 mA/cm² | 80 | 175 |
| E7 | 3.4 | 58 | 54 | 15.5% | 0.33/0.63 | 20 mA/cm² | 80 | 200 |
| E8 | 3.6 | 56 | 49 | 15.4% | 0.34/0.62 | 20 mA/cm² | 80 | 235 |
| E9 | 3.5 | 56 | 50 | 15.7% | 0.30/0.64 | 20 mA/cm² | 80 | 200 |
| E10 | 3.4 | 57 | 53 | 15.4% | 0.31/0.64 | 20 mA/cm² | 80 | 180 |
| E11 | 4.6 | 12 | 8 | 12.5% | 0.67/0.33 | 4000 cd/m² | 80 | 405 |
| E12 | 3.5 | 58 | 52 | 15.5% | 0.33/0.64 | 20 mA/cm² | 80 | 175 |
| E13 | 3.4 | 57 | 53 | 15.2% | 0.33/0.63 | 20 mA/cm² | 80 | 190 |
| E14 | 3.6 | 58 | 51 | 15.7% | 0.32/0.63 | 20 mA/cm² | 80 | 215 |
| E15 | 3.7 | 56 | 48 | 15.1% | 0.34/0.63 | 20 mA/cm² | 80 | 220 |
| E16 | 3.5 | 59 | 53 | 15.7% | 0.33/0.63 | 20 mA/cm² | 80 | 230 |
| E17 | 3.6 | 53 | 46 | 15.2% | 0.34/0.61 | 20 mA/cm² | 80 | 225 |
| E18 | 3.6 | 57 | 50 | 15.3% | 0.32/0.64 | 20 mA/cm² | 80 | 215 |
| E19 | 4.4 | 13 | 9 | 13.5% | 0.67/0.33 | 4000 cd/m² | 80 | 470 |
| E20 | 4.5 | 16 | 11 | 14.1% | 0.67/0.33 | 4000 cd/m² | 80 | 460 |
| E21 | 3.6 | 55 | 48 | 15.6% | 0.34/0.61 | 20 mA/cm² | 80 | 180 |
| E22 | 3.5 | 58 | 52 | 15.8% | 0.31/0.64 | 20 mA/cm² | 80 | 175 |
| E23 | 3.6 | 57 | 50 | 15.7% | 0.31/0.64 | 20 mA/cm² | 80 | 190 |
| E24 | 3.4 | 55 | 51 | 15.6% | 0.34/0.61 | 20 mA/cm² | 80 | 195 |
| E25 | 3.6 | 58 | 51 | 15.5% | 0.33/0.64 | 20 mA/cm² | 80 | 145 |
| E26 | 3.3 | 62 | 59 | 17.3% | 0.31/0.64 | 20 mA/cm² | 80 | 125 |
| E27 | 3.4 | 53 | 49 | 15.1% | 0.33/0.62 | 20 mA/cm² | 80 | 185 |
| E28 | 3.2 | 63 | 62 | 17.5% | 0.32/0.63 | 20 mA/cm² | 80 | 120 |
| E29 | 4.5 | 14 | 10 | 13.8% | 0.67/0.33 | 4000 cd/m² | 80 | 445 |
| E30 | 4.4 | 16 | 11 | 13.9% | 0.66/0.34 | 4000 cd/m² | 80 | 440 |
| E31 | 4.7 | 13 | 9 | 12.4% | 0.67/0.33 | 4000 cd/m² | 80 | 390 |
| E32 | 3.3 | 56 | 53 | 15.3% | 0.31/0.64 | 20 mA/cm² | 80 | 175 |
| E33 | 3.5 | 56 | 50 | 15.6% | 0.31/0.63 | 20 mA/cm² | 80 | 180 |
| E34 | 3.4 | 59 | 55 | 15.7% | 0.34/0.63 | 20 mA/cm² | 80 | 175 |
| E35 | 3.6 | 60 | 52 | 15.9% | 0.33/0.63 | 20 mA/cm² | 80 | 165 |
| E36 | 3.4 | 62 | 57 | 17.3% | 0.31/0.64 | 20 mA/cm² | 80 | 130 |
| E37 | 3.4 | 64 | 59 | 17.4% | 0.32/0.64 | 20 mA/cm² | 80 | 120 |
| E38 | 3.5 | 59 | 53 | 15.7% | 0.32/0.64 | 20 mA/cm² | 80 | 170 |
| E39 | 3.4 | 59 | 55 | 15.9% | 0.33/0.64 | 20 mA/cm² | 80 | 165 |

**Tabelle 3: Strukturformeln der Materialien für die OLEDs**

| | |
|---|---|
| 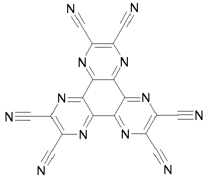 | 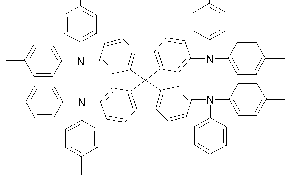 |
| HATCN | SpA1 |
| 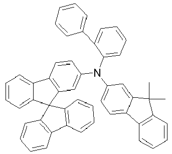 | 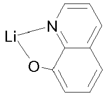 |
| SpMA1 | LiQ |
| 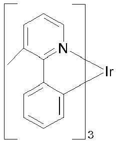 | 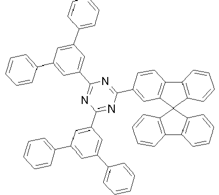 |
| TEG1 | ST2 |
| 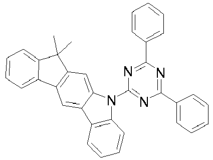 | 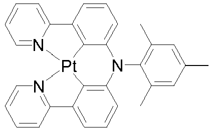 |
| IC1 | TER3 |
| 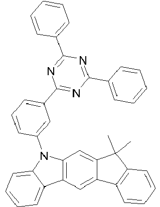 | 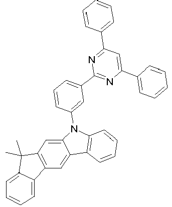 |
| SdT1 (gemäß WO 2010/136109) | SdT2 (gemäß WO 2012/069121) |
| 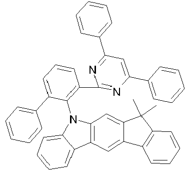 | 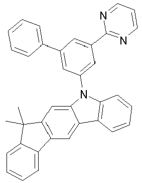 |
| SdT3 (gemäß WO 2014/146752) | SdT4 (gemäß WO 2012/069121) |
| 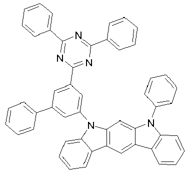 | 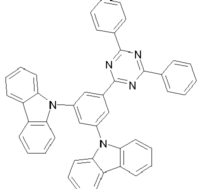 |
| SdT5 (gemäß WO 2015/046916) | SdT6 (gemäß JP 2009-021336) |
| 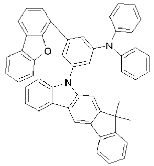 | 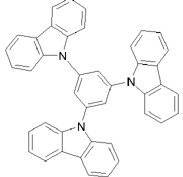 |
| SdT7 (gemäß WO 2014/196805) | SdT8 (gemäß Thin Solid Films, 2003, 436(2), 264-268) |
| 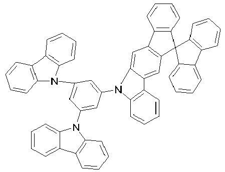 | 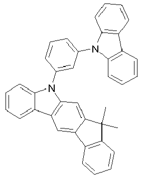 |
| SdT9 (gemäß KR 2010-028471) | SdT10 (gemäß WO 2013/041176) |
| 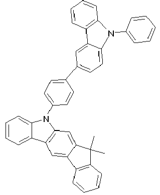 | 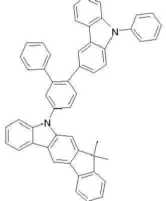 |
| SdT11 (gemäß WO 2013/041176) | EG8 |
| 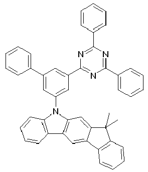 | 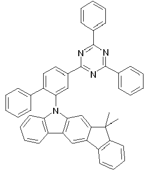 |
| EG1 | EG2 |
| 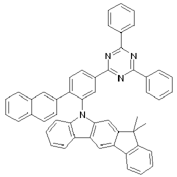 | 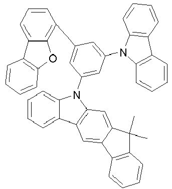 |
| EG3 | EG4 |
| 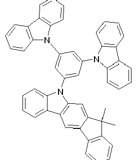 | 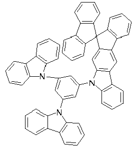 |
| EG5 | EG6 |
| 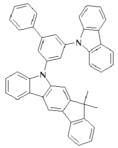 | 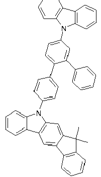 |
| EG7 | 6k |
| 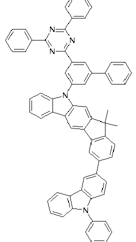 | 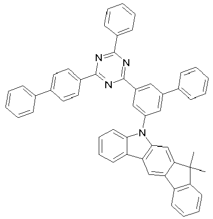 |
| 8b | 8c |
| 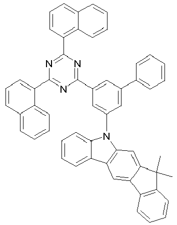 | 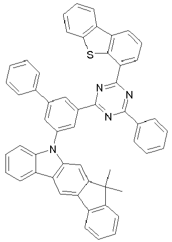 |
| 8d | 8e |
| 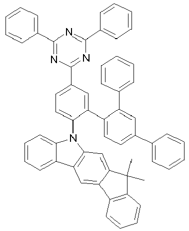 | |
| 8f | |
| 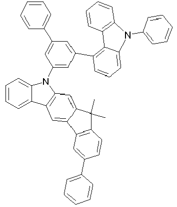 | 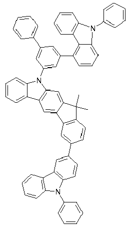 |
| 8h | 8i |
| 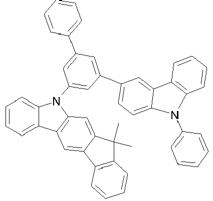 | 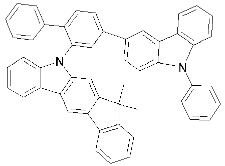 |
| 8i | 8l |
| 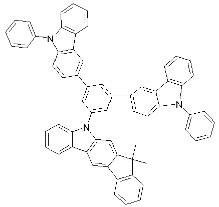 | 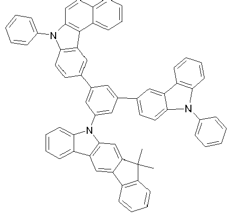 |
| 8m | 8n |
| 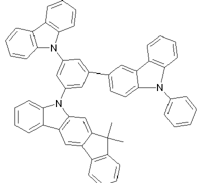 | 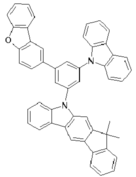 |
| 8o | 8q |
| 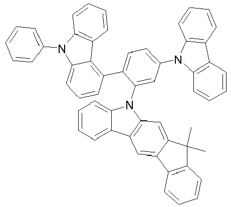 | 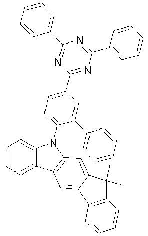 |
| 8r | 12a |
| 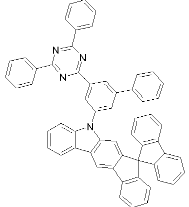 | 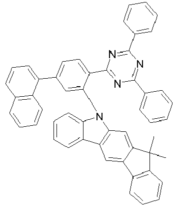 |
| 12b | 12c |
| 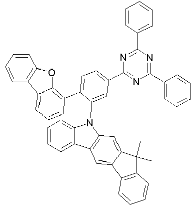 | 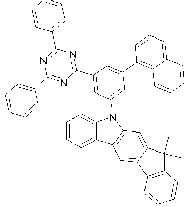 |
| 12d | 12e |
| 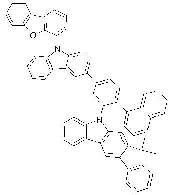 | 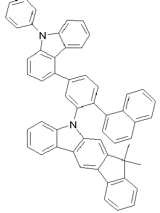 |
| 18a | 18b |
| 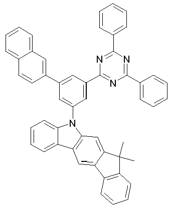 | 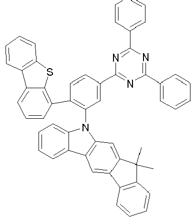 |
| 18d | 25 |
| 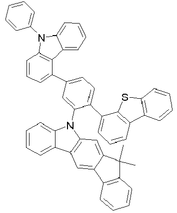 | 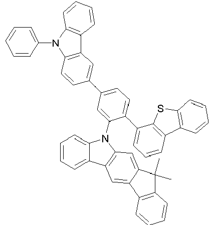 |
| 25a | 25b |
| 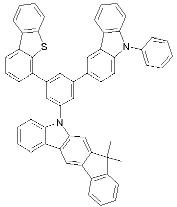 | 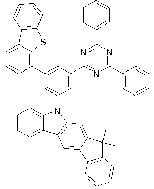 |
| 25c | 25d |
| 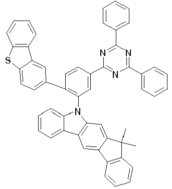 | 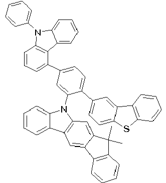 |
| 31 | 31a |
| 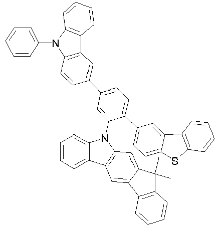 | |
| 31b | |

## Patentansprüche

1. Verbindung gemäß Formel (1), 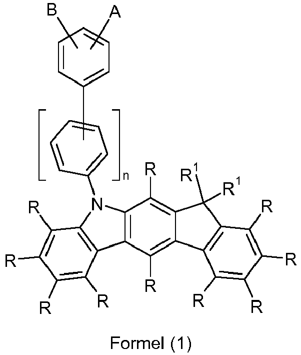 wobei für die verwendeten Symbole und Indizes gilt:
A ist ausgewählt aus den Strukturen der Formeln (A-1) bis (A-6), 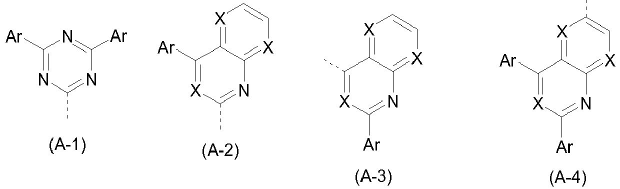 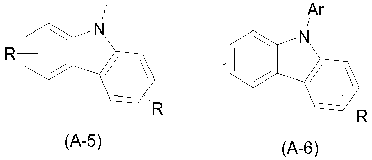 wobei die gestrichelte Bindung die Verknüpfung dieser Gruppen darstellt und wobei an die Gruppen (A-5) und (A-6) optional eine Benzogruppe ankondensiert sein kann, die durch einen oder mehrere Reste R substituiert sein kann;
B ist ein aromatisches Ringsystem mit 6 bis 30 aromatischen Ringatomen oder ein elektronenreiches heteroaromatisches Ringsystem mit 13 bis 30 aromatischen Ringatomen, welches jeweils mit einem oder mehreren nicht-aromatischen Resten R substituiert sein kann;
X ist bei jedem Auftreten gleich oder verschieden CH oder N, wobei maximal ein X für N steht;
Ar ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R substituiert sein kann;
R, R¹ ist bei jedem Auftreten gleich oder verschieden ausgewählt aus der Gruppe bestehend aus H, D, F, Cl, Br, I, Si(R²)₃, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenyl- oder Alkinylgruppe mit 2 bis 20 C-Atomen, die jeweils mit einem oder mehreren Resten R² substituiert sein kann, wobei eine oder mehrere nicht-benachbarte CH₂-Gruppen durch R²C=CR², C≡C, Si(R²)₂, C=O, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R² substituiert sein kann, oder einer Aryloxy- oder Heteroaryloxygruppe mit 5 bis 40 aromatischen Ringatomen, die mit einem oder mehreren Resten R² substituiert sein kann; dabei können optional zwei benachbarte Substituenten R¹ ein monocyclisches oder polycyclisches, aliphatisches, aromatisches oder heteroaromatisches Ringsystem bilden, das mit einem oder mehreren Resten R² substituiert sein kann;
R² ist bei jedem Auftreten gleich oder verschieden ausgewählt aus der Gruppe bestehend aus H, D, F, CN, einem aliphatischen Kohlenwasserstoffrest mit 1 bis 20 C-Atomen, oder einem aromatischen oder heteroaromatischen Ringsystem mit 5 bis 30 aromatischen Ringatomen, in dem ein oder mehrere H-Atome durch D, F, CN oder eine Alkylgruppe mit 1 bis 10 C-Atomen ersetzt sein können, wobei zwei oder mehr benachbarte Substituenten R² miteinander ein mono- oder polycyclisches, aliphatisches Ringsystem bilden können;
n ist 0 oder 1.

2. Verbindung nach Anspruch 1 gemäß Formel (2), 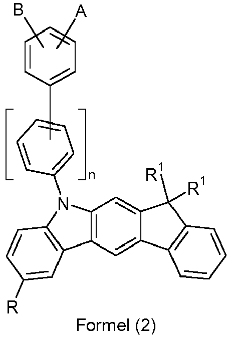 wobei die verwendeten Symbole und Indizes die in Anspruch 1 genannten Bedeutungen aufweisen.

3. Verbindung nach Anspruch 1 oder 2 gemäß den Formeln (3) oder (4), 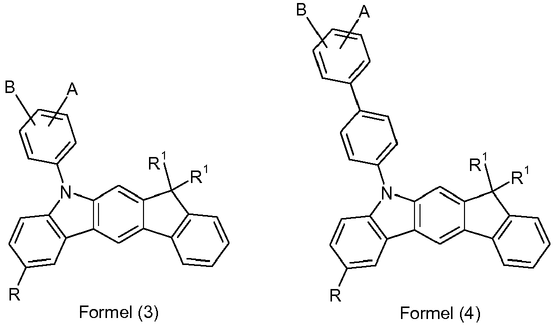 wobei die verwendeten Symbole die in Anspruch 1 genannten Bedeutungen aufweisen.

4. Verbindung nach einem oder mehreren der Ansprüche 1 bis 3 gemäß den Formeln (3a) oder (4a), 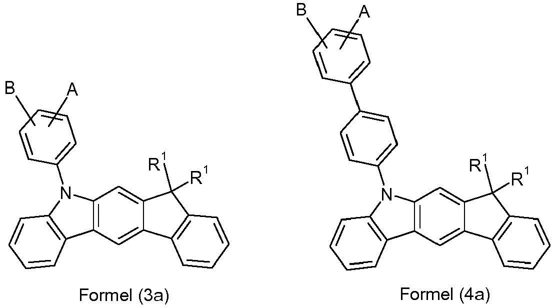 wobei die verwendeten Symbole die in Anspruch 1 genannten Bedeutungen aufweisen.

5. Verbindung nach einem oder mehreren der Ansprüche 1 bis 4, **dadurch gekennzeichnet, dass** die Phenylgruppe, an die die Gruppen A und B gebunden sind, ausgewählt ist aus den folgenden Strukturen, 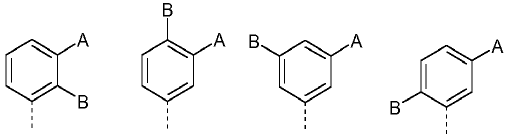 wobei die Gruppen A und B mit eingezeichnet sind, die gestrichelte Bindung für n = 0 die Bindung an das Stickstoffatom des Indenocarbazols und für n = 1 die Bindung an die Phenylengruppe darstellt und A und B die in Anspruch 1 genannten Bedeutungen aufweisen.

6. Verbindung nach einem oder mehreren der Ansprüche 1 bis 5, **dadurch gekennzeichnet, dass** die Gruppe A ausgewählt ist aus den Gruppen (A-1a), (A-2a) bis (A-2d), (A-3a), (A-4a), (A-5a) bis (A-5d) und (A-6a) bis (A-6f), 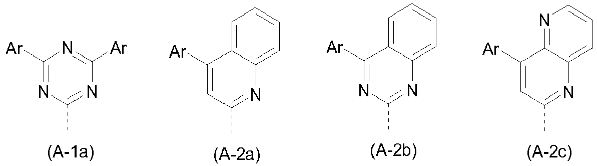 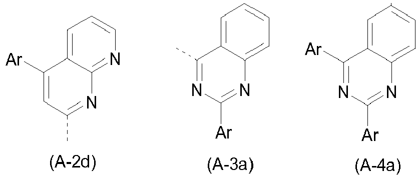 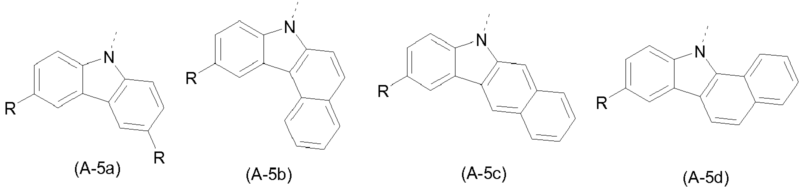 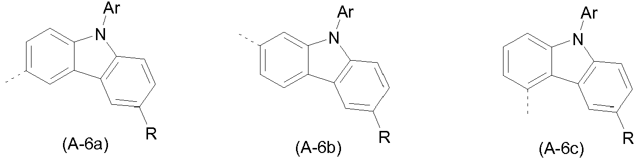 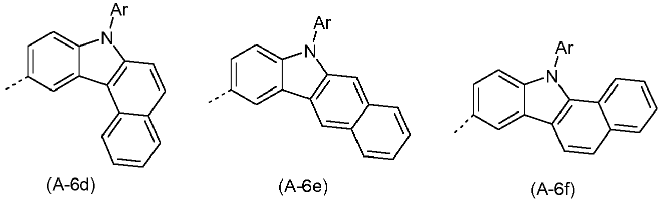 wobei die verwendeten Symbole die in Anspruch 1 genannten Bedeutungen haben.

7. Verbindung nach einem oder mehreren der Ansprüche 1 bis 6, ausgewählt aus den Verbindungen der Formeln (3b) und (4b), 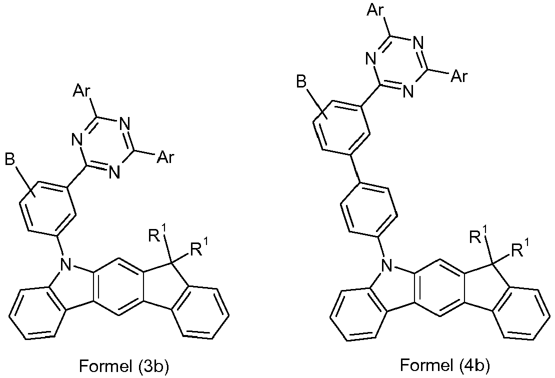 wobei die verwendeten Symbole die in Anspruch 1 genannten Bedeutungen aufweisen.

8. Verbindung nach einem oder mehreren der Ansprüche 1 bis 7, **dadurch gekennzeichnet, dass** Ar ausgewählt ist aus der Gruppe bestehend aus Phenyl, Biphenyl, Terphenyl, Quaterphenyl, Fluorenyl, Spirobifluorenyl, Dibenzofuranyl, Dibenzothienyl oder N-Phenyl-Carbazolyl, wobei diese Gruppen jeweils durch einen oder mehrere nicht-aromatische Reste R substituiert sein können.

9. Verbindung nach einem oder mehreren der Ansprüche 1 bis 8, **dadurch gekennzeichnet, dass** die Gruppe B ausgewählt ist aus der Gruppe bestehend aus Phenyl, Biphenyl, Terphenyl, Quaterphenyl, Fluorenyl, Spirobifluorenyl, Naphthyl, Phenanthrenyl, Triphenylenyl, Dibenzofuranyl, Dibenzothienyl oder N-Phenyl-Carbazolyl, wobei diese Gruppen jeweils durch einen oder mehrere nicht-aromatische Reste R substituiert sein können.

10. Verbindung nach einem oder mehreren der Ansprüche 1 bis 9, **dadurch gekennzeichnet, dass** die Gruppe B ausgewählt ist aus den Strukturen der Formeln (B-1a) bis (B-10b), 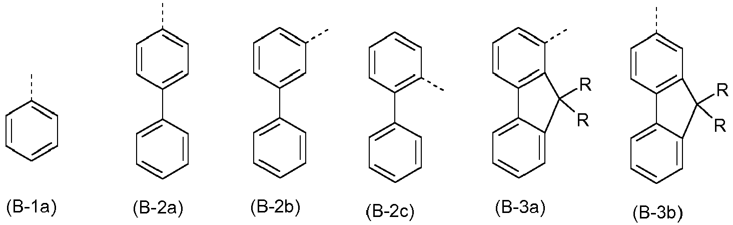 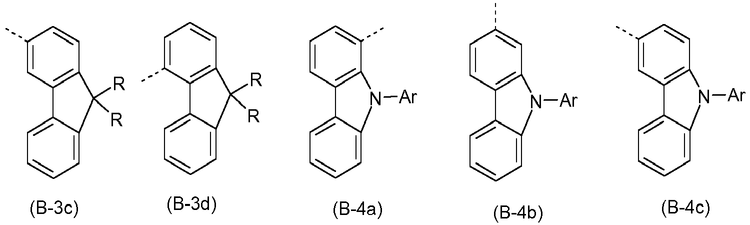 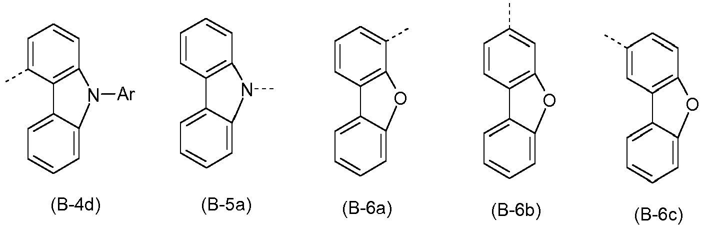 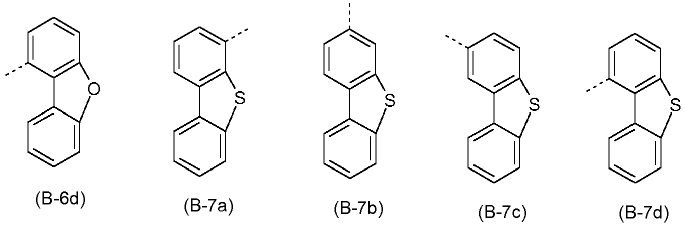 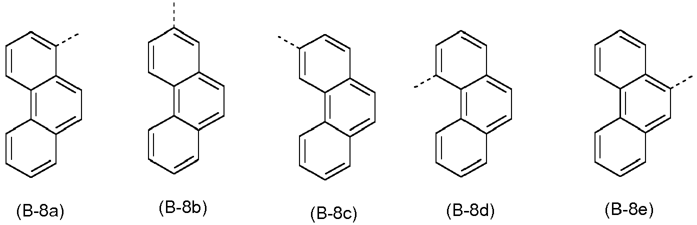 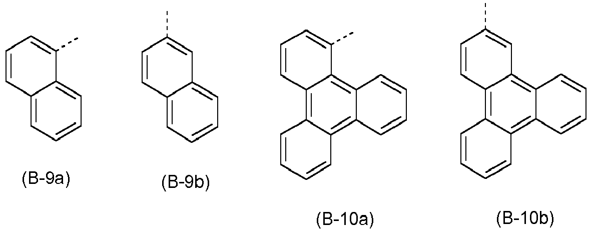 wobei die gestrichelte Bindung die Verknüpfung dieser Gruppe darstellt und die weiteren verwendeten Symbole die in Anspruch 1 genannten Bedeutungen aufweisen.

11. Formulierung, enthaltend mindestens eine Verbindung nach einem oder mehreren der Ansprüche 1 bis 10 und mindestens eine weitere Verbindung, insbesondere ein Lösemittel.

12. Verwendung einer Verbindung nach einem oder mehreren der Ansprüche 1 bis 10 in einer elektronischen Vorrichtung.

13. Elektronische Vorrichtung enthaltend mindestens eine Verbindung nach einem oder mehreren der Ansprüche 1 bis 10, insbesondere ausgewählt aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, farbstoffsensibilisierten organischen Solarzellen, organischen optischen Detektoren, organischen Photorezeptoren, organischen Feld-Quench-Devices, lichtemittierenden elektrochemischen Zellen, organischen Laserdioden und "organic plasmon emitting devices".

14. Elektronische Vorrichtung nach Anspruch 13, wobei es sich um eine organische Elektrolumineszenzvorrichtung handelt, **dadurch gekennzeichnet, dass** die Verbindung nach einem oder mehreren der Ansprüche 1 bis 10 als Matrixmaterial für phosphoreszierende Emitter in einer emittierenden Schicht oder als Elektronentransportmaterial in einer Lochblockierschicht oder eine Elektronentransportschicht eingesetzt wird

## Claims

1. Compound of formula (1) 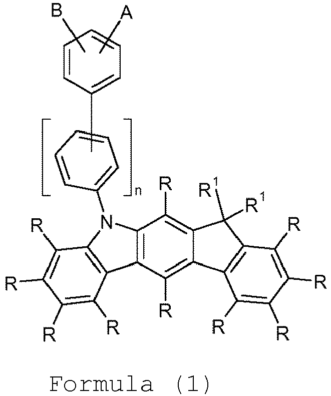 where the symbols and indices used are as follows:
A is selected from the structures of the formulae (A-1) to (A-6) 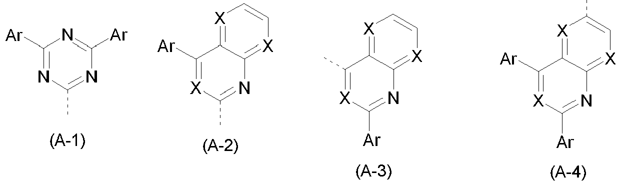 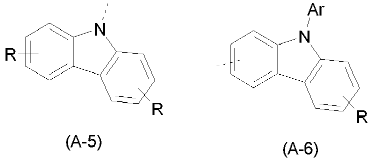
where the dotted bond represents the attachment of these groups and where a benzo group may optionally be fused to the (A-5) and (A-6) groups and may be substituted by one or more R radicals;
B is an aromatic ring system having 6 to 30 aromatic ring atoms or an electron-rich heteroaromatic ring system having 13 to 30 aromatic ring atoms, each of which may be substituted by one or more nonaromatic R radicals;
X is the same or different at each instance and is CH or N, where not more than one X is N;
Ar is the same or different at each instance and is an aromatic or heteroaromatic ring system which has 5 to 30 aromatic ring atoms and may be substituted by one or more nonaromatic R radicals;
R, R¹ is the same or different at each instance and is selected from the group consisting of H, D, F, Cl, Br, I, Si(R²)₃, a straight-chain alkyl, alkoxy or thioalkyl group having 1 to 20 carbon atoms or a branched or cyclic alkyl, alkoxy or thioalkyl group having 3 to 20 carbon atoms or an alkenyl or alkynyl group having 2 to 20 carbon atoms, each of which may be substituted by one or more R² radicals, where one or more nonadjacent CH₂ groups may be replaced by R²C=CR², C=C, Si(R²)₂, C=O, C=NR², P(=O)(R²), SO, SO₂, NR², O, S or CONR² and where one or more hydrogen atoms may be replaced by D, F, Cl, Br, I, CN or NO₂, an aromatic or heteroaromatic ring system which has 5 to 40 aromatic ring atoms and may be substituted in each case by one or more R² radicals, or an aryloxy or heteroaryloxy group which has 5 to 40 aromatic ring atoms and may be substituted by one or more R² radicals; at the same time, it is optionally possible for two adjacent R¹ substituents to form a monocyclic or polycyclic, aliphatic, aromatic or heteroaromatic ring system which may be substituted by one or more R² radicals;
R² is the same or different at each instance and is selected from the group consisting of H, D, F, CN, an aliphatic hydrocarbyl radical having 1 to 20 carbon atoms and an aromatic or heteroaromatic ring system having 5 to 30 aromatic ring atoms in which one or more hydrogen atoms may be replaced by D, F, CN or an alkyl group having 1 to 10 carbon atoms, where two or more adjacent R² substituents together may form a mono- or polycyclic, aliphatic ring system;
n is 0 or 1.

2. Compound according to Claim 1 of formula (2) 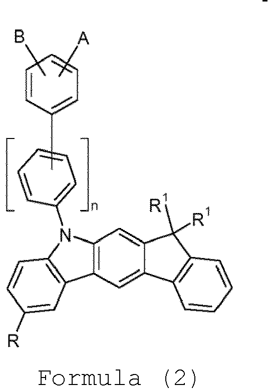 where the symbols and indices used have the definitions given in Claim 1.

3. Compound according to Claim 1 or 2 of the formula (3) or (4) 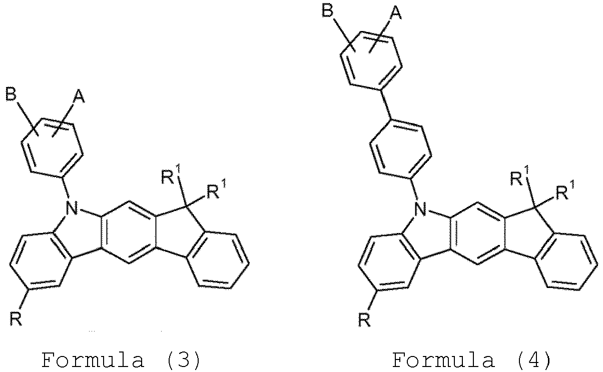 where the symbols used have the definitions given in Claim 1.

4. Compound according to one or more of Claims 1 to 3 of the formula (3a) or (4a) 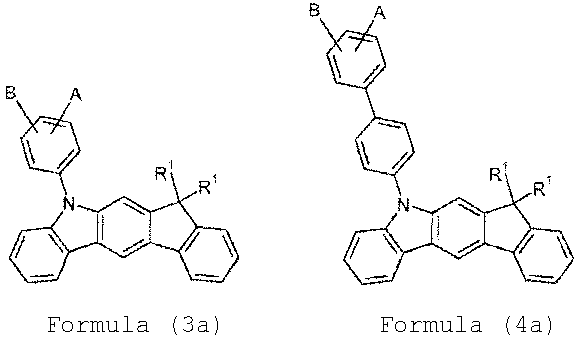 where the symbols used have the definitions given in Claim 1.

5. Compound according to one or more of Claims 1 to 4, **characterized in that** the phenyl group to which the A and B groups are bonded is selected from the following structures: 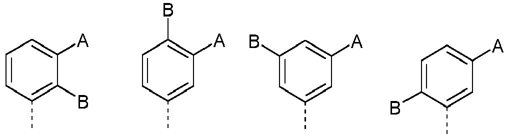 where the A and B groups are shown as well, the dotted bond when n = 0 represents the bond to the nitrogen atom of the indenocarbazole and when n = 1 represents the bond to the phenylene group and A and B have the definitions given in Claim 1.

6. Compound according to one or more of Claims 1 to 5, **characterized in that** the A group is selected from the (A-la), (A-2a) to (A-2d), (A-3a), (A-4a), (A-5a) to (A-5d) and (A-6a) to (A-6f) groups 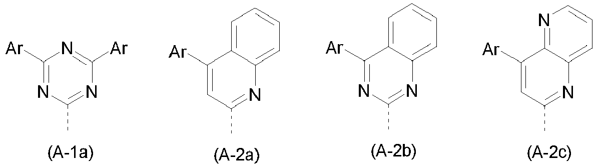 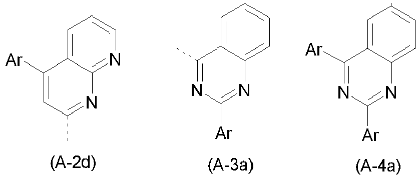 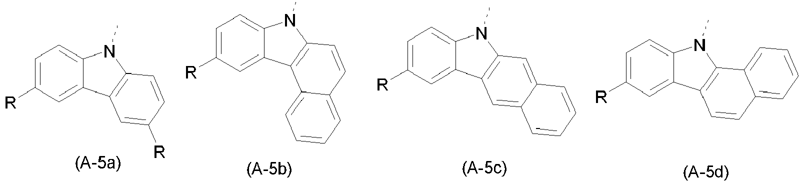 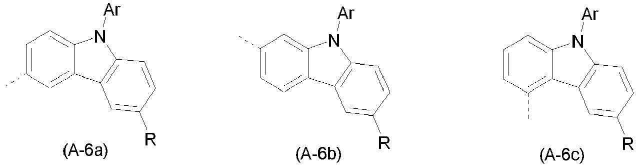 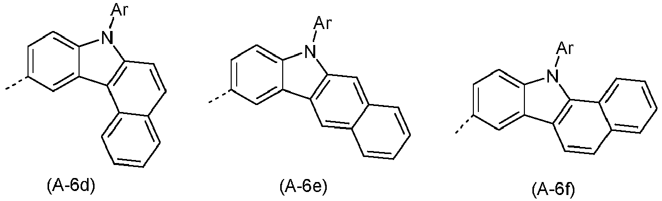 where the symbols used have the definitions given in Claim 1.

7. Compound according to one or more of Claims 1 to 6, selected from the compounds of the formulae (3b) and (4b) 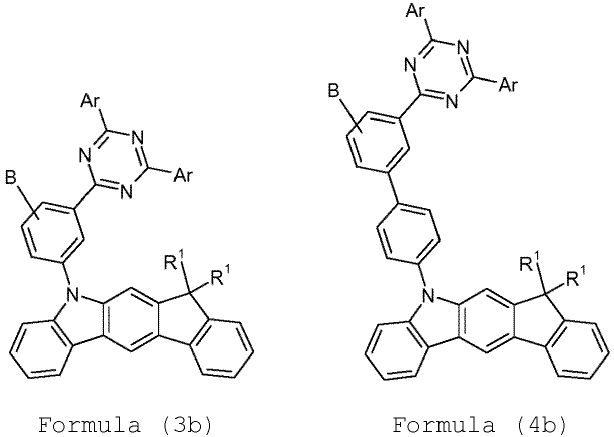 where the symbols used have the definitions given in Claim 1.

8. Compound according to one or more of Claims 1 to 7, **characterized in that** Ar is selected from the group consisting of phenyl, biphenyl, terphenyl, quaterphenyl, fluorenyl, spirobifluorenyl, dibenzofuranyl, dibenzothienyl and N-phenylcarbazolyl, where these groups may each be substituted by one or more nonaromatic R radicals.

9. Compound according to one or more of Claims 1 to 8, **characterized in that** the B group is selected from the group consisting of phenyl, biphenyl, terphenyl, quaterphenyl, fluorenyl, spirobifluorenyl, naphthyl, phenanthrenyl, triphenylenyl, dibenzofuranyl, dibenzothienyl and N-phenylcarbazolyl, where these groups may each be substituted by one or more nonaromatic R radicals.

10. Compound according to one or more of Claims 1 to 9, **characterized in that** the B group is selected from the structures of the formulae (B-1a) to (B-10b) 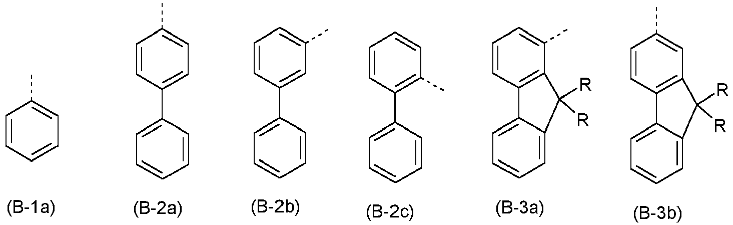 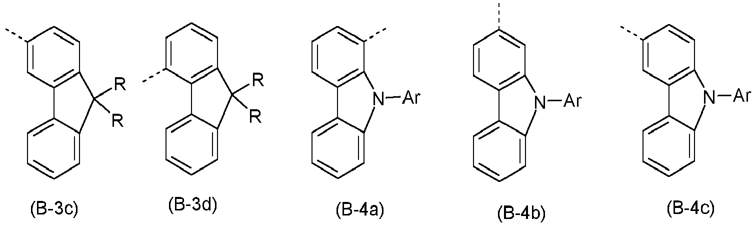 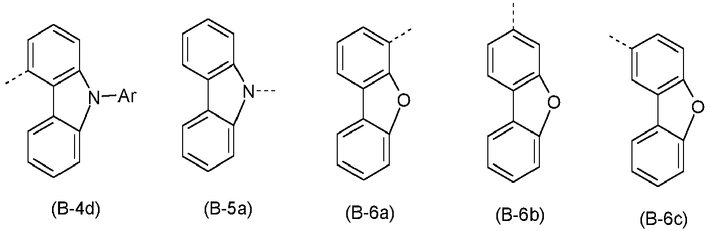 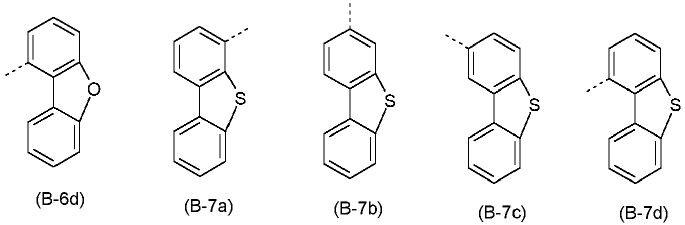 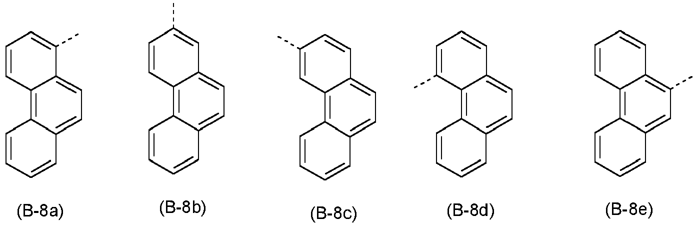 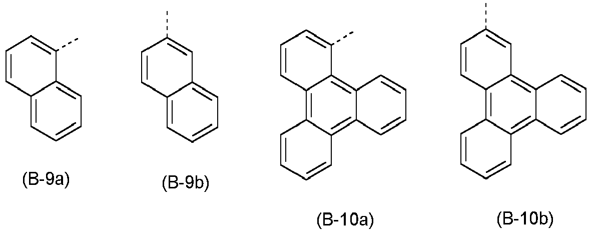 where the dotted bond represents the attachment of this group and the further symbols used have the definitions given in Claim 1.

11. Formulation comprising at least one compound according to one or more of Claims 1 to 10 and at least one further compound, especially a solvent.

12. Use of a compound according to one or more of Claims 1 to 10 in an electronic device.

13. Electronic device comprising at least one compound according to one or more of Claims 1 to 10, especially selected from the group consisting of organic electroluminescent devices, organic integrated circuits, organic field-effect transistors, organic thin-film transistors, organic light-emitting transistors, organic solar cells, dye-sensitized organic solar cells, organic optical detectors, organic photoreceptors, organic field-quench devices, light-emitting electrochemical cells, organic laser diodes and organic plasmon-emitting devices.

14. Electronic device according to Claim 13 which is an organic electroluminescent device, **characterized in that** the compound according to one or more of Claims 1 to 10 is used as matrix material for phosphorescent emitters in an emitting layer or as electron transport material in a hole blocker layer or an electron transport layer.

## Revendications

1. Composé de formule (1), 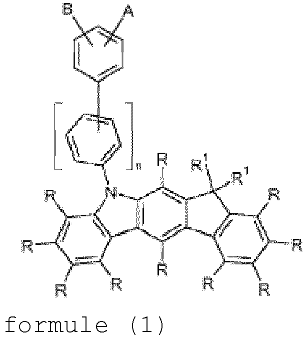 ceci s'appliquant pour les symboles et indices utilisés :
A étant choisi parmi les structures des formules (A-1) à (A-6), 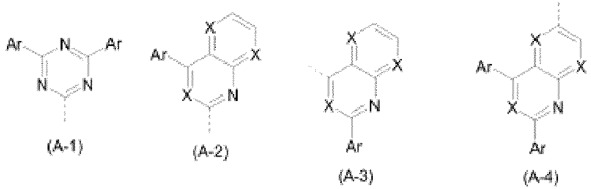 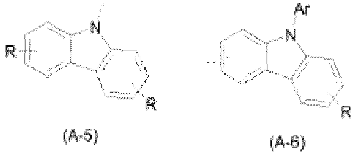 la liaison en pointillés représentant le lien à ces groupes et un groupe benzénique pouvant éventuellement être condensé aux groupes (A-5) et (A-6), qui peut être substitué par un ou plusieurs radicaux R ;
B étant un système cyclique aromatique comportant 6 à 30 atomes de cycle aromatiques ou un système cyclique hétéroaromatique riche en électrons comportant 13 à 30 atomes de cycle aromatiques, qui peut être à chaque fois substitué par un ou plusieurs radicaux R non aromatiques ;
X identique ou différent en chaque occurrence, étant CH ou N ; au maximum un X représentant N ;
Ar identique ou différent en chaque occurrence, étant un système cyclique aromatique ou hétéroaromatique comportant 5 à 30 atomes de cycle aromatiques, qui peut être substitué par un ou plusieurs radicaux R non aromatiques ;
R, R¹ identiques ou différents en chaque occurrence, étant choisis dans le groupe constitué par H, D, F, Cl, Br, I, Si(R²)₃, un groupe alkyle, alcoxy ou thioalkyle linéaire comportant 1 à 20 atome(s) C ou un groupe alkyle, alcoxy ou thioalkyle ramifié ou cyclique comportant 3 à 20 atomes C ou un groupe alcényle ou alcynyle comportant 2 à 20 atomes de c, qui peut à chaque fois être substitué par un ou plusieurs radicaux R², un ou plusieurs groupes CH₂ non voisins pouvant être remplacés par R²C=CR², C=C, Si(R²)₂, C=O, C=NR², P(=O)(R²), SO, SO₂, NR², O, S ou CONR² et un ou plusieurs atomes H pouvant être remplacés par D, F, Cl, Br, I, CN ou NO₂, un système cyclique aromatique ou hétéroaromatique comportant 5 à 40 atomes de cycle aromatiques, qui à chaque fois peut être substitué par un ou plusieurs radicaux R², ou un groupe aryloxy ou hétéroaryloxy comportant 5 à 40 atomes de cycle aromatiques, qui peut être substitué par un ou plusieurs radicaux R² ; deux substituants R¹ voisins pouvant éventuellement former un système cyclique monocyclique ou polycyclique, aliphatique, aromatique ou hétéroaromatique, qui peut être substitué par un ou plusieurs radicaux R² ;
R² identique ou différent en chaque occurrence, étant choisi dans le groupe constitué par H, D, F, CN, un radical hydrocarboné aliphatique comportant 1 à 20 atome (s) C, ou un système cyclique aromatique ou hétéroaromatique comportant 5 à 30 atomes de cycle aromatiques, dans lequel un ou plusieurs atomes H peuvent être remplacés par D, F, CN ou un groupe alkyle comportant 1 à 10 atome(s) C, deux substituants R² voisins ou plus pouvant former ensemble un système cyclique aliphatique monocyclique ou polycyclique ;
n étant 0 ou 1.

2. Composé selon la revendication 1 de formule (2), 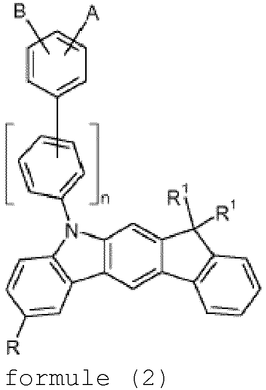 les symboles et les indices utilisés présentant les significations mentionnées dans la revendication 1.

3. Composé selon la revendication 1 ou 2 de formules (3) ou (4), 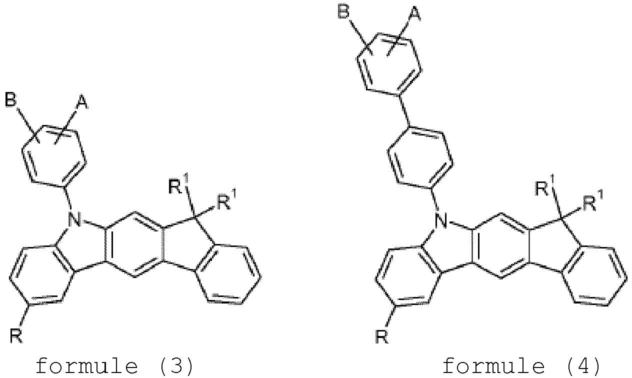 les symboles utilisés présentant les significations mentionnées dans la revendication 1.

4. Composé selon l'une ou plusieurs des revendications 1 à 3 de formules (3a) ou (4a), 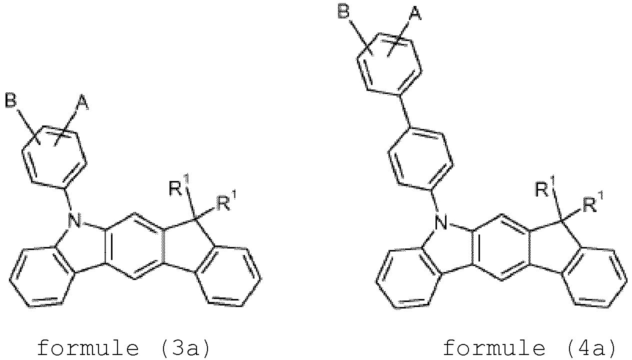 les symboles utilisés présentant les significations mentionnées dans la revendication 1.

5. Composé selon l'une ou plusieurs des revendications 1 à 4, **caractérisé en ce que** le groupe phényle, auquel les groupes A et B sont liés, est choisi parmi les structures suivantes : 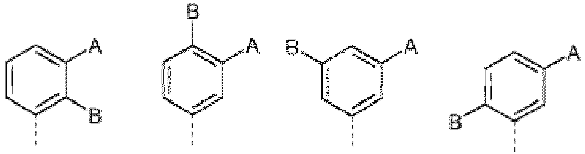 les groupes A et B étant signalés, la liaison en pointillés représentant pour n = 0 la liaison à l'atome d'azote de l'indénocarbazole et pour n = 1 la liaison au groupe phénylène et A et B présentant les significations mentionnées dans la revendication 1.

6. Composé selon l'une ou plusieurs des revendications 1 à 5, **caractérisé en ce que** le groupe A est choisi parmi les groupes (A-1a), (A-2a) à (A-2d), (A-3a), (A-4a), (A-5a) à (A-5d) et (A-6a) à (A-6f), 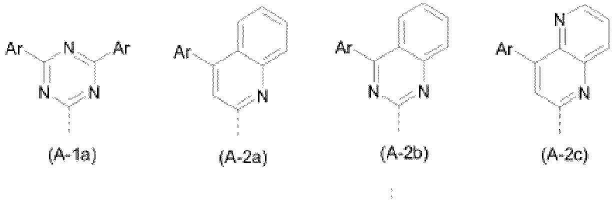 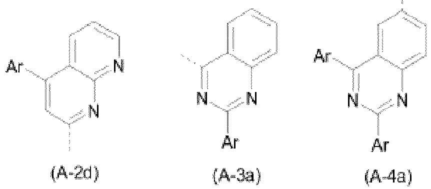 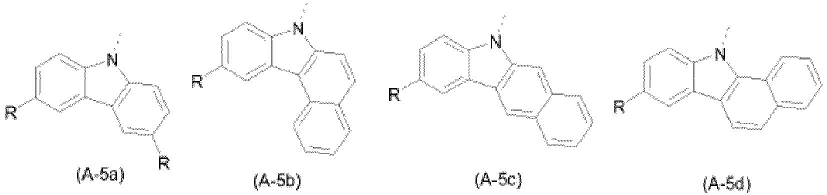 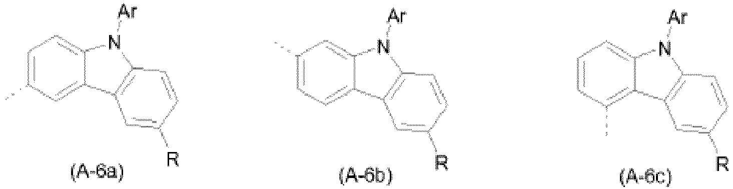 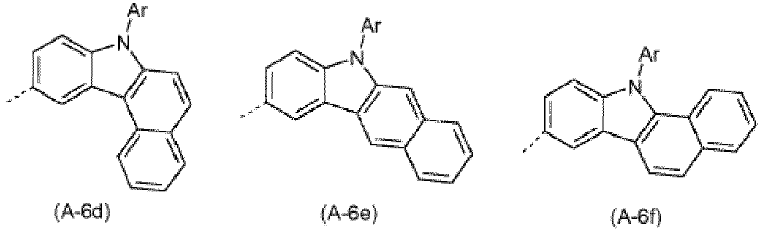 les symboles utilisés présentant les significations mentionnées dans la revendication 1.

7. Composé selon l'une ou plusieurs des revendications 1 à 6, choisi parmi les composés des formules (3b) et (4b), 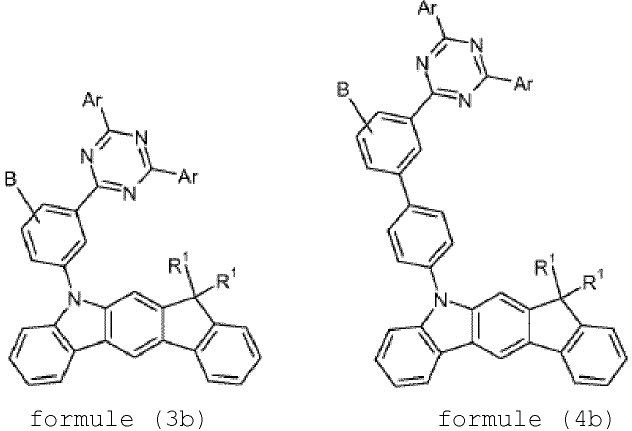 les symboles utilisés présentant les significations mentionnées dans la revendication 1.

8. Composé selon l'une ou plusieurs des revendications 1 à 7, **caractérisé en ce que** Ar est choisi dans le groupe constitué par phényle, biphényle, terphényle, quaterphényle, fluorényle, spirobifluorényle, dibenzofurannyle, dibenzothiényle ou N-phényl-carbazolyle, ces groupes pouvant à chaque fois être substitués par un ou plusieurs radicaux R non aromatiques.

9. Composé selon l'une ou plusieurs des revendications 1 à 8, **caractérisé en ce que** le groupe B est choisi dans le groupe constitué par phényle, biphényle, terphényle, quaterphényle, fluorényle, spirobifluorényle, naphtyle, phénanthrényle, triphénylényle, dibenzofurannyle, dibenzothiényle ou N-phényl-carbazolyle, ces groupes pouvant à chaque fois être substitués par un ou plusieurs radicaux R non aromatiques.

10. Composé selon l'une ou plusieurs des revendications 1 à 9, **caractérisé en ce que** le groupe B est choisi parmi les structures des formules (B-1a) à (B-10b), 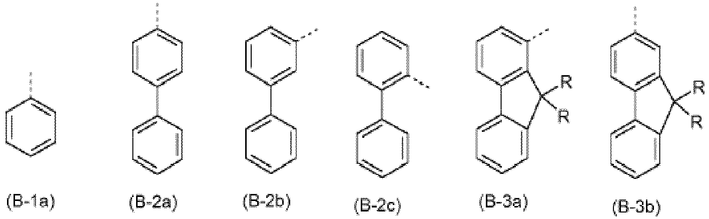 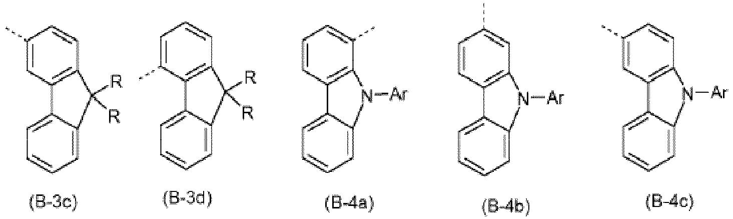 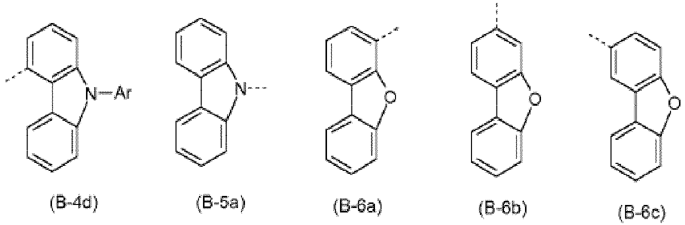 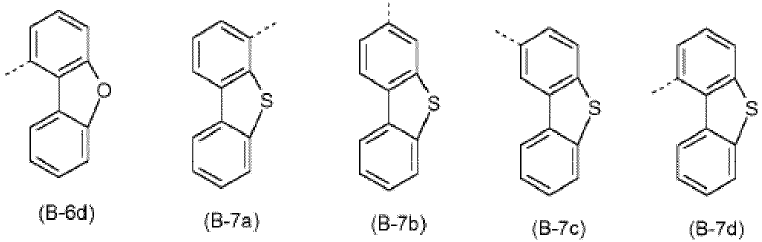 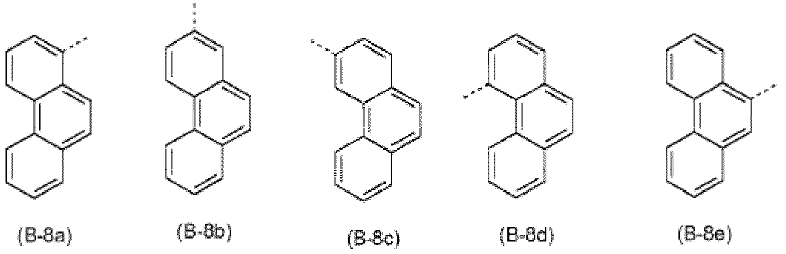 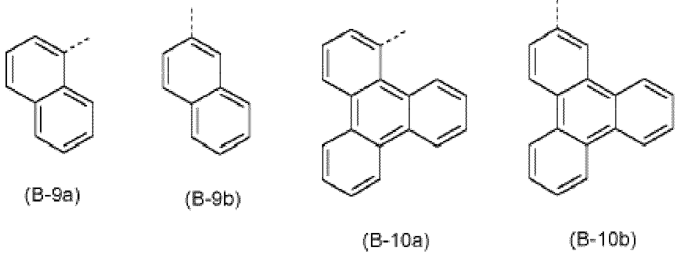 la liaison en pointillés représentant le lien à ce groupe et les autres symboles utilisés présentant les significations mentionnées dans la revendication 1.

11. Formulation, contenant au moins un composé selon l'une ou plusieurs des revendications 1 à 10 et au moins un autre composé, en particulier un solvant.

12. Utilisation d'un composé selon l'une ou plusieurs des revendications 1 à 10 dans un dispositif électronique.

13. Dispositif électronique contenant au moins un composé selon l'une ou plusieurs des revendications 1 à 10, en particulier choisi dans le groupe constitué par des dispositifs organiques électroluminescents, des circuits intégrés organiques, des transistors organiques à effet de champ, des transistors organiques de film mince, des transistors organiques émetteurs de lumière, des cellules solaires organiques, les cellules solaires organiques sensibilisées par un colorant, des détecteurs optiques organiques, des photorécepteurs organiques, des dispositifs organiques d'extinction de champ, des cellules électrochimiques émettrices de lumière, des diodes laser organiques et des « organic plasmon emitting devices » (dispositifs organiques d'émission de plasmon).

14. Dispositif électronique selon la revendication 13, qui est un dispositif organique électroluminescent, **caractérisé en ce que** le composé selon l'une ou plusieurs des revendications 1 à 10 est utilisé en tant que matériau matriciel pour des émetteurs phosphorescents dans une couche émettrice ou en tant que matériau de transport d'électrons dans une couche de blocage de trous ou une couche de transport d'électrons.
